(19)

Europäisches Patentamt

European Patent Office

Office européen des brevets

(11) **EP 4 439 571 A1**

(12) **EUROPEAN PATENT APPLICATION**

(43) Date of publication:
**02.10.2024 Bulletin 2024/40**

(21) Application number: **24165815.2**

(22) Date of filing: **25.03.2024**

(51) International Patent Classification (IPC):
***G16H 10/40*** (2018.01)

(52) Cooperative Patent Classification (CPC):
**G16H 10/40;** G16H 40/63; G16H 40/67

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.03.2023 JP 2023052355**

(71) Applicant: **SYSMEX CORPORATION
Kobe-shi
Hyogo 651-0073 (JP)**

(72) Inventors:
• **MASUDA, Yuji
  Kobe-shi, Hyogo 651-0073 (JP)**
• **MICHIMOTO, Kenjirou
  Kobe-shi, Hyogo 651-0073 (JP)**
• **KIMURA, Konobu
  Kobe-shi, Hyogo 651-0073 (JP)**
• **SUZUKI, Kenichiro
  Kobe-shi, Hyogo 651-0073 (JP)**
• **NAKANISHI, Noriyuki
  Kobe-shi, Hyogo 651-0073 (JP)**

(74) Representative: **Hoffmann Eitle
Patent- und Rechtsanwälte PartmbB
Arabellastraße 30
81925 München (DE)**

(54) **ANALYSIS METHOD, SPECIMEN ANALYZER, AND PROGRAM**

(57) Disclosed is an analysis method for analyzing an analyte in a specimen, the analysis method including: obtaining first data corresponding to an optical signal obtained from the analyte; inputting set data composed of a plurality of pieces of the first data, to an artificial intelligence algorithm capable of calculating a relevance degree between the pieces of the first data; and determining a type of the analyte by using the relevance degree.

FIG. 9

EP 4 439 571 A1

**Description**

CROSS REFERENCE TO RELATED APPLICATIONS

This application claims priority from prior Japanese Patent Application No. 2023-052355, filed on March 28, 2023, entitled "ANALYSIS METHOD, SPECIMEN ANALYZER, AND PROGRAM", the entire content of which is incorporated herein by reference.

FIELD OF THE INVENTION

The present invention relates to an analysis method for a specimen, a specimen analyzer that analyzes a specimen, and a program.

BACKGROUND OF THE INVENTION

International Publication No. WO2018/203568 describes a method in which: a result obtained by measuring cells by a flow cytometer is analyzed by an AI algorithm; and the cells are classified according to the types.

SUMMARY OF THE INVENTION

In the method described in International Publication No. WO2018/203568, information obtained from each cell in a measurement sample is compared with reference information by an AI algorithm, and the probability of the type to which the cell belongs (e.g., the probability that a certain cell is A is 70%) is calculated. As in International Publication No. WO2018/203568, in an AI algorithm that individually analyzes features of each cell, a case where it is analyzed that there are stochastically a plurality of types to which a certain cell may belong (e.g., in such a case where, with respect to a certain cell, it is analyzed that the probability of being A is 48% and the probability of being B is 48%) is assumable. In this case, there is a problem that the type of the cell becomes difficult to be identified, and as a result, the cell type classification accuracy by the AI algorithm decreases.

In view of the above problem, an object of the present invention is to provide an analysis method, a specimen analyzer, and a program that can improve the classification accuracy of the type of an analyte by an artificial intelligence algorithm.

An analysis method of the present invention relates to a method for analyzing an analyte in a specimen. The analysis method of the present invention includes: obtaining (S1, S4, S11, S111, S121, S131, S303) first data (81, 81a, 81b, 81c) corresponding to an optical signal (80a, 80b, 80c) obtained from the analyte; inputting set data (S) composed of a plurality of pieces of the first data (81, 81a, 81b, 81c), to an artificial intelligence algorithm (60) capable of calculating a relevance degree between the pieces of the first data (81, 81a, 81b, 81c); and determining (S2, S5, S14, S74, S84, S95, S112, S122, S134, S201) a type of the analyte by using the relevance degree.

According to the analysis method of the present invention, since the set data is processed by the artificial intelligence algorithm to obtain the relevance degree between the pieces of the first data, it is possible to determine which of a plurality of types certain first data is more relevant to. Therefore, it is possible to reduce the number of pieces of the first data for which it is difficult to determine which of a plurality of types the analyte belongs to, and thus, the classification accuracy of the type of the analyte by the artificial intelligence algorithm can be improved.

A specimen analyzer (4000) of the present invention relates to a specimen analyzer configured to analyze an analyte in a specimen. The specimen analyzer (4000) of the present invention includes: a measurement unit (400) configured to obtain an optical signal (80a, 80b, 80c) from the analyte; and an analysis unit (300, 600) configured to analyze set data (S) composed of a plurality of pieces of first data (81, 81a, 81b, 81c) corresponding to the optical signal (80a, 80b, 80c). The analysis unit (300, 600) analyzes the set data (S) by using an artificial intelligence algorithm (60) configured to determine a type of the analyte on the basis of a relevance degree between the pieces of the first data (81, 81a, 81b, 81c).

According to the specimen analyzer of the present invention, since the set data is processed by the artificial intelligence algorithm to obtain the relevance degree between the pieces of the first data, it is possible to determine which of a plurality of types certain first data is more relevant to. Therefore, it is possible to reduce the number of pieces of the first data for which it is difficult to determine which of a plurality of types the analyte belongs to, and thus, the classification accuracy of the type of the analyte by the artificial intelligence algorithm can be improved.

A program (3100, 6100) of the present invention relates to a program for causing a computer (300, 600) to execute a process of analyzing an analyte in a specimen. The program (3100, 6100) of the present invention includes a process of analyzing set data (S) composed of a plurality of pieces of data (81, 81a, 81b, 81c) corresponding to an optical signal (80a, 80b, 80c) obtained from the analyte. This process calculates a relevance degree between the pieces of the data (81, 81a, 81b, 81c), and analyzes the set data (S) by using an artificial intelligence algorithm (60) configured to determine a type of the analyte on the basis of the relevance degree.

According to the program of the present invention, since the set data is processed by the artificial intelligence algorithm to obtain the relevance degree between the pieces of the data, it is possible to determine which of a plurality of types certain data is more relevant to. Therefore, it is possible to reduce the number of pieces of the data for which it is difficult to determine which of a plurality of types the analyte belongs to, and thus, the classification accuracy of the type of the analyte by the artificial intelligence algorithm can be improved.

According to the present invention, the set data being a collection of a plurality of pieces of the first data corresponding to the optical signal of the analyte is processed by the AI algorithm, to analyze the relevance degree between pieces of the first data. As a result, it is possible to determine which of a plurality of types certain first data is more relevant to. Therefore, it is possible to reduce the number of pieces of the first data for which it is difficult to determine which of a plurality of types the analyte belongs to, and thus, the classification accuracy of the type of the analyte by the AI algorithm is improved.

BRIEF DESCRIPTION OF THE DRAWINGS

FIG. 1 schematically shows a configuration example of a specimen analyzer according to Embodiment 1;
FIG. 2 schematically shows a configuration example of an optical system of an FCM detection part, according to Embodiment 1;
FIG. 3 is a schematic diagram for describing generation of waveform data, according to Embodiment 1;
FIG. 4 schematically shows a method for generating analysis data from optical signals, according to Embodiment 1;
FIG. 5 schematically shows a method for analyzing analysis data by an AI algorithm, according to Embodiment 1;
FIG. 6 shows label values corresponding to cell types, according to Embodiment 1;
FIG. 7 shows a configuration example of set data composed of a plurality of pieces of analysis data, according to Embodiment 1;
FIG. 8 shows an example of a process performed on set data by a relevance degree calculation part of the AI algorithm, according to Embodiment 1;
FIG. 9 is a flowchart showing an operation example of a specimen analysis process performed by the specimen analyzer, according to Embodiment 1;
FIG. 10 shows configuration examples of set data composed of a plurality of pieces of analysis data, a correct label value, and classification information that are used in learning, according to Embodiment 1;
FIG. 11 schematically shows a method in which training data is inputted to the AI algorithm to cause the AI algorithm to learn, according to Embodiment 1;
FIG. 12 shows an outline of a calculation processing analysis and an AI analysis according to Embodiment 2;
FIG. 13 schematically shows waveform data and a representative value according to Embodiment 2;
FIG. 14 is a flowchart showing an example of a specimen analysis method according to Embodiment 2;
FIG. 15 is a flowchart showing an example in which an analysis operation is set on the basis of a rule set to an analysis unit, according to Embodiment 3;
FIG. 16 is a flowchart showing an example in which analysis is executed in accordance with a measurement item, according to Embodiment 4;
FIG. 17 is an exemplary drawing schematically showing a screen for setting the AI analysis or the calculation processing analysis for each measurement item, according to Embodiment 4;
FIG. 18 is a flowchart showing an example in which analysis is executed in accordance with a measurement order, according to Embodiment 4;
FIG. 19 is an exemplary drawing schematically showing a screen for setting an analysis mode for a measurement order, according to Embodiment 4;
FIG. 20 is a flowchart showing an example in which analysis is executed in accordance with an analysis mode of an apparatus, according to Embodiment 4;
FIG. 21 is an exemplary drawing schematically showing a screen for setting an analysis mode of the analysis unit, according to Embodiment 4;
FIG. 22 is a flowchart showing an example in which analysis is executed in accordance with the type of a measurement order, according to Embodiment 4;
FIG. 23 is an exemplary drawing schematically showing a screen for setting an analysis mode for each type of a measurement order, according to Embodiment 4;
FIG. 24 is a flowchart showing an example in which analysis is executed in accordance with a measurement item and the type of a measurement order, according to Embodiment 4;

FIG. 25 is an exemplary drawing schematically showing a screen for setting the AI analysis or the calculation processing analysis for each measurement item and each type of a measurement order, according to Embodiment 4;

FIG. 26 is a flowchart showing an example in which whether or not the AI analysis is necessary is determined on the basis of a flag provided through the calculation processing analysis, according to Embodiment 4;

FIG. 27 is an exemplary drawing schematically showing a screen for setting the AI analysis for each flag of an analysis result, according to Embodiment 4;

FIG. 28 is a flowchart showing an example in which the AI analysis is executed with respect to a specific analyte classified in the calculation processing analysis, according to Embodiment 4;

FIG. 29 is an exemplary drawing schematically showing a screen for setting whether or not to perform the AI analysis for each type of analyte, according to Embodiment 4;

FIG. 30 is a diagram describing a classification method according to the calculation processing analysis and the AI analysis executed in the process shown in FIG. 28, according to Embodiment 4;

FIG. 31 is a flowchart showing an example in which the AI analysis is executed when a specific classification has been performed in the calculation processing analysis, according to Embodiment 4;

FIG. 32 is an exemplary drawing schematically showing a screen for setting whether or not to perform the AI analysis with respect to an analyte of a specific type, according to Embodiment 4;

FIG. 33 is a block diagram showing a functional configuration of the electric/control system of the analysis unit, according to Embodiment 5;

FIG. 34 is a block diagram showing a functional configuration of the electric/control system of a measurement unit, according to Embodiment 5;

FIG. 35 is a block diagram showing a functional configuration of the fluidic system of a specimen suction part and a sample preparation part in the measurement unit in FIG. 34, according to Embodiment 5;

FIG. 36 is a block diagram showing another functional configuration of the fluidic system of the sample preparation part shown in FIG. 35, according to Embodiment 5;

FIG. 37 is a flowchart showing an example in which analysis is executed in accordance with a measurement channel, according to Embodiment 5;

FIG. 38 is an exemplary drawing schematically showing a screen for setting the AI analysis or the calculation processing analysis for each measurement channel, according to Embodiment 5;

FIG. 39 is a flowchart showing an example in which the AI analysis is executed on waveform data obtained in a WDF channel, according to Embodiment 5;

FIG. 40 is a flowchart showing an example in which, on the basis of waveform data obtained in the WDF channel, nucleated red blood cells and basophils are classified through the AI analysis, and the others are classified through the calculation processing analysis, according to Embodiment 5;

FIG. 41 is a flowchart showing an example in which the AI analysis is executed with respect to neutrophils/basophils specified through the calculation processing analysis in the WDF channel, according to Embodiment 5;

FIG. 42 is a block diagram showing a functional configuration of the electric/control system of the specimen analyzer, according to Embodiment 6;

FIG. 43 is a block diagram showing a functional configuration of the electric/control system of the analysis unit, according to Embodiment 6;

FIG. 44 is a block diagram showing another functional configuration of the electric/control system of the specimen analyzer, according to Embodiment 6;

FIG. 45 shows a configuration example of a parallel-processing processor according to Embodiment 6;

FIG. 46 schematically shows an installation example of the parallel-processing processor, according to Embodiment 6;

FIG. 47 schematically shows an installation example of the parallel-processing processor, according to Embodiment 6;

FIG. 48 schematically shows an installation example of the parallel-processing processor, according to Embodiment 6;

FIG. 49 shows another installation example of the parallel-processing processor, according to Embodiment 6;

FIG. 50 shows a configuration example of the parallel-processing processor which executes arithmetic processes, according to Embodiment 6;

FIG. 51 shows an outline of a matrix operation executed by the parallel-processing processor, according to Embodiment 6;

FIG. 52 is a conceptual diagram showing that a plurality of arithmetic processes are executed in parallel in the parallel-processing processor, according to Embodiment 6;

FIG. 53 schematically shows an outline of an arithmetic process regarding a convolution layer, according to Embodiment 6;

FIG. 54 is a flowchart showing analysis operations performed by the analysis unit and the measurement unit, according to Embodiment 6;

FIG. 55 is a flowchart showing details of the AI analysis performed in step S201 in FIG. 54, according to Embodiment 6;

FIG. 56 is a flowchart showing details of step S2011 in FIG. 55, according to Embodiment 6;

FIG. 57 is a block diagram showing another functional configuration of the electric/control system of the measurement unit, according to Embodiment 6;

FIG. 58 is a block diagram showing another functional configuration of the electric/control system of the analysis unit, according to Embodiment 6;

FIG. 59 is a block diagram showing another functional configuration of the electric/control system of the measurement unit, according to Embodiment 6;

FIG. 60 is a block diagram showing another functional configuration of the electric/control system of the analysis unit, according to Embodiment 6;

FIG. 61 is a block diagram showing another functional configuration of the electric/control system of the specimen analyzer, according to Embodiment 6;

FIG. 62 is a block diagram showing another functional configuration of the electric/control system of the measurement unit, according to Embodiment 6;

FIG. 63 is a block diagram showing another functional configuration of the electric/control system of the analysis unit, according to Embodiment 6;

FIG. 64 shows a configuration example of the parallel-processing processor which executes arithmetic processes, according to Embodiment 6;

FIG. 65 is a block diagram showing a functional configuration of the electric/control system of a computer, according to Embodiment 6;

FIG. 66 is a block diagram showing another functional configuration of the electric/control system of the measurement unit, according to Embodiment 6;

FIG. 67 is a block diagram showing another functional configuration of the electric/control system of the analysis unit, according to Embodiment 6;

FIG. 68 is a block diagram showing another functional configuration of the electric/control system of the measurement unit, according to Embodiment 6;

FIG. 69 is a block diagram showing another functional configuration of the electric/control system of the analysis unit, according to Embodiment 6;

FIG. 70 is a block diagram showing another functional configuration of the electric/control system of the measurement unit, according to Embodiment 6;

FIG. 71 is a block diagram showing another functional configuration of the electric/control system of the analysis unit, according to Embodiment 6;

FIG. 72 is a block diagram showing another functional configuration of the electric/control system of the measurement unit, according to Embodiment 6;

FIG. 73 is a block diagram showing another functional configuration of the electric/control system of the analysis unit, according to Embodiment 6;

FIG. 74 schematically shows a configuration of a waveform data analysis system according to Embodiment 7;

FIG. 75 is a block diagram showing a functional configuration of the electric/control system of a deep learning apparatus, according to Embodiment 7;

FIG. 76 is a block diagram showing a functional configuration of the electric/control system of the deep learning apparatus, according to Embodiment 7;

FIG. 77 is a flowchart showing a process performed by the deep learning apparatus, according to Embodiment 7; and

FIG. 78 is a schematic diagram showing an example of the structure of a neural network, a schematic diagram showing arithmetic operations at each node, and a schematic diagram showing arithmetic operations between nodes, according to Embodiment 7.

DETAILED DESCRIPTION

Hereinafter, outlines and embodiments of the disclosure will be described in detail with reference to the attached drawings. In the following description and drawings, the same reference characters denote the same or similar components, and description of the same or similar components will be omitted for convenience.

[Embodiment 1]

The present embodiment discloses a specimen analysis method in which analysis is performed by an artificial intelligence algorithm (AI (Artificial Intelligence) algorithm) on data obtained through measurement of a specimen, a specimen analyzer that can execute analysis, and a program. In the analysis by the AI algorithm, data is analyzed by a large number of matrix operation processes, for example. Hereinafter, analysis by the AI algorithm will be referred to as "AI analysis".

In the present embodiment, an analyte in a measurement sample is measured by a specimen analyzer. For example, on the basis of a signal regarding the analyte in the measurement sample, the specimen analyzer obtains data corresponding to the signal. The data is obtained with respect to a plurality of analytes measured by the specimen analyzer.

In the present embodiment, for example, set data being a collection of pieces of data respectively corresponding to a plurality of analytes is analyzed by the AI algorithm. The AI algorithm calculates the relevance degree between the pieces of data forming the set data, and classifies the type of the analyte corresponding to each piece of data on the basis of the calculated relevance degree. For example, when the type of an analyte is classified on the basis of a feature of data corresponding to the analyte, there may be a case where it is determined that the analyte may correspond to both of type A and type B (e.g., the probability of being type A is 45% and the probability of being type B is 45%). In such a case, it is difficult to identify which of type A and type B the analyte is.

In such a case, the AI algorithm of the present embodiment calculates (1) the relevance degree between the data and another piece of data that has been determined as type A, and (2) the relevance degree between the data and another piece of data that has been determined as type B, thereby being able to determine which relevance degree is higher. Therefore, for example, when it has been determined that the data has a higher relevance degree to the other piece of data having been determined as type A, it can be determined that the data is of an analyte of type A. Therefore, the AI algorithm of the present embodiment enables reduction of the cases where classification of the type of an analyte is difficult, and thus, the classification accuracy of an analyte is improved as compared with an algorithm that does not determine the relevance degree between pieces of data.

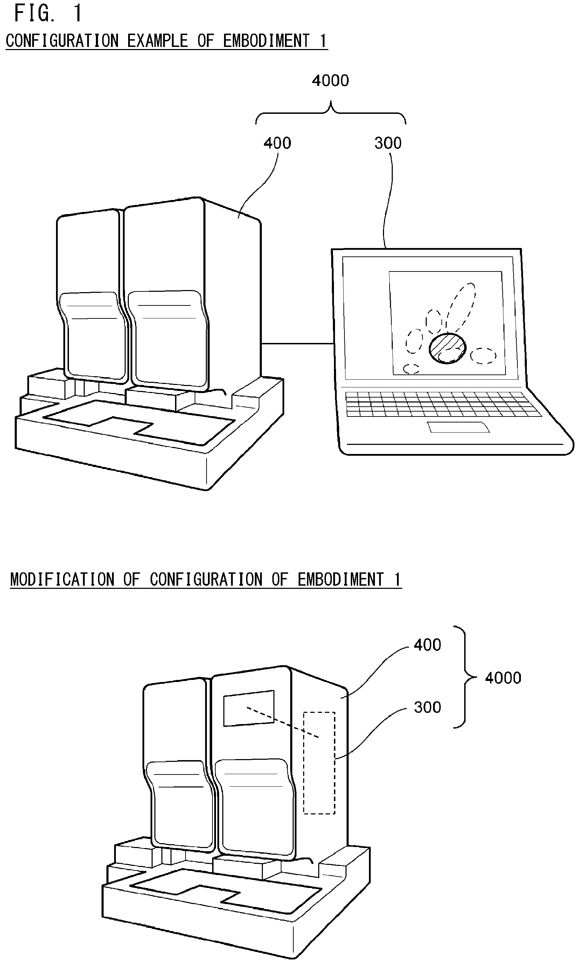

FIG. 1 schematically shows a configuration example of a specimen analyzer 4000 of Embodiment 1.

As shown in the upper part of FIG. 1, the specimen analyzer 4000 of Embodiment 1 includes a measurement unit 400 and an analysis unit 300, for example. As shown in the lower part of FIG. 1, the specimen analyzer 4000 may be implemented by the measurement unit 400 and the analysis unit 300 that are integrally formed. In this case, a touch panel-type display that allows input and display of information may be provided at the front face of the measurement unit 400.

The specimen analyzer 4000 is, for example, a blood cell analyzer, a urine analyzer, or the like. An analyte to serve as an analysis target of the specimen analyzer 4000 is, for example, a cell, a particle, a protein, or the like.

The measurement unit 400 measures a specimen and obtains data regarding the specimen. The analysis unit 300 analyzes the data obtained by the measurement unit 400. The analysis unit 300 may have a function of performing control for setting a measurement condition of a measurement sample and for execution of measurement in the measurement unit 400. In the upper part of FIG. 1, the analysis unit 300 is implemented as an apparatus (e.g., a computer) different from the measurement unit 400, and is connected to the measurement unit 400. The analysis unit 300 and the measurement unit 400 are connected to each other in a wired or wireless manner.

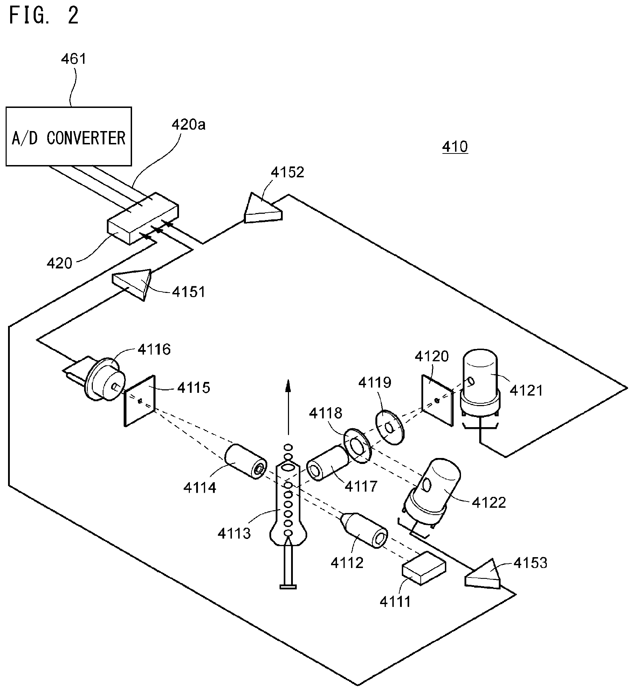

The measurement unit 400 includes an optical detection part for measuring a measurement sample prepared from a specimen. The optical detection part is, for example, an FCM detection part 410 (see FIG. 2) based on flow cytometry, and is used in measurement of a blood specimen or a urine specimen. The optical detection part applies light to a measurement sample flowing in a flow cell, thereby obtaining optical signals. For example, the optical detection part applies light to a measurement sample containing an analyte (e.g., cell or particle) flowing in the flow cell, thereby causing forward scattered light, side scattered light, and fluorescence to be generated from the analyte. A photodetector provided to the optical detection part receives the generated lights, and outputs optical signals corresponding to the intensities of the received lights. The optical signals are analog signals having waveform shapes corresponding to temporal changes in forward scattered light, side scattered light, and fluorescence.

An A/D converter 461 (see FIG. 2) provided to the optical detection part performs digital conversion on each optical signal to obtain digital data (hereinafter, referred to as "waveform data") having a waveform shape corresponding to each of analytes. For example, the waveform data in this case is used for classification of the types of white blood cells in a blood specimen, classification of the numbers of red blood cells and white blood cells in a blood specimen, classification of particles in a urine specimen, and the like.

FIG. 2 schematically shows a configuration example of an optical system of the FCM detection part 410.

Light emitted from a light source 4111 is applied via a light application lens system 4112 to each analyte in a measurement sample passing through a flow cell (sheath flow cell) 4113. Accordingly, scattered light and fluorescence are emitted from the analyte flowing in the flow cell 4113.

The wavelength of light emitted from the light source 4111 is not limited in particular, and a wavelength suitable for excitation of the fluorescent dye is selected. As the light source 4111, for example, a semiconductor laser light source, a gas laser light source such as an argon laser light source or a helium-neon laser, a mercury arc lamp, or the like is used. A semiconductor laser light source is very inexpensive when compared with a gas laser light source.

Forward scattered light generated from an analyte in the flow cell 4113 is received by a light receiving element 4116 via a condenser lens 4114 and a pin hole part 4115. The light receiving element 4116 is a photodiode, for example.

Side scattered light generated from the analyte in the flow cell 4113 is received by a light receiving element 4121 via a condenser lens 4117, a dichroic mirror 4118, a bandpass filter 4119, and a pin hole part 4120. The light receiving element 4121 is a photodiode, for example. Fluorescence generated from the analyte in the flow cell 4113 is received by a light receiving element 4122 via the condenser lens 4117 and the dichroic mirror 4118. The light receiving element 4122 is an avalanche photodiode, for example. As the light receiving elements 4116, 4121, 4122, a photomultiplier may be used.

The analog reception light signals (optical signals) outputted from the respective light receiving elements 4116, 4121, 4122 are inputted to an analog processing part 420 via amplifiers 4151, 4152, 4153, respectively. The analog processing part 420 performs processes such as noise removal and smoothing on the optical signals inputted from the FCM detection part 410, and outputs the processed optical signals to the A/D converter 461. The A/D converter 461 converts each analog optical signal from measurement start to measurement end of the measurement sample outputted from the analog processing part 420, into digital data.

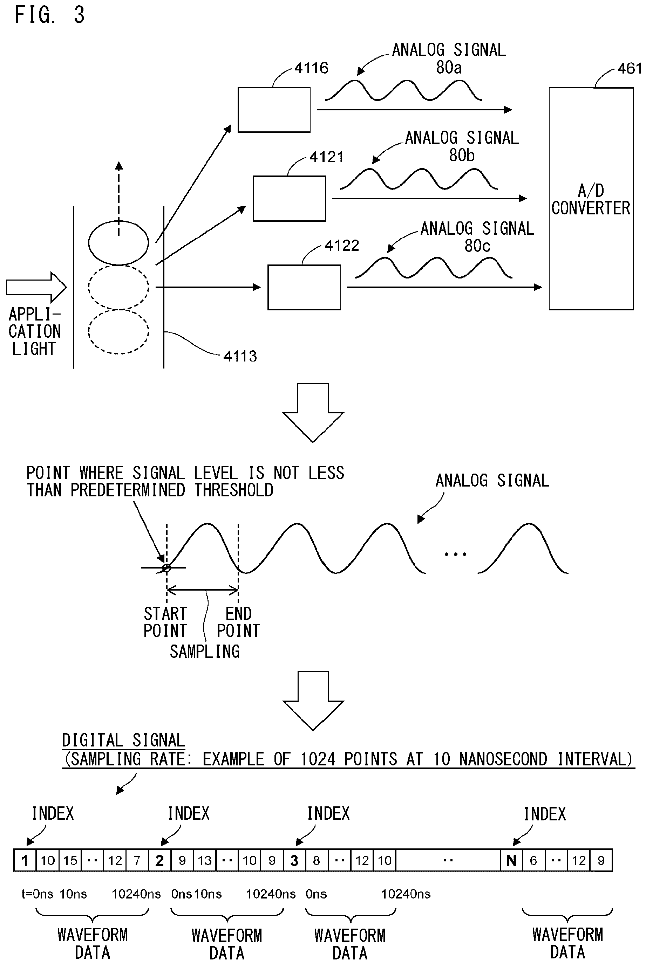

FIG. 3 is a schematic diagram for describing generation of waveform data.

As shown as an example in the upper part of FIG. 3, three types of optical signals (forward scattered light signal, side scattered light signal, and fluorescence signal) outputted from the light receiving elements 4116, 4121, 4122 are inputted via a plurality of respectively corresponding signal transmission paths 420a (see FIG. 2), to the A/D converter 461. The A/D converter 461 converts each of the optical signals inputted from the plurality of signal transmission paths 420a, into digital data. Each signal transmission path 420a may be configured to transmit an analog optical signal as a differential signal, for example.

As shown as an example in the middle part of FIG. 3, the A/D converter 461 compares the signal level of an optical signal and a predetermined threshold, and when the signal level of the optical signal becomes higher than the threshold, starts sampling of the optical signal and samples the optical signal for a predetermined sampling period. The predetermined sampling period is a fixed period that is longer than the period in which one analyte passes through a light application region of the flow cell 4113 and that is shorter than the period in which the next analyte of the analyte reaches the light application region. The A/D converter 461 samples the analog optical signal at a predetermined sampling rate (e.g., when the predetermined sampling period is 10240 nanoseconds, sampling at 1024 points at a 10 nanosecond interval, sampling at 128 points at an 80 nanosecond interval, sampling at 64 points at a 160 nanosecond interval, or the like).

By executing sampling processes on the three types of optical signals corresponding to each analyte, for example, the A/D converter 461 generates digital data (waveform data) of the forward scattered light signal, digital data (waveform data) of the side scattered light signal, and digital data (waveform data) of the fluorescence signal for each analyte. Each piece of digital data (waveform data) corresponds to one of the analytes in the specimen.

As shown as an example in the lower part of FIG. 3, through the sampling, matrix data (in the example in FIG. 3, one-dimensional array data) of which elements are values that digitally indicate analog signal levels at a plurality of time points, is obtained as the waveform data corresponding to a single analyte. The A/D converter 461 generates, for each analyte, waveform data of forward scattered light, waveform data of side scattered light, and waveform data of fluorescence. The A/D converter 461 repeats the generation of waveform data, for example, until the obtained number of analytes reaches a predetermined number, or until a predetermined time elapses after a specimen has been caused to flow in the flow cell 4113. Accordingly, digital data composed of waveform data of N analytes contained in a single specimen is obtained. The set (e.g., a set of 1024 digital values obtained every 10 nanoseconds from t=0 ns to t=10240 ns) of sampling data with respect to each analyte corresponds to waveform data.

Each piece of waveform data generated by the A/D converter 461 may be provided with an index for identifying the corresponding analyte, as shown as an example in the lower part of FIG. 3. As the indexes, for example, integers of 1 to N are provided in the sequential order of the generated pieces of waveform data, and the waveform data of forward scattered light, the waveform data of side scattered light, and the waveform data of fluorescence that have been obtained from the same analyte are each provided with an identical index. Since an identical index is provided to the pieces of waveform data that correspond to the same analyte, an AI algorithm 60 described later can: use, as one set, the waveform data of forward scattered light, the waveform data of side scattered light, and the waveform data of fluorescence that correspond to each individual analyte; analyze set data being a collection of a plurality of the sets; and classify the type of the analyte.

The waveform data generated by the A/D converter 461 is sent to the analysis unit 300. The analysis unit 300 analyzes the waveform data by the AI algorithm.

Next, an example of analysis performed by the analysis unit 300 by using the waveform data obtained by the measurement unit 400 will be described.

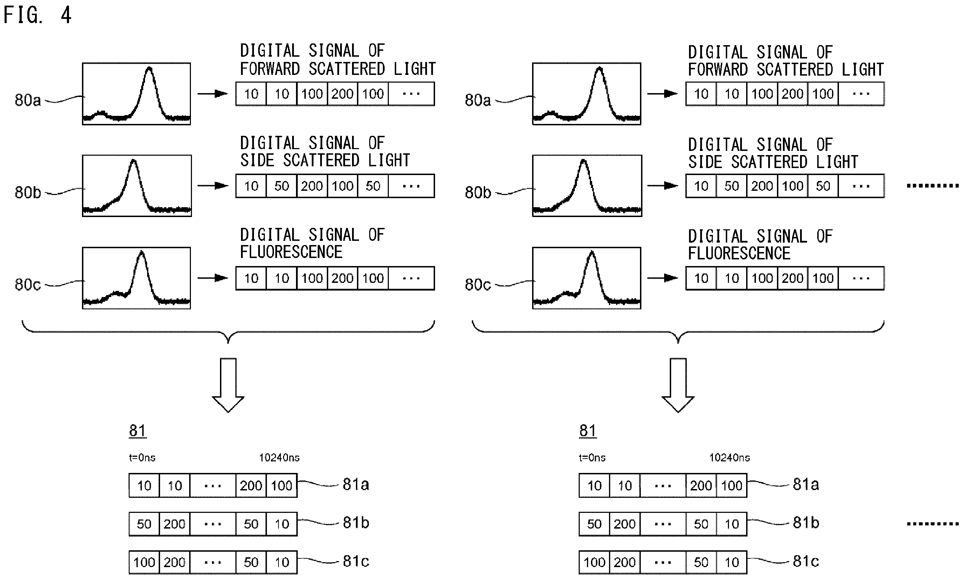

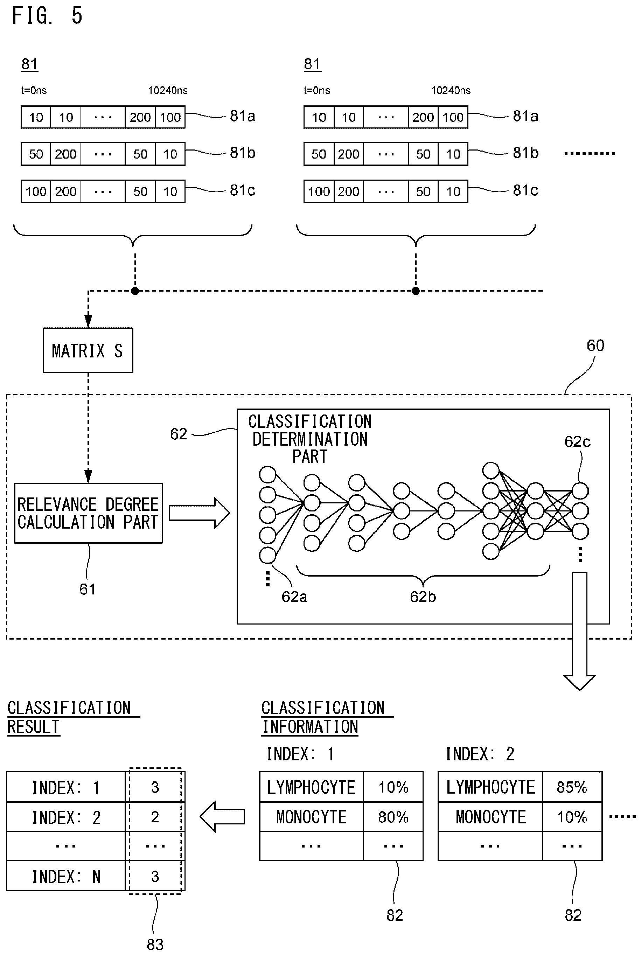

FIGS. 4 and 5 schematically show a method for analyzing waveform data of an analyte in a specimen by the AI algorithm 60. The AI algorithm 60 is, for example, an algorithm constructed on the basis of deep learning.

As shown in FIG. 4, when an analyte is measured according to flow cytometry, an optical signal 80a corresponding to forward scattered light, an optical signal 80b corresponding to side scattered light, and an optical signal 80c corresponding to fluorescence are obtained from the analyte. Pieces of waveform data 81a, 81b, 81c corresponding to the analyte are obtained on the basis of the optical signals 80a, 80b, 80c, respectively. Then, analysis data 81 composed of the waveform data 81a, 81b, 81c is generated. A plurality of pieces of the analysis data 81 are generated for each of the analytes having been measured.

As shown in FIG. 5, set data (e.g., a matrix S described later) composed of a plurality of pieces of the analysis data 81 is inputted to the AI algorithm 60 having been trained. The AI algorithm 60 includes, as functions, a relevance degree calculation part 61 and a classification determination part 62. The relevance degree calculation part 61 and the classification determination part 62 are each implemented by a neural network. In FIG. 5, the neural network of the classification determination part 62 is schematically shown.

The relevance degree calculation part 61 of the AI algorithm 60 calculates the relevance degree between a plurality of pieces of the analysis data 81 forming the set data. For example, the relevance degree calculation part 61 corrects pieces of the analysis data 81 such that features of pieces of the analysis data 81, out of the plurality of pieces of the analysis data 81 forming the set data, that have been determined to be relevant to each other are close to each other. The correction is assigning of a weight to the feature amount of data based on the relevance degree. For example, when the probability of a certain analyte being A is 48% and the probability of the analyte being B is 48% before correction is performed, the correction based on the relevance degree by the relevance degree calculation part 61 causes the probability of the analyte being A to be 70% and the probability of the analyte being B to be 25%. In other words, the feature amount of waveform data forming the analysis data 81 is reconfigured by the relevance degree calculation part 61 in consideration of the relevance degree to another piece of waveform data.

As in this example, through the calculation of the relevance degree by the relevance degree calculation part 61, for example, groups of analytes having high relevance degrees are formed, and the difference between: the feature amount of the analysis data 81 corresponding to an analyte belonging to a certain group; and the feature amount of the analysis data 81 corresponding to an analyte belonging to another group becomes large. In addition, through the calculation of the relevance degree by the relevance degree calculation part 61, for example, the feature amounts between pieces of the analysis data 81 corresponding to analytes belonging to a certain group become similar to each other. Accordingly, stochastic differentiation of the classification type of each analyte is facilitated, and as a result, the classification accuracy by the AI algorithm 60 is improved.

The classification determination part 62 of the AI algorithm 60 classifies the type of each analyte on the basis of the analysis data 81 corrected by the relevance degree calculation part 61. The corrected analysis data 81 is inputted, for example, to an input layer 62a of the neural network forming the classification determination part 62. A middle layer 62b is positioned between the input layer 62a and an output layer 62c. To the relevance degree calculation part 61, the set data (matrix S) is inputted. Meanwhile, to the input layer 62a, data is inputted for each piece of analysis data 81 forming the corrected set data, for example. For example, the analysis data 81 (e.g., three pieces of waveform data corresponding to an analyte) corresponding to a certain analyte is inputted to the input layer 62a, and the type of the analyte corresponding to this analysis data 81 is classified. This process is sequentially executed on all pieces of the analysis data 81 to serve as the target of the analysis.

Classification information 82 is outputted for each analyte (for each index of the analysis data 81) from the output layer 62c. For example, the classification information 82 includes a probability at which the analyte corresponds to each of a plurality of types. Then, a type having the highest probability is determined to be the type to which the analyte belongs, and a label value 83, which is an identifier representing the type, is outputted as a classification result. The label value 83 may include a character string indicating the type corresponding to the label value 83 or a number indicating the index.

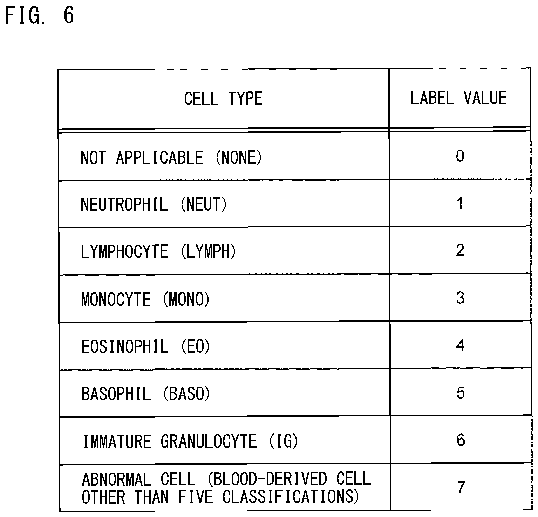

In FIG. 5, an example in which the analytes are cells in a blood specimen is shown. In this case, a label value 83 corresponding to the cell type as shown as an example in FIG. 6 is generated as a classification result. For example, when the probability that the cell type of a certain analyte is neutrophil has been determined to be the highest, the label value 83 corresponding to the analyte is determined as "1". With respect to the analyte corresponding to each of pieces of the analysis data 81 forming the set data, the label value 83 is determined.

In the present embodiment, since the analysis data 81 has been corrected according to the relevance degree, there is a gap in probability, between the type having the highest probability and the type having the second highest probability in the classification information 82. Therefore, the possibility that the type having the highest probability is the true type becomes high, and the type having the highest probability can be accurately provided as a classification result.

The set data of corrected pieces of analysis data 81 may be inputted, at one time, to the input layer 62a. In this case, the classification information 82 regarding all the types of analytes is outputted from the output layer 62c. Although the AI algorithm 60 outputs the classification information 82 from the output layer 62c, the AI algorithm 60 may be configured to output a label value 83 of each analyte from the output layer 62c.

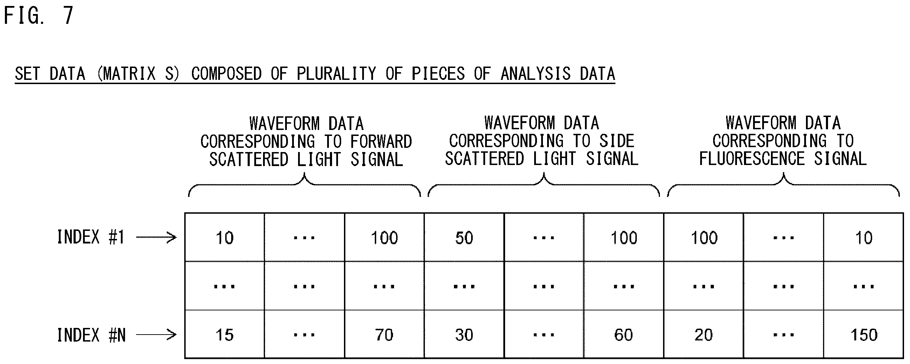

FIG. 7 shows a configuration example of the set data composed of a plurality of pieces of the analysis data 81.

The set data shown as an example in FIG. 7 is the matrix S to be inputted to the AI algorithm 60. The matrix S is a set of pieces of the analysis data 81 (first data) respectively corresponding to a plurality of analytes having been measured. The matrix S stores a plurality of pieces of the analysis data 81 respectively corresponding to N analytes. The size of the matrix S is determined according to the number of analytes to serve as the analysis target and the size of waveform data. For example, when each of the three types of waveform data is generated at 1024 sampling points and the number of analytes is N, the size of the matrix S is $N \times (1024 \times 3)$.

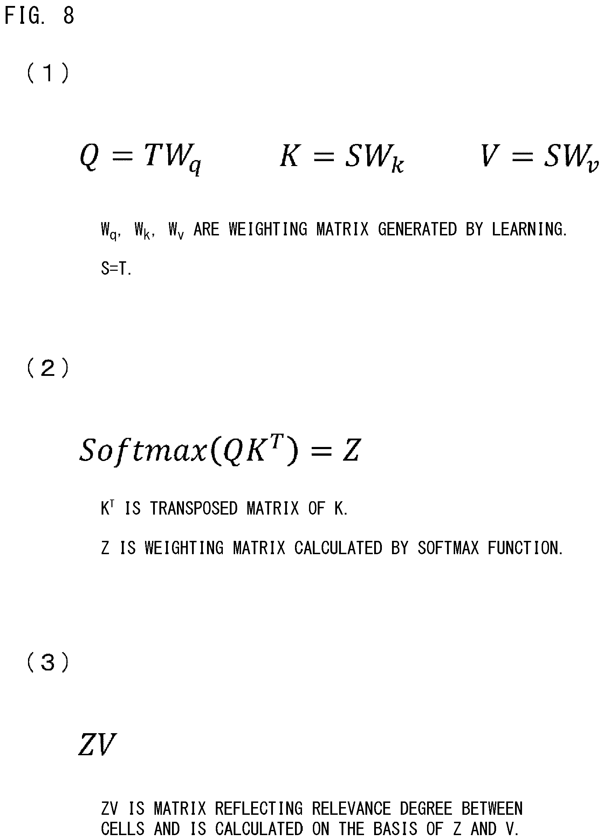

FIG. 8 shows an example of a process performed on the set data by the relevance degree calculation part 61 of the AI algorithm 60.

As shown as an example in (1) of FIG. 8, on the basis of the matrix S and a matrix T (T=S), matrixes Q, K, V are respectively calculated. Q is a query, K is a key, and V is a value. $W_q$, $W_k$, and $W_v$ in (1) of FIG. 8 are weights obtained through learning by the AI algorithm 60, respectively. $W_q$, $W_k$, and $W_v$ are each a matrix.

Next, by the formula shown as an example in (2) of FIG. 8, the relevance degree between pieces of the analysis data 81 corresponding to respective cells is calculated. The inner product of the matrix Q and a matrix $K^T$, which is a transposed matrix of the matrix K, is calculated by a Softmax function. A matrix Z is calculated by the Softmax function. The matrix Z is a matrix representing the relevance degree between pieces of the analysis data 81 corresponding to respective cells. The matrix Z is a matrix for, in accordance with the relevance degree between pieces of the analysis data 81, assigning of a weight to each piece of data. As shown in (1) and (2) of FIG. 8, the matrix Z is a matrix representing the relevance degree between each row of the matrix S and the matrix T (T=S). Each row of the matrix S and the matrix T corresponds to the analysis data 81 of each cell. Therefore, the matrix Z representing the relevance degree between each row of the matrix S and the matrix T represents the relevance degree between the pieces of the analysis data 81. When the matrix S is a matrix that has N rows as shown as an example in FIG. 7, the matrix Z is a matrix of $N \times N$.

Next, by the formula shown as an example in (3) of FIG. 8, a process of reflecting the relevance degree described above on the matrix V is executed. In the example in (3) of FIG. 8, the inner product of the matrix V and the matrix Z is calculated. The classification determination part 62 of the AI algorithm 60 classifies the cell type on the basis of the matrix data (the inner product of the matrix V and the matrix Z) calculated in (3) of FIG. 8.

The AI algorithm 60 of the present embodiment calculates the relevance degree between pieces of the analysis data 81 respectively corresponding to a plurality of analytes. The relevance degree calculation part 61 of the AI algorithm 60 corrects the inputted analysis data 81 such that the features of pieces of the analysis data 81 having a high relevance degree become closer to each other. For example, when the analysis data 81 that is near the boundary between a group A and a group B has been determined to have higher relevance to the group B, the analysis data 81 is corrected so as to be close to the group B. Through the correction based on the relevance degree, it is possible to reduce the number of pieces of the analysis data 81 for which classification of the type is difficult due to ambiguity of the group to which the analyte belongs. Accordingly, in the AI algorithm 60 of the present embodiment capable of determining the relevance degree, classification accuracy of the analyte is improved when compared with an AI algorithm that does not determine the relevance degree.

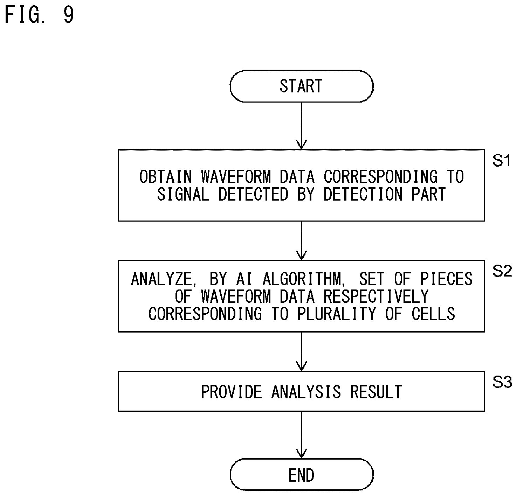

FIG. 9 is a flowchart showing an operation example of a specimen analysis process performed by the specimen analyzer 4000.

In step S1, the measurement unit 400 detects a measurement sample containing analytes by means of a detection part. The detection part is, for example, a flow cytometer capable of detecting optical signals corresponding to each analyte, and specifically is the FCM detection part 410. In step S1, the measurement unit 400 obtains digital data (waveform data) from the signals detected by the detection part. The obtained waveform data is sent to the analysis unit 300.

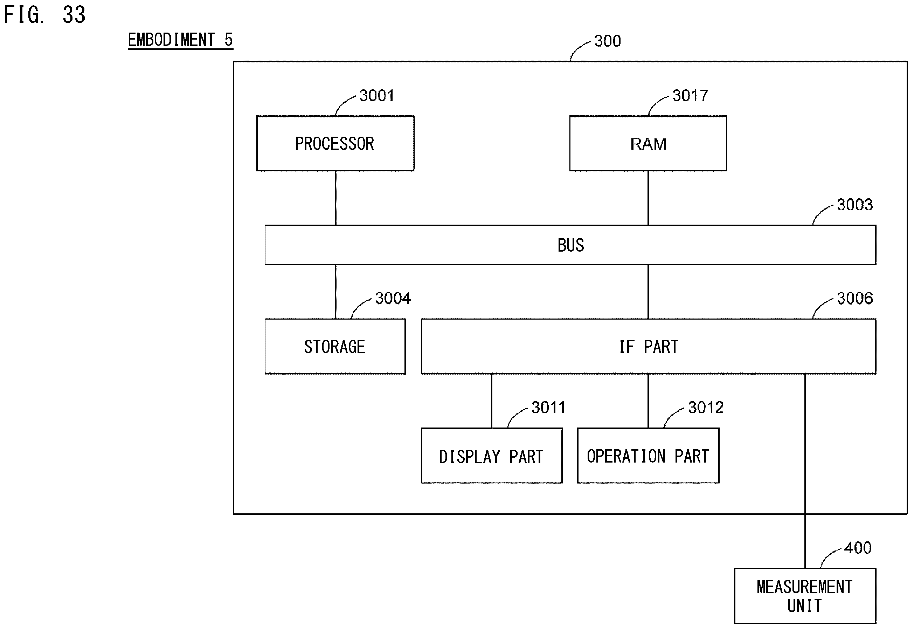

In step S2, the analysis unit 300 (specifically, for example, a processor 3001 shown in FIG. 33 described later) analyzes the waveform data obtained by the measurement unit 400. Specifically, the analysis unit 300 analyzes, by the AI algorithm 60, a set of pieces of waveform data respectively corresponding to a plurality of cells. The analysis process of the set data by the AI algorithm 60 has been described above. Accordingly, the type of the analyte is determined and the classification information 82 and a classification result (label value 83) are obtained.

In step S3, the analysis unit 300 (specifically, for example, the processor 3001 shown in FIG. 33 described later) provides an analysis result. The analysis result is the content reflecting the classification result of the type of each analyte. For example, the analysis result is the number of the analytes or the concentration thereof according to the classification result. In step S3, for example, the analysis unit 300 performs display of the analysis result to a display part, transmission of the analysis result to another computer, and the like.

Next, an example of learning by the AI algorithm 60 will be described.

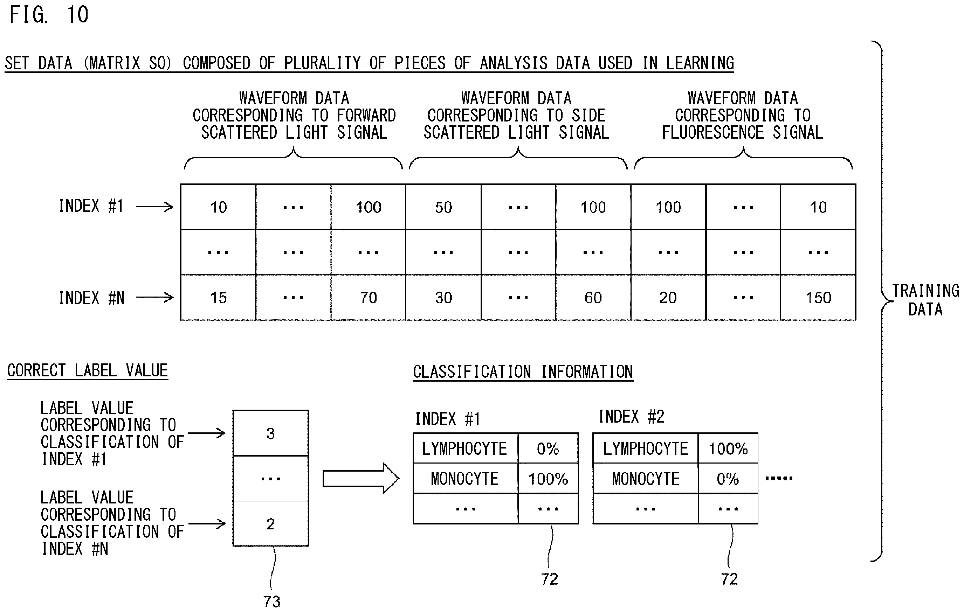

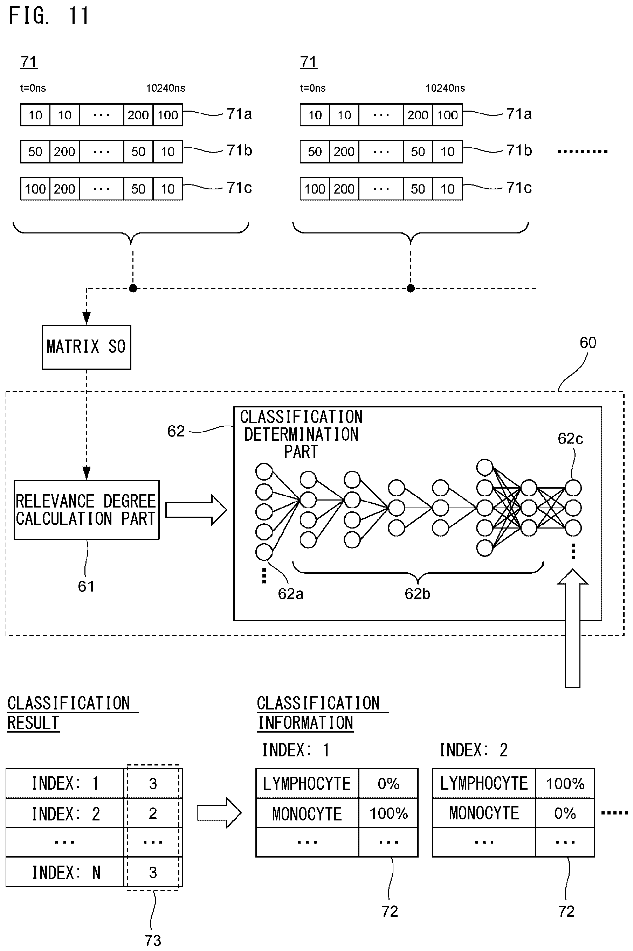

FIGS. 10 and 11 schematically show a method for causing the AI algorithm 60 to learn by using training data.

The set data shown as an example in the upper part of FIG. 10 is a matrix S0. The matrix S0 has a configuration similar to that of the matrix S described above, and is a set of pieces of analysis data 71 respectively corresponding to a plurality of analytes having been measured. A correct label value 73 shown as an example in the lower part of FIG. 10 indicates the type of each analyte corresponding to the matrix S0, for example. On the basis of the correct label value 73, classification information 72 is generated for each analyte. In the classification information 72 in this case, only the probability of the type indicated by the correct label value 73 is set to 100%. For example, the analyte of index #1 is a monocyte having a correct label value 73 of 3, and thus, the classification information 72 of the analyte is generated such that the probability of monocyte is 100% and the probabilities of the other types are 0%. Training data is formed by the matrix S0 and the classification information 72 of each analyte. Such training data is inputted to the AI algorithm 60 for learning.

The matrix S0 is generated on the basis of waveform data accumulated as measurement results of specimens in a hospital or a test facility, for example. The matrix S0 is generated on the basis of pieces of waveform data respectively corresponding to forward scattered light, side scattered light, and fluorescence of each analyte measured in a hospital or a test facility, for example.

The correct label value 73 is generated from a classification result based on analysis of waveform data forming the matrix S0, for example. The classification result in this case is generated through analysis different from the analysis performed by the AI algorithm 60. For example, on the basis of the peak value of waveform data based on side scattered light and the peak value of waveform data based on fluorescence, each analyte (e.g., cell) in the specimen is classified into a group of neutrophil, lymphocyte, monocyte, eosinophil, basophil, immature granulocyte, or abnormal cell. The correct label value 73 corresponding to the classified cell type is associated with the analysis data 71 (e.g., the three types of waveform data) of that cell.

The generation method of the training data is not limited thereto. For example, the training data may be generated by measuring, according to flow cytometry, known types of cells collected by a cell sorter, to obtain the analysis data 71 (waveform data).

For example, as shown in FIG. 11, similar to FIG. 4, each analyte is measured according to flow cytometry, whereby training waveform data 71a, 71b, 71c is obtained from the analyte. Then, the analysis data 71 composed of the waveform data 71a, 71b, 71c is generated. The pieces of the waveform data 71a, 71b, 71c are waveform data of forward scattered light, waveform data of side scattered light, and waveform data of fluorescence, respectively. The analysis data 71 is generated for each of the analytes having been measured. On the basis of the pieces of the analysis data 71 of the plurality of analytes, the matrix S0 is generated. In addition, classification results are respectively obtained for the plurality of analytes, and the correct label values 73 are generated on the basis of the classification results. Then, for each analyte, the classification information 72 is generated on the basis of the correct label value 73.

As in the example in FIG. 11, the matrix S0 is inputted to the relevance degree calculation part 61, and the classification information 72 is sequentially inputted to the output layer 62c for each analyte. The matrix S0 is processed by the AI algorithm 60, and the weighting matrix ($W_q$, $W_k$, $W_v$ in FIG. 8) of the relevance degree calculation part 61 and the weighting matrix of the classification determination part 62 are adjusted such that the classification result of each analyte by the AI algorithm 60 becomes close to the corresponding classification information 72. At this time, the matrix S0 is corrected by the relevance degree calculation part 61 in a state of including various parameters, and learning by the AI algorithm 60 is performed such that classification by the classification determination part 62 based on the corrected matrix S0 and the inputted classification information 72 become close to each other. Thus, through the input of the matrix S0 and the classification information 72 to the AI algorithm 60, both of the weighting matrixes of the relevance degree calculation part 61 and the classification determination part 62 are adjusted.

The weighting matrixes of the relevance degree calculation part 61 and the classification determination part 62 of the AI algorithm 60 are replaced with the adjusted weighting matrixes, whereby the learning result is applied to the AI algorithm 60. Then, the AI algorithm 60 having learned through the process shown as an example in FIG. 11 is set to the analysis unit 300.

Next, other configurations of Embodiment 1 will be described.

Examples of the specimen in Embodiment 1 can include a biological sample collected from a subject. For example, the specimen can include peripheral blood such as venous blood and arterial blood, urine, and a body fluid other than blood and urine. The body fluid other than blood and urine can include bone marrow aspirate, ascites, pleural effusion, cerebrospinal fluid, and the like, for example. Hereinafter, the body fluid other than blood and urine may be simply referred to as a "body fluid". The blood sample may be any blood sample that is in a state where the number of cells can be counted and the cell types can be determined. Preferably, blood is peripheral blood. Examples of blood include peripheral blood collected using an anticoagulant agent such as ethylenediamine tetraacetate (sodium salt or potassium salt), heparin sodium, or the like. Peripheral blood may be collected from an artery or may be collected from a vein.

The cell types to be determined in the present embodiment are those according to the cell types based on morphological classification, and are different depending on the type of the specimen. When the specimen is blood and the blood is collected from a healthy individual, the cell types to be determined in the present embodiment include, for example, nucleated cell such as nucleated red blood cell and white blood cell, red blood cell, platelet, and the like. Nucleated cells include, for example, neutrophils, lymphocytes, monocytes, eosinophils, and basophils. Neutrophils include, for example, segmented neutrophils and band neutrophils. When blood is collected from an unhealthy individual, nucleated cells may include, for example, at least one type selected from the group consisting of immature granulocyte and abnormal cell. Such cells are also included in the cell types to be determined in the present embodiment. Immature granulocytes can include, for example, cells such as metamyelocytes, myelocytes, promyelocytes, and myeloblasts.

The nucleated cells may include, in addition to normal cells, abnormal cells that are not contained in peripheral blood of a healthy individual. Examples of abnormal cells are cells that appear when a person has a certain disease, and such abnormal cells are tumor cells, for example. In a case of the hematopoietic system, the certain disease can be a disease selected from the group consisting of, for example: myelodysplastic syndrome; leukemia such as acute myeloblastic leukemia, acute promyelocytic leukemia, acute myelomonocytic leukemia, acute monocytic leukemia, erythroleukemia, acute megakaryoblastic leukemia, acute myeloid leukemia, acute lymphocytic leukemia, lymphoblastic leukemia, chronic myelogenous leukemia, or chronic lymphocytic leukemia; malignant lymphoma such as Hodgkin's lymphoma or non-Hodgkin's lymphoma; and multiple myeloma.

Further, abnormal cells can include, for example, cells that are not usually observed in peripheral blood of a healthy individual, such as: lymphoblasts; plasma cells; atypical lymphocytes; reactive lymphocytes; erythroblasts, which are nucleated red blood cells, such as proerythroblasts, basophilic erythroblasts, polychromatic erythroblasts, orthochromatic erythroblasts, promegaloblasts, basophilic megaloblasts, polychromatic megaloblasts, and orthochromatic megaloblasts; megakaryocytes including micromegakaryocytes; and the like.

When the specimen is urine, the cell types to be determined in the present embodiment can include, for example, epithelial cell such as that of transitional epithelium and squamous epithelium, red blood cell, white blood cell, and the like. Examples of abnormal cells include, for example, bacteria, fungi such as filamentous fungi and yeast, tumor cells, and the like.

When the specimen is a body fluid that usually does not contain blood components, such as ascites, pleural effusion, or spinal fluid, the cell types can include, for example, red blood cell, white blood cell, and large cell. The large cell here means a cell that is separated from an inner membrane of a body cavity or a peritoneum of a viscus, and that is larger than white blood cells. For example, mesothelial cells, histiocytes, tumor cells, and the like correspond to the large cell.

When the specimen is bone marrow aspirate, the cell types to be determined in the present embodiment can include, as normal cells, mature blood cells and immature hematopoietic cells. Mature blood cells include, for example, nucleated cells such as nucleated red blood cells and white blood cells, red blood cells, platelets, and the like. Nucleated cells such as white blood cells include, for example, neutrophils, lymphocytes, plasma cells, monocytes, eosinophils, and basophils. Neutrophils include, for example, segmented neutrophils and band neutrophils. Immature hematopoietic cells include, for example, hematopoietic stem cells, immature granulocytic cells, immature lymphoid cells, immature monocytic cells, immature erythroid cells, megakaryocytic cells, mesenchymal cells, and the like. Immature granulocytes can include cells such as, for example, metamyelocytes, myelocytes, promyelocytes, myeloblasts, and the like. Immature lymphoid cells include, for example, lymphoblasts and the like. Immature monocytic cells include monoblasts and the like. Immature erythroid cells include, for example, nucleated red blood cells such as proerythroblasts, basophilic erythroblasts, polychromatic erythroblasts, orthochromatic erythroblasts, promegaloblasts, basophilic megaloblasts, polychromatic megaloblasts, and orthochromatic megaloblasts. Megakaryocytic cells include, for example, megakaryoblasts and the like.

Examples of abnormal cells that can be contained in bone marrow include, for example, hematopoietic tumor cells of a disease selected from the group consisting of: myelodysplastic syndrome; leukemia such as acute myeloblastic leukemia, acute promyelocytic leukemia, acute myelomonocytic leukemia, acute monocytic leukemia, erythroleukemia, acute megakaryoblastic leukemia, acute myeloid leukemia, acute lymphocytic leukemia, lymphoblastic leukemia, chronic myelogenous leukemia, or chronic lymphocytic leukemia; malignant lymphoma such as Hodgkin's lymphoma or non-Hodgkin's lymphoma; and multiple myeloma, which have been described above, and metastasized tumor cells of a malignant tumor developed in an organ other than bone marrow.

Preferably, the optical signal is a light signal obtained as an optical response to application of light to a cell. The light signal can include at least one type selected from a signal based on light scattering, a signal based on light absorption, a signal based on transmitted light, and a signal based on fluorescence.

The signal based on light scattering can include a scattered light signal caused by light application and a light loss signal caused by light application. The scattered light signal represents a feature of an analyte in the specimen in accordance with the light reception angle of scattered light with respect to the advancing direction of application light. The forward scattered light signal is used in calculation of a representative value that indicates the size of the analyte. The side scattered light signal is used in calculation of a representative value that indicates, when the analyte in the specimen is a cell, complexity of the nucleus of the cell.

"Forward" of the forward scattered light means the advancing direction of light emitted from a light source. When the angle of application light is defined as 0°, "forward" can include a forward low angle at which the light reception angle is about 0° to 5°, and/or a forward high angle at which the light reception angle is about 5° to 20°. "Side" is not limited as long as the "side" does not overlap "forward". When the angle of application light is defined as 0°, "side" can include a light reception angle being about 25° to 155°, preferably about 45° to 135°, and more preferably about 90°. Fluorescence in the present embodiment is detected at a light reception angle similar to that of side scattered light.

The signal based on light scattering may include polarized light or depolarized light as a component of the signal. For example, when scattered light caused by application of light to an analyte in the specimen is received through a polarizing plate, only scattered light polarized at a specific angle can be received. Meanwhile, when light is applied to an analyte in the specimen through a polarizing plate, and the resultant scattered light is received through a polarizing plate that allows passage therethrough of only polarized light having an angle different from that of the polarizing plate for light application, only depolarized scattered light can be received.

A light loss signal indicates the loss amount of received light based on decrease, of the received light amount at a light receiving part, which is caused by application of light to an analyte and scattering of the light. Preferably, the light loss signal is obtained as a light loss (axial light loss) in the optical axis direction of the application light. The light loss signal can be expressed as a proportion of the received light amount at the time of flowing of a measurement sample in the flow cell, when the received light amount at the light receiving part in a state where the measurement sample is not flowing in the flow cell is defined as 100%. Similar to the forward scattered light signal, the axial light loss is used in calculation of a representative value that indicates the size of the analyte, but the signal that is obtained differs depending on whether the analyte has translucency or not.

The signal based on fluorescence may be fluorescence that is excited as a result of application of light to an analyte labeled with a fluorescent substance, or may be an intrinsic fluorescence that is generated from a non-stained analyte. When the analyte in the specimen is a cell, the fluorescent substance may be a fluorescent dye that binds to nucleic acid or membrane protein, or may be a labeled antibody obtained by modifying, with a fluorescent dye, an antibody that binds to a specific protein of the cell.

The optical signal may be obtained in a form of image data obtained by applying light to an analyte in the specimen and capturing an image of the analyte to which the light has been applied. The image data can be obtained by capturing, with an imaging element such as a TDI camera or a CCD camera, an image of each individual analyte flowing in a flow path in a flow cell. Alternatively, a specimen or a measurement sample containing cells is applied, sprayed, or spot-applied on a slide glass, and an image of the slide glass is captured by an imaging element, whereby image data of cells may be obtained.

The signal obtained from an analyte in the specimen is not limited to an optical signal, and may be an electrical signal obtained from a cell. As for the electrical signal, for example, DC current is applied to the flow cell, and change in impedance caused by an analyte flowing in the flow cell may be used as the electrical signal. The thus obtained electrical signal is used in calculation of a representative value that reflects the volume of the analyte. Alternatively, the electrical signal may be the change in impedance at the time of application of a radio frequency to an analyte flowing in the flow cell. The thus obtained electrical signal is used in calculation of a representative value that reflects conductivity of the analyte.

The signal obtained from an analyte in the specimen may be a combination of at least two types of signals out of the above-described signals. Through combination of a plurality of signals, the features of an analyte can be pleiotropically analyzed, and thus, cell classification with a higher accuracy is enabled. As for the combination, for example, at least two out of a plurality of optical signals, e.g., a forward scattered light signal, a side scattered light signal, and a fluorescence signal, may be combined. Alternatively, scattered light signals having different angles, e.g., a low angle scattered light signal and a high angle scattered light signal, may be combined. Still alternatively, an optical signal and an electrical signal may be combined, and the type and number of the signals to be combined are not limited in particular.

[Embodiment 2]

Embodiment 2 discloses a specimen analyzer, a specimen analysis method, and a program that can execute both analyses, i.e., the AI analysis and analysis that does not use the AI algorithm, on data obtained through measurement of a specimen.

In the analysis that does not use the AI algorithm, data is analyzed through calculation processing with respect to a representative value corresponding to a feature of an analyte, for example. Hereinafter, an analysis method that analyzes data through calculation processing with respect to a representative value corresponding to a feature of an analyte, without using the AI algorithm, will be referred to as "calculation processing analysis" or "non-AI analysis", for convenience. The representative value that is processed in the calculation processing analysis has a data amount smaller than that of data that is inputted to the AI algorithm in the AI analysis. In the calculation processing analysis, the data amount to be processed and the amount of arithmetic processing are smaller than those of the AI analysis, and thus, the load on the computer that performs analysis is smaller than that in the AI analysis. Accordingly, the TAT (Turn Around Time) of analysis of a measurement result can be shortened.

According to the specimen analysis method, the specimen analyzer, and the program of Embodiment 2, analysis of data obtained through measurement of a specimen is apportioned between and executed by the AI analysis and the calculation processing analysis, whereby the load on the computer that performs the analysis can be reduced.

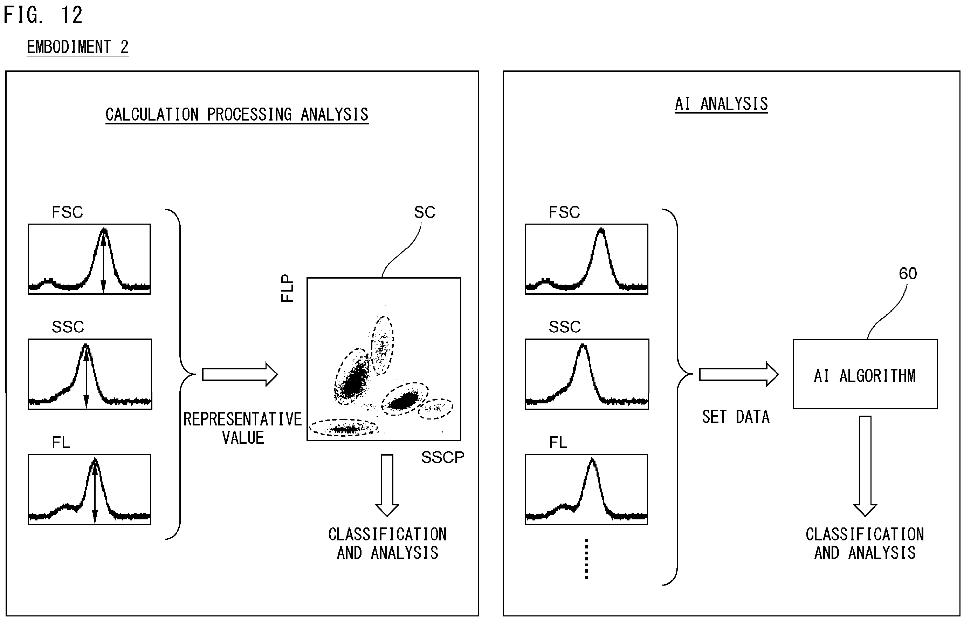

FIG. 12 shows an outline of the calculation processing analysis and the AI analysis.

In FIG. 12, the left drawing shows an outline of the calculation processing analysis, and the right drawing shows an outline of the AI analysis. FSC, SSC, and FL in FIG. 12 respectively show optical signals corresponding to the forward scattered light intensity, the side scattered light intensity, and the fluorescence obtained by the optical detection part of the measurement unit 400. In the AI analysis, a set (set data) of pieces of waveform data respectively corresponding to a plurality of analytes is inputted to the AI algorithm 60.

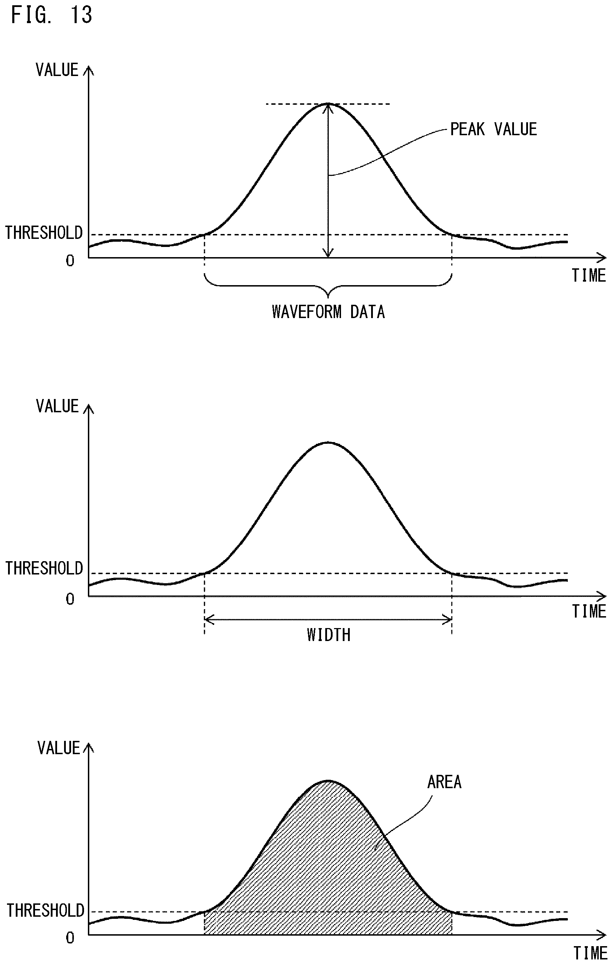

As shown in the graph in the upper part of FIG. 13, the measurement unit 400 specifies, in the digital data obtained through digital conversion of the optical signal, a region having a value greater than a predetermined threshold, as a region corresponding to an analyte in the specimen. The region having a value greater than the threshold of the digital data obtained through digital conversion of the optical signal is a region corresponding to each of analytes in the specimen. Each graph of FIG. 13 schematically shows a region (e.g., the region of the "waveform data" in the graph in the upper part of FIG. 13) that corresponds to one analyte in the specimen and that has been specified in the digital data. It should be noted that specifying a region having a value greater than the predetermined threshold may be performed on the optical signal.

The measurement unit 400 obtains, as waveform data, the region corresponding to each of the analytes in the specimen, from the digital data obtained through digital conversion of the optical signal, for example. The waveform data is obtained so as to correspond to a plurality of analytes in the specimen. Through calculation processing, the analysis unit 300 calculates a representative value, of the waveform data, corresponding to a feature of the analyte. As shown in each graph in FIG. 13, the analysis unit 300 calculates, as a representative value, an amount such as the peak value, the width, or the area of the waveform data, for example. The peak value is the maximum value of the waveform data, the width is the width in the time axis direction of the waveform data, and the area is the area surrounded by the waveform data.

In the calculation processing analysis, a representative value corresponding to a feature of each analyte is determined in advance. For example, in a case where classification and counting of blood cells being an analyte are to be performed, the representative value determined in advance in the algorithm of the calculation processing analysis is the peak value. The analysis unit 300 obtains from the waveform data a representative value determined in advance, through a predetermined calculation, and processes the representative value obtained for analyzing the analyte. With respect to each of a plurality of pieces of the waveform data obtained by the measurement unit 400, the analysis unit 300 obtains a representative value determined in advance. That is, through the predetermined calculation by the analysis unit 300, representative values (e.g., peak value) of an identical type are obtained from the plurality of respective pieces of the waveform data. The representative value determined in advance may be obtained by the measurement unit 400 and the obtained representative value and the waveform data may be transmitted to the analysis unit 300.

Meanwhile, in the AI analysis, since the AI algorithm 60 extracts a feature of the waveform data, the representative value is not determined in advance. Since the feature (i.e., a feature corresponding to an analyte) of the waveform data extracted by the AI algorithm 60 and the relevance degree between pieces of the waveform data may vary in accordance with the content learned by the AI algorithm 60, the representative value need not necessarily be determined in advance in the AI analysis. Since the AI algorithm 60 can extract various features of the waveform data in accordance with the learned content, not the representative value alone but the set data composed of the waveform data itself is inputted to the AI algorithm 60. Since the set data composed of the waveform data itself is inputted to the AI algorithm 60, the computer load for arithmetic operations of data is increased in the AI analysis and the TAT (Turn Around Time) required in the arithmetic operations is also increased, when compared with the calculation processing analysis.

As shown on the left side of FIG. 12, in the calculation processing analysis, the analysis unit 300 obtains representative values from the waveform data obtained so as to correspond to each analyte, and generates, for example, a scattergram SC on the basis of the obtained representative values. In the scattergram SC shown as an example on the left side of FIG. 12, SSCP in the horizontal axis represents the peak value of the waveform data based on side scattered light, and FLP in the vertical axis represents the peak value of the waveform data based on fluorescence. On the scattergram SC, a plurality of analytes are plotted. On the basis of the scattergram SC, the analysis unit 300 executes classification and analysis of each analyte in the specimen.

As shown on the right side of FIG. 12, in the AI analysis, the analysis unit 300 inputs, into the AI algorithm 60, the set (set data) of pieces of waveform data respectively corresponding to a plurality of analytes, and executes classification and analysis of each analyte in the specimen. The AI algorithm 60 is a learned AI algorithm, and, as described with reference to FIG. 11, is generated by inputting the set (set data) of pieces of the waveform data 71a, 71b, 71c to the AI algorithm before being trained, to cause the AI algorithm to learn. The data amount of the representative value obtained in the calculation processing analysis is smaller than that of the waveform data inputted to the AI algorithm 60 in the AI analysis.

The types of the analytes classified through the calculation processing analysis and the AI analysis are, for example, the types of blood cells in a blood specimen, the types of particles in a urine specimen, and the like. For example, the analysis unit 300 executes the AI analysis with respect to measurement items for classifying the types of white blood cells in a blood specimen, and executes the calculation processing analysis with respect to the other measurement items.

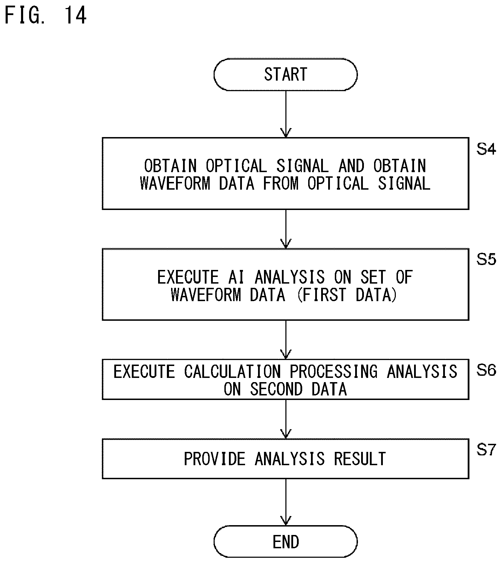

FIG. 14 is a flowchart showing an example of the specimen analysis method of Embodiment 2.

In step S4, the measurement unit 400 obtains optical signals by means of the optical detection part, and obtains waveform data from each obtained optical signal.

In step S5, the analysis unit 300 executes the AI analysis on the set of waveform data (first data) to serve as a target of the AI analysis, out of the waveform data obtained by the measurement unit 400. For example, the analysis unit 300 specifies, as the first data, waveform data that corresponds to a measurement item being a target of the AI analysis, and executes the AI analysis on the set data composed of a plurality of pieces of the specified first data.

In step S6, the analysis unit 300 executes the calculation processing analysis on waveform data (second data) to serve as a target of the calculation processing analysis, out of the waveform data obtained by the measurement unit 400. For example, the analysis unit 300 specifies, as the second data, waveform data that corresponds to a measurement item being a target of the calculation processing analysis, and executes the calculation processing analysis on the specified second data.

With respect to steps S5 and S6 above, an example case where measurement for classifying the types of white blood cells in a blood specimen is the target of the AI analysis will be described. For example, the measurement unit 400 prepares a blood specimen by using a reagent that corresponds to measurement of white blood cell classification, and measures the prepared measurement sample by means of an optical detection part based on flow cytometry. Since the measurement regarding white blood cell classification is a target of the AI analysis, the analysis unit 300 specifies the set of waveform data (first data) based on the measurement sample for white blood cell classification. The analysis unit 300 analyzes the set data of the first data by means of the AI algorithm 60, and classifies white blood cells. Meanwhile, the analysis unit 300 specifies, as the second data, waveform data based on a measurement sample other than that for white blood cell classification. The analysis unit 300 specifies a representative value corresponding to a feature of each analyte from the second data, executes the calculation processing analysis of processing the specified representative value, and classifies blood cells other than white blood cells.

In step S7, the analysis unit 300 provides obtained analysis results. In step S7, for example, the analysis unit 300 performs display of the analysis results on a display part, transmission of the analysis results to another computer, and the like.

It should be noted that, in step S4, the measurement unit 400 may obtain the optical signals by means of the optical detection part from a single measurement sample, and may obtain waveform data from each obtained optical signal. In this case, the first data and the second data are each composed of a plurality of pieces of data, and a part thereof may be the same data between the first data and the second data.

Further, in step S4, from each of a plurality of measurement samples containing a specimen collected from an identical subject, optical signals may be obtained by the optical detection part, and from each of the obtained optical signals, waveform data may be obtained. For example, the analysis unit 300 executes, in step S5, the AI analysis on the set data being a collection of a plurality of pieces of the waveform data (first data) obtained from one measurement sample, and executes, in step S6, the calculation processing analysis on the waveform data (second data) obtained from another measurement sample. The plurality of measurement samples containing a specimen collected from an identical subject may be prepared by using a reagent of the same type with each other, or may be prepared by using reagents of different types from each other.

Further, in step S4, from each of a plurality of measurement samples respectively containing specimens collected from subjects different from each other, optical signals may be obtained by the optical detection part, and from each of the obtained optical signals, waveform data may be obtained. For example, the analysis unit 300 executes, in step S5, the AI analysis on the set data being a collection of a plurality of pieces of the waveform data (first data) obtained from one measurement sample, and executes, in step S6, the calculation processing analysis on the waveform data (second data) obtained from another measurement sample. The plurality of measurement samples respectively containing specimens collected from subjects different from each other may be prepared by using a reagent of the same type with each other, or may be prepared by using reagents of different types from each other.

In Embodiment 2 above, as the calculation processing analysis, in step S6, the analysis unit 300 specifies a representative value corresponding to a feature of an analyte from the second data, and processes the specified representative value. However, the present disclosure is not limited thereto. For example, in step S4, the measurement unit 400 may obtain a representative value from the waveform data, and output the waveform data and the representative value to the analysis unit 300, and as the calculation processing analysis, in step S6, the analysis unit 300 may process the representative value obtained from the measurement unit 400.

[Embodiment 3]

In Embodiment 3, the AI analysis and the calculation processing analysis are selected on the basis of a rule set to the analysis unit 300.

The rule for selecting an analysis operation is set by a user via the analysis unit 300, for example. The user can set, to the analysis unit 300, a rule according to an operation policy of a laboratory, for example. Accordingly, in accordance with the operation policy of the laboratory, apportioning between the AI analysis and the calculation processing analysis can be changed as appropriate.

Since the rule for the analysis operation can be set, apportioning between the AI analysis and the calculation processing analysis can be flexibly changed while the load on the analysis unit 300 is reduced. For example, when the accuracy of the AI analysis has been improved as a result of causing the AI algorithm 60 to additionally learn, it is possible to set the rule such that data to serve as the target of the AI analysis is increased. Further, for example, when shortening of the TAT (Turn Around Time) of analysis of the measurement result is a priority, it is also possible to set the rule such that data to serve as the target of the calculation processing analysis is increased.

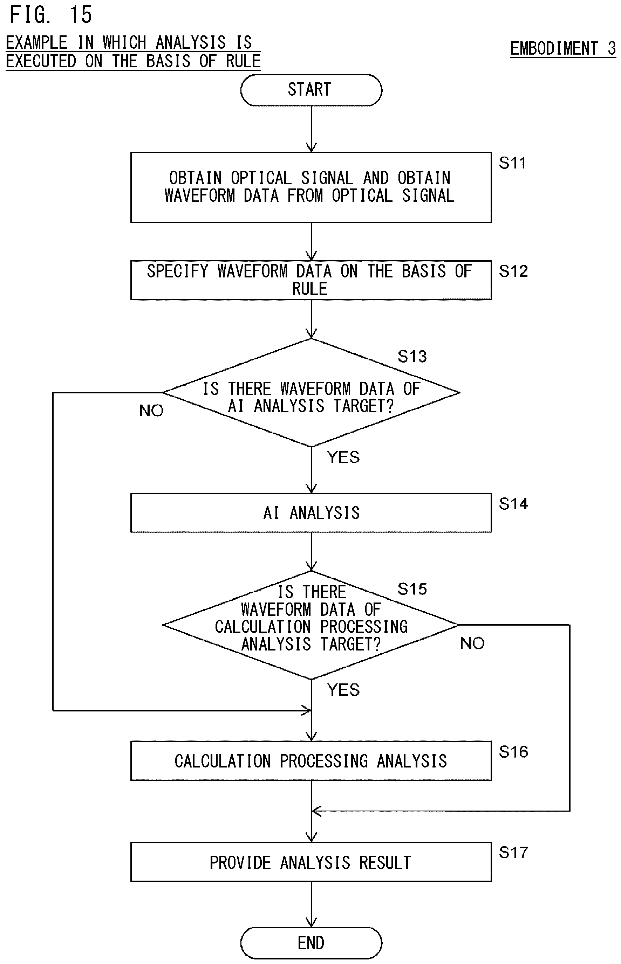

FIG. 15 is a flowchart showing an example in which an analysis operation is set on the basis of a rule set to the analysis unit 300.

In step S11, the measurement unit 400 obtains optical signals by means of the optical detection part, and obtains waveform data from each obtained optical signal. In step S12, the analysis unit 300 refers to a rule for selecting an analysis operation, and on the basis of the rule referred to, the analysis unit 300 specifies, with respect to the waveform data obtained in step S11, waveform data to serve as a target of the AI analysis and waveform data to serve as a target of the calculation processing analysis.

In step S13, the analysis unit 300 determines whether or not waveform data to serve as a target of the AI analysis is included in the waveform data specified in step S12. When the waveform data to serve as a target of the AI analysis is included (S13: YES), the analysis unit 300 executes, in step S14, the AI analysis on the set data of the waveform data (first data) to serve as a target of the AI analysis specified in step S12.

Subsequently, in step S15, the analysis unit 300 determines whether or not there is waveform data to serve as a target of the calculation processing analysis other than the waveform data having been subjected to the AI analysis. When there is waveform data (second data) to serve as a target of the calculation processing analysis (S15: YES), the analysis unit 300 executes, in step S16, the calculation processing analysis on the waveform data to serve as a target of the calculation processing analysis specified in step S12.

In some cases, the measurement unit 400 obtains, in step S12, both of waveform data to serve as a target of the AI analysis and waveform data to serve as a target of the calculation processing analysis. For example, when, in accordance with a measurement order, the measurement unit 400 has executed measurement regarding white blood cell classification and measurement regarding reticulocytes, the measurement unit 400 obtains waveform data for white blood cell classification and waveform data for reticulocyte measurement. When the white blood cell classification is the target of the AI analysis and the reticulocyte measurement is the target of the calculation processing analysis, the analysis unit 300 determines that waveform data for white blood cell classification being the target of the AI analysis is included (S13: YES), and executes the AI analysis on the set data composed of a plurality of pieces of the waveform data. Further, the analysis unit 300 determines that waveform data for reticulocyte classification being the target of the calculation processing analysis is also included (S15: YES), and executes the calculation processing analysis on the waveform data.

On the other hand, when waveform data to serve as a target of the AI analysis is not included in the waveform data obtained by the measurement unit 400 (S13: NO), the analysis unit 300 executes, in step S16, the calculation processing analysis on the waveform data to serve as a target of the calculation processing analysis specified in step S12. When the AI analysis has been executed and waveform data to serve as a target of the calculation processing analysis is not included (S15: NO), the calculation processing analysis is not executed and the process is advanced to step S17.

In step S17, the analysis unit 300 provides the analysis result.

It should be noted that the analysis unit 300 may determine, in step S13, whether or not waveform data to serve as a target of the calculation processing analysis is included, and may determine, in step S15, whether or not waveform data to serve as a target of the AI analysis is included. In this case, when the analysis unit 300 has determined, in step S13, that waveform data to serve as a target of the calculation processing analysis is included, the analysis unit 300 executes the calculation processing analysis in step S14. Further, when the analysis unit 300 has determined, in step S15, that waveform data to serve as a target of the AI analysis is included, the analysis unit 300 executes the AI analysis in step S16.

[Embodiment 4]

In Embodiment 4, various examples of apportioning between the AI analysis and the calculation processing analysis will be described.

For example, apportioning between the AI analysis and the calculation processing analysis is determined by a software program with which the analysis unit 300 executes analysis of waveform data. The software program of the analysis unit 300 specifies waveform data to serve as a target of the AI analysis and waveform data to serve as a target of the calculation processing analysis, and executes analysis. The software program is designed in accordance with a requirement (e.g., improving the TAT, increasing the analysis accuracy, etc.) regarding the test, for example.

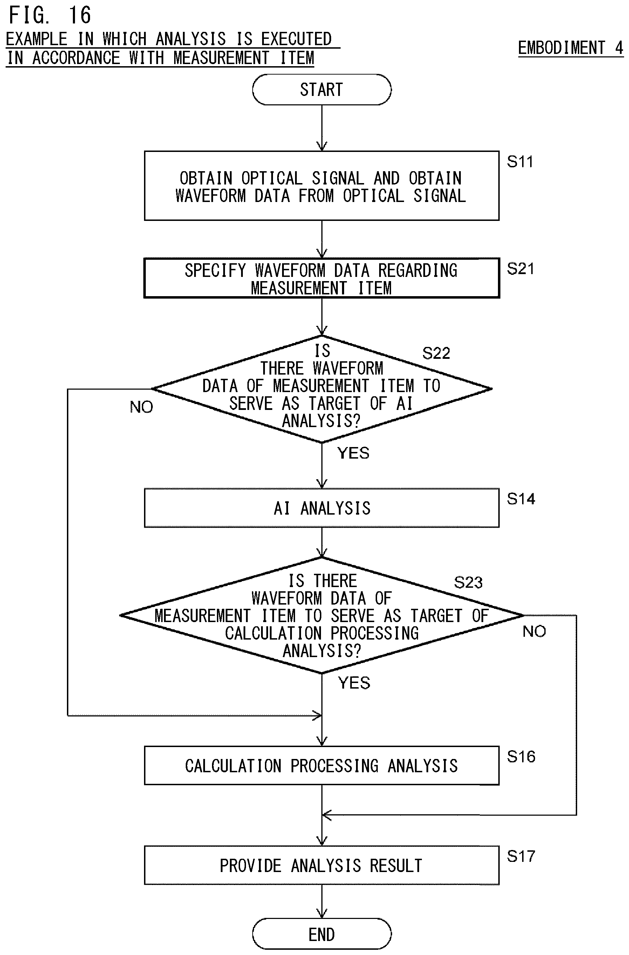

FIG. 16 is a flowchart showing an example in which analysis is executed in accordance with a measurement item.

In FIG. 16, when compared with FIG. 15, steps S21, S22, and S23 are added in place of steps S12, S 13, and S15. Hereinafter, changes from FIG. 15 will be described.

In step S21, the analysis unit 300 refers to a rule that includes which of the AI analysis and the calculation processing analysis is to be performed on the basis of measurement items, and on the basis of the rule referred to, the analysis unit 300 specifies, with respect to the waveform data obtained in step S11, waveform data of a measurement item to serve as a target of the AI analysis and waveform data of a measurement item to serve as a target of the calculation processing analysis.

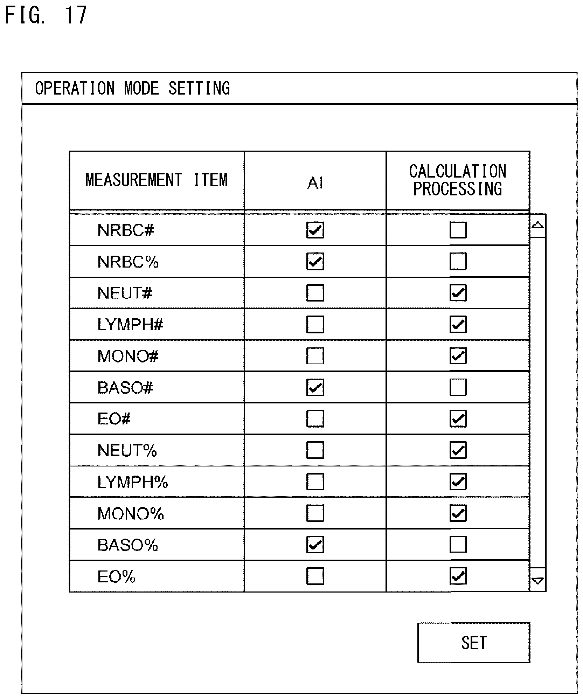

FIG. 17 is an exemplary drawing schematically showing a screen for setting the AI analysis or the calculation processing analysis for each measurement item. The measurement items shown as examples in FIG. 17 are those for a blood cell analyzer.

The screen in FIG. 17 is displayed on a display part of the analysis unit 300, for example. The screen in FIG. 17 includes, for each measurement item, a check box for setting the AI analysis and a check box for setting the calculation processing analysis. The check box for the AI analysis and the check box for the calculation processing analysis that correspond to one measurement item are configured such that only either one is selectable. The user operates a check box for each measurement item, to select which analysis out of the AI analysis and the calculation processing analysis is to be performed, and operates a setting button. Accordingly, a rule is stored in a storage of the analysis unit 300.

Although the user sets either one of the AI analysis and the calculation processing analysis with respect to each measurement item via the screen shown in FIG. 17, the screen may be configured such that both of the AI analysis and the calculation processing analysis can be set. Accordingly, the result of the AI analysis and the result of the calculation processing analysis can be compared with each other. Selection of the analysis with respect to each measurement item may be set in advance at the time of shipment of the apparatus, or only a manager may be allowed to change the setting.

With reference back to FIG. 16, in step S22, the analysis unit 300 determines whether or not waveform data of a measurement item to serve as a target of the AI analysis is included in the waveform data specified in step S21. When waveform data of a measurement item to serve as a target of the AI analysis is included (S22: YES), the analysis unit 300 executes, in step S14, the AI analysis regarding the measurement item on the set data being a collection of a plurality of pieces of the waveform data of the measurement item to serve as a target of the AI analysis specified in step S21.

For example, in accordance with a measurement order of classifying white blood cells (e.g., five classifications of neutrophil, lymphocyte, monocyte, eosinophil, and basophil), the measurement unit 400 mixes a specimen with a reagent that corresponds to the classification, to prepare a white blood cell measurement sample. The measurement unit 400 obtains optical signals that correspond to the white blood cell measurement sample by means of the optical detection part. On the basis of waveform data that corresponds to each obtained optical signal, the measurement unit 400 obtains set data being a collection of a plurality of pieces of the waveform data. When a measurement item (e.g., the count and proportion of each of neutrophils, lymphocytes, monocytes, eosinophils, and basophils) regarding the white blood cell classification is the target of the AI analysis, the analysis unit 300 executes the AI analysis on the set data composed of the set of the waveform data obtained, by the measurement unit 400, through measurement of the white blood cell measurement sample.

Subsequently, in step S23, the analysis unit 300 determines whether or not there is waveform data of a measurement item to serve as a target of the calculation processing analysis in the waveform data specified in step S21. When there is waveform data to serve as a target of the calculation processing analysis (S23: YES), the analysis unit 300 executes, in step S16, the calculation processing analysis regarding the measurement item on the waveform data to serve as a target of the calculation processing analysis specified in step S21.

For example, in accordance with a measurement order of classifying reticulocytes, the measurement unit 400 mixes a specimen with a reagent that corresponds to the classification, to prepare a reticulocyte measurement sample. The measurement unit 400 obtains optical signals that correspond to the reticulocyte measurement sample by means of the optical detection part. The measurement unit 400 obtains waveform data that corresponds to each obtained optical signal. When a measurement item (e.g., the count and proportion of reticulocytes) regarding classification of reticulocytes is the target of the calculation processing analysis, the analysis unit 300 executes the calculation processing analysis on the waveform data obtained, by the measurement unit 400, through measurement of the reticulocyte measurement sample.

Not limited to a blood cell analyzer, the specimen analyzer 4000 may be a urine analyzer. For example, when the specimen analyzer 4000 is a urine analyzer, the analysis unit 300 executes the AI analysis with respect to some of measurement items and performs the calculation processing analysis with respect to the remaining measurement items.

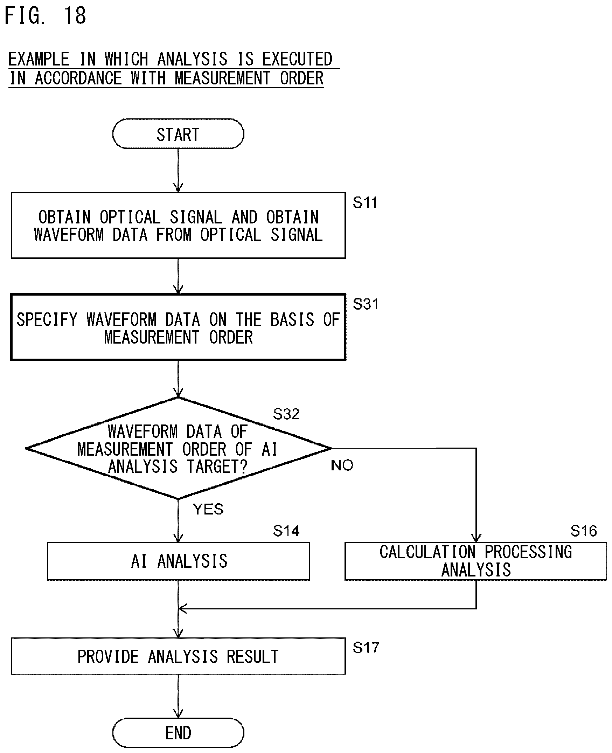

FIG. 18 is a flowchart showing an example in which analysis is executed in accordance with a measurement order.

In FIG. 18, when compared with FIG. 15, steps S31 and S32 are added in place of steps S12 and S13, and step S15 is deleted. Hereinafter, changes from FIG. 15 will be described.

In step S31, on the basis of a measurement order, the analysis unit 300 specifies which of waveform data to serve as a target of the AI analysis and waveform data to serve as a target of the calculation processing analysis the waveform data obtained in step S11 is. For example, the analysis mode for a measurement order is either an AI analysis mode or a calculation processing analysis mode, and is stored in a storage of the analysis unit 300 in association with the measurement order.

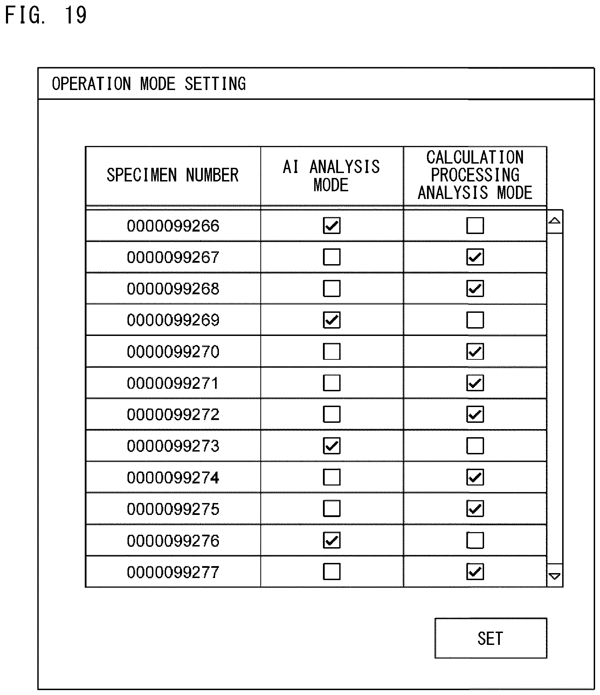

FIG. 19 is an exemplary drawing schematically showing a screen for setting an analysis mode for a measurement order.

The screen in FIG. 19 is displayed on a display part of the analysis unit 300, for example. In the screen in FIG. 19, each row corresponds to a measurement order identified by a specimen number. The screen in FIG. 19 includes, for each measurement order, a check box for setting the AI analysis mode and a check box for setting the calculation processing analysis mode. The user operates a check box for each measurement order, to select which analysis out of the AI analysis and the calculation processing analysis is to be performed by the analysis unit 300, and operates a setting button. Accordingly, an analysis mode is stored in association with the measurement order, in a storage of the analysis unit 300.

Not limited to the configuration in which the analysis mode that is associated with each measurement order is set by the user via the analysis unit 300, the analysis mode may be set in advance at the time of setting of a measurement order at a host computer or the like.

With reference back to FIG. 18, in step S32, the analysis unit 300 determines whether or not the waveform data specified in step S31 is waveform data of a measurement order being a target of the AI analysis. When the specified waveform data is waveform data of a measurement order being a target of the AI analysis (S32: YES), the analysis unit 300 performs, in step S14, the AI analysis on the set data being a collection of a plurality of pieces of the waveform data of the measurement order. On the other hand, when the specified waveform data is waveform data of a measurement order being a target of the calculation processing analysis (S32: NO), the analysis unit 300 performs, in step S16, the calculation processing analysis on the waveform data of the measurement order.

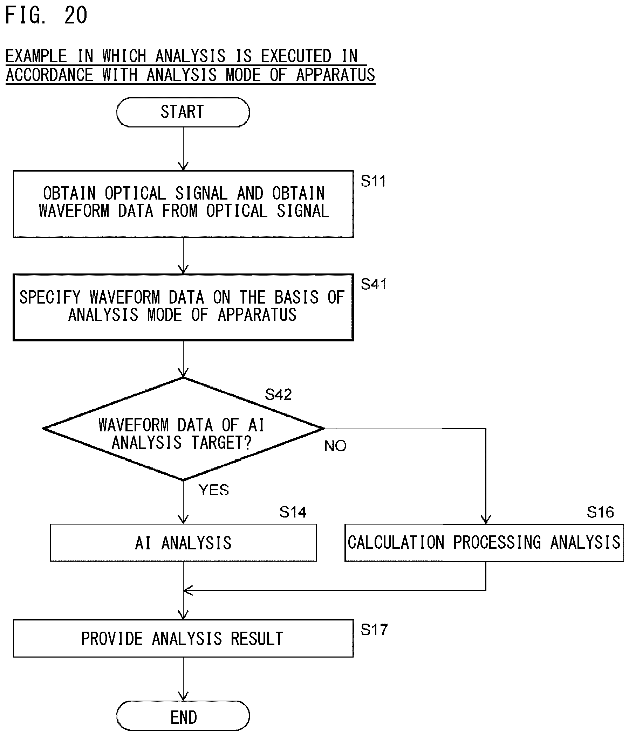

FIG. 20 is a flowchart showing an example in which analysis is executed in accordance with the analysis mode of the apparatus.

In FIG. 20, when compared with FIG. 15, steps S41 and S42 are added in place of steps S12 and S13, and step S15 is deleted. Hereinafter, changes from FIG. 15 will be described.

In step S41, the analysis unit 300 refers to a rule including the analysis mode of the analysis unit 300, and on the basis of the rule referred to, the analysis unit 300 specifies which of waveform data to serve as a target of the AI analysis and waveform data to serve as a target of the calculation processing analysis the waveform data obtained in step S11 is. When the AI analysis mode has been set in the above rule, the waveform data obtained while the AI analysis mode has been set serves as the target of the AI analysis, and when the calculation processing analysis mode has been set in the above rule, the data obtained while the calculation processing analysis mode has been set serves as the target of the calculation processing analysis.

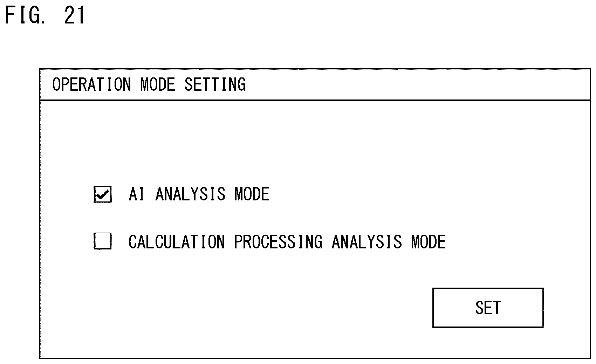

FIG. 21 is an exemplary drawing schematically showing a screen for setting an analysis mode of the analysis unit 300.

The screen in FIG. 21 is displayed on a display part of the analysis unit 300, for example. The screen in FIG. 21 includes a check box for setting the AI analysis mode and a check box for setting the calculation processing analysis mode, to the analysis unit 300. The user operates a check box, to select which analysis out of the AI analysis and the calculation processing analysis is to be performed by the analysis unit 300, and operates a setting button. Accordingly, a rule is stored in a storage of the analysis unit 300.

With reference back to FIG. 20, in step S42, the analysis unit 300 determines whether or not the waveform data specified in step S41 is waveform data to serve as a target of the AI analysis. When the specified waveform data is data of a target of the AI analysis (S42: YES), i.e., the analysis mode of the analysis unit 300 is the AI analysis mode, the analysis unit 300 performs, in step S14, the AI analysis on the set data being a collection of a plurality of pieces of the waveform data. On the other hand, when the specified waveform data is waveform data of a target of the calculation processing analysis (S42: NO), i.e., the analysis mode of the analysis unit 300 is the calculation processing analysis mode, the analysis unit 300 performs, in step S16, the calculation processing analysis on the waveform data.

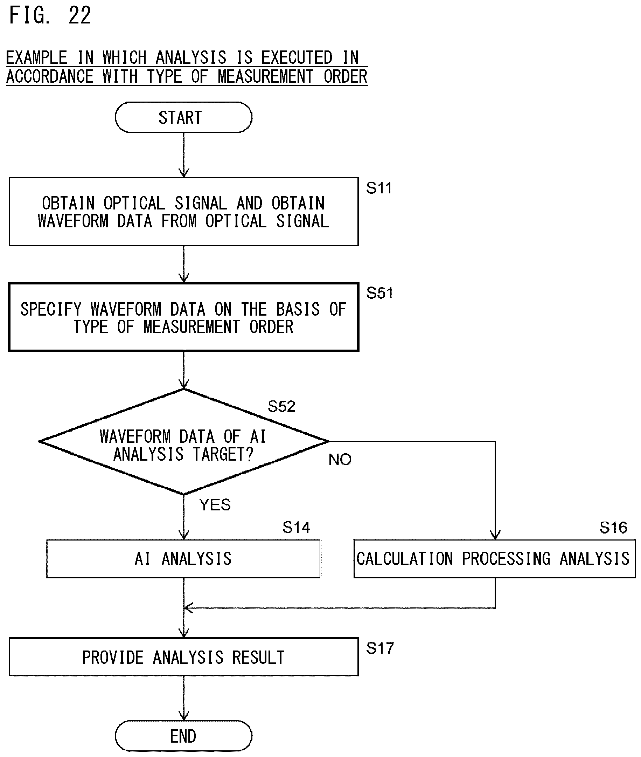

FIG. 22 is a flowchart showing an example in which analysis is executed in accordance with the type of a measurement order.

In FIG. 22, when compared with FIG. 15, steps S51 and S52 are added in place of steps S12 and S13, and step S15 is deleted. Hereinafter, changes from FIG. 15 will be described.

In step S51, the analysis unit 300 refers to a rule that includes an analysis mode that corresponds to the type of the measurement order, and on the basis of the type of the measurement order and the rule referred to, the analysis unit 300 specifies which of waveform data to serve as a target of the AI analysis and waveform data to serve as a target of the calculation processing analysis the waveform data obtained in step S11 is. The type of the measurement order includes "Normal" corresponding to normal measurement such as an initial test, "Rerun" corresponding to a re-test in which a measurement item identical to that of the initial test is set, and "Reflex" corresponding to a re-test in which the measurement item has been changed from that of the initial test. In the above rule, for each type of the measurement order, either one of the AI analysis mode and the calculation processing analysis mode is set.

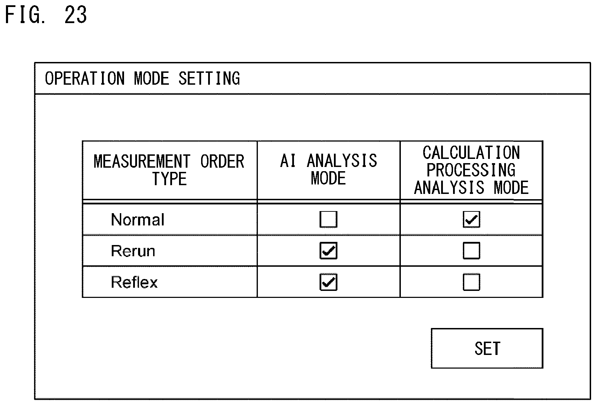

FIG. 23 is an exemplary drawing schematically showing a screen for setting an analysis mode for each type of the measurement order.

The screen in FIG. 23 is displayed on a display part of the analysis unit 300, for example. The screen in FIG. 23 includes, for each type (Normal, Rerun, Reflex) of the measurement order, a check box for setting the AI analysis mode and a check box for setting the calculation processing analysis mode. The user operates a check box, to select which analysis out of the AI analysis and the calculation processing analysis is to be performed for each type of the measurement order, and operates a setting button. Accordingly, a rule is stored in a storage of the analysis unit 300.

Not limited to the configuration in which the analysis mode that is associated with the type of each measurement order is set by the user via the analysis unit 300, the analysis mode may be set in advance in accordance with the type of the measurement order at a host computer or the like.

With reference back to FIG. 22, in step S52, the analysis unit 300 determines whether or not the waveform data specified in step S51 is waveform data to serve as a target of the AI analysis. When the specified waveform data is waveform data of a target of the AI analysis (S52: YES), i.e., the analysis mode according to the type of the measurement order is the AI analysis mode, the analysis unit 300 performs, in step S14, the AI analysis on the set data being a collection of a plurality of pieces of the waveform data. On the other hand, when the specified waveform data is waveform data of a target of the calculation processing analysis (S52: NO), i.e., the analysis mode according to the type of the measurement order is the calculation processing analysis mode, the analysis unit 300 performs, in step S16, the calculation processing analysis on the waveform data.

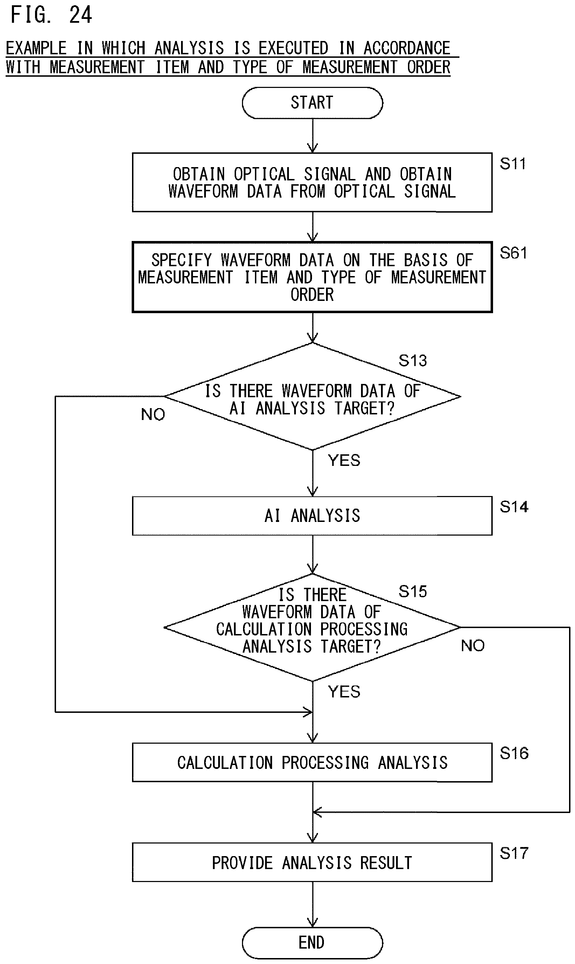

FIG. 24 is a flowchart showing an example in which analysis is executed in accordance with a measurement item and the type of a measurement order.

In FIG. 24, when compared with FIG. 15, step S61 is added in place of step S12. Hereinafter, changes from FIG. 15 will be described.

In step S61, the analysis unit 300 refers to a rule for selecting an analysis operation, and on the basis of the measurement item and the type of the measurement order, the analysis unit 300 specifies, with respect to the waveform data obtained in step S 11, waveform data to serve as a target of the AI analysis and waveform data to serve as a target of the calculation processing analysis.

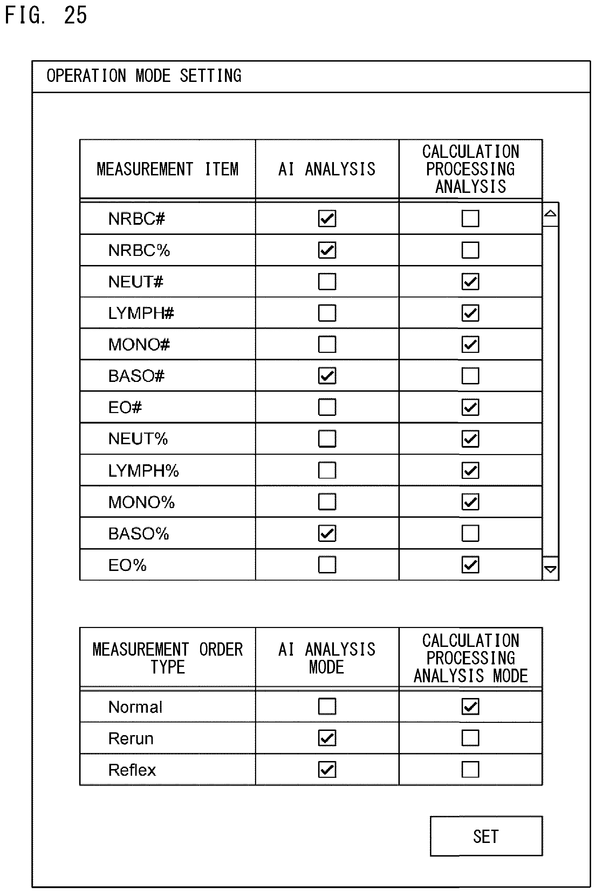

FIG. 25 is an exemplary drawing schematically showing a screen for setting the AI analysis or the calculation processing analysis for each measurement item and each type of the measurement order.

The screen in FIG. 25 is displayed on a display part of the analysis unit 300, for example. The screen in FIG. 25 includes, for each measurement item, a check box for setting the AI analysis and a check box for setting the calculation processing analysis, and includes, for each type (Normal, Rerun, Reflex) of the measurement order, check boxes for setting either one of the AI analysis and the calculation processing analysis. The user operates, for each measurement item, a check box in the list in the upper part, to select which analysis out of the AI analysis and the calculation processing analysis is to be performed, operates, for each type of the measurement order, a check box in the list in the lower part, to select which analysis out of the AI analysis and the calculation processing analysis is to be performed, and operates a setting button. Accordingly, a rule is stored in a storage of the analysis unit 300.

In a case where setting has been performed as shown in FIG. 25, when the type of the measurement order is "Normal", the analysis unit 300 specifies the waveform data obtained in the measurement unit 400, as a target of the calculation processing analysis. For example, when the type of the measurement order is "Normal", irrespective of the setting of the analysis for each measurement item, the calculation processing analysis is executed with respect to all of the measurement items based on the measurement order. When the type of the measurement order is "Rerun" or "Reflex", the analysis unit 300 sets the waveform data obtained in the measurement unit 400, as a target of the AI analysis or the calculation processing analysis, in accordance with the analysis setting set for each measurement item. For example, when the type of the measurement order is "Rerun" and "Reflex", the measurement items regarding nucleated red blood cells (NRBC) and basophils (BASO) serve as targets of the AI analysis, and the other measurement items serve as targets of the calculation processing analysis.

With reference back to FIG. 24, in step S13, the analysis unit 300 determines whether or not data to serve as a target of the AI analysis is included in the waveform data specified in step S61. When there is waveform data to serve as a target of the AI analysis (S13: YES), the analysis unit 300 executes, in step S14, the AI analysis on the set data being a collection of a plurality of pieces of the waveform data to serve as a target of the AI analysis specified in step S61.

Subsequently, in step S15, the analysis unit 300 determines whether or not waveform data to serve as a target of the calculation processing analysis is included in the waveform data specified in step S61. When there is waveform data to serve as a target of the calculation processing analysis (S15: YES), the analysis unit 300 executes, in step S16, the calculation processing analysis on the waveform data to serve as a target of the calculation processing analysis specified in step S61.

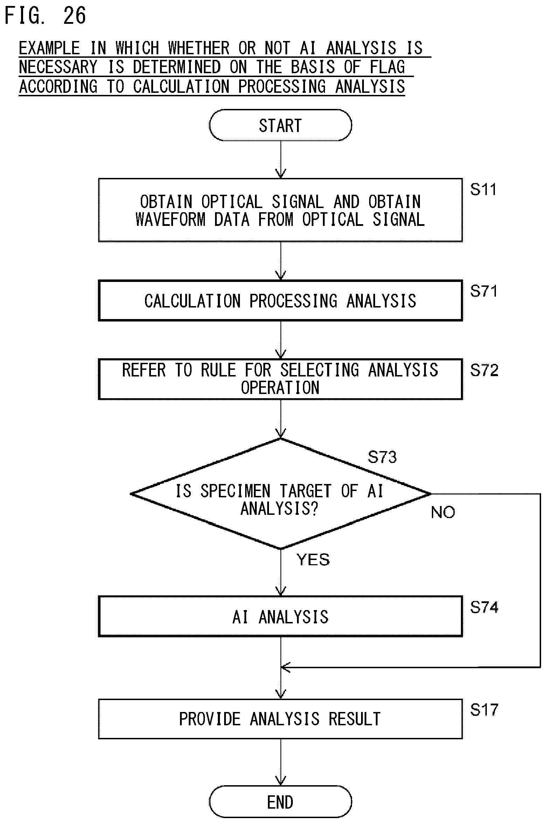

FIG. 26 is a flowchart showing an example in which whether or not the AI analysis is necessary is determined on the basis of a flag provided through the calculation processing analysis.

In FIG. 26, when compared with FIG. 15, steps S71 to S74 are added after step S11, and steps S12 to S16 are deleted. Hereinafter, changes from FIG. 15 will be described.

In step S71, the analysis unit 300 executes the calculation processing analysis on the waveform data obtained in step S11, and sets a flag that suggests an abnormality regarding an analyte in the specimen, on the basis of the result of the calculation processing analysis. The flag is, for example, a flag indicating that a predetermined abnormal cell has been detected, a flag indicating that the count value of predetermined blood cells is an abnormal value, or the like. In step S72, the analysis unit 300 refers to a rule that includes whether or not to perform the AI analysis on an analysis result having a flag.

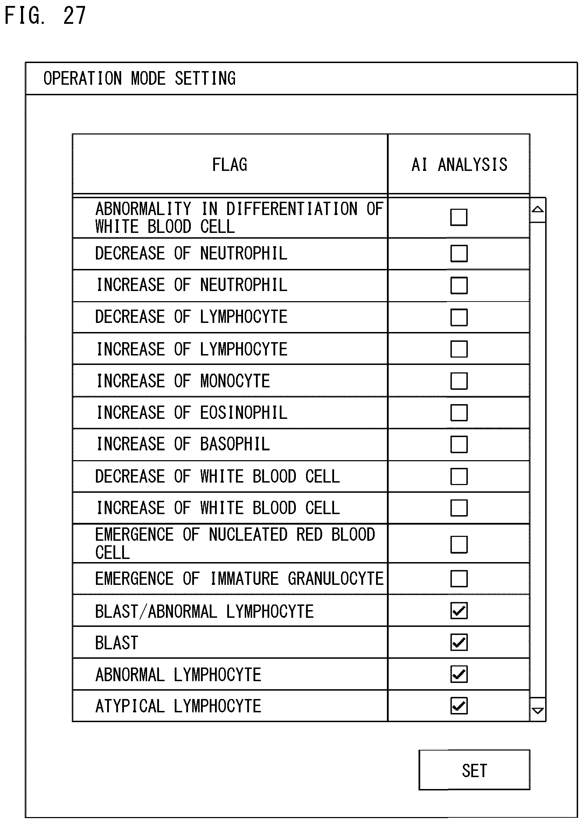

FIG. 27 is an exemplary drawing schematically showing a screen for setting the AI analysis for each flag of an analysis result.

The screen in FIG. 27 is displayed on a display part of the analysis unit 300, for example. The screen in FIG. 27 includes, for each flag provided through the calculation processing analysis to an analysis result, a check box for setting the AI analysis. When the specimen analyzer 4000 is a blood cell analyzer, the flag provided through the calculation processing analysis to an analysis result includes decrease of blood cells, increase of blood cells, emergence of an abnormal cell, and the like. The user operates a check box for each flag, to select whether to perform the AI analysis, and operates a setting button. Accordingly, a rule is stored in a storage of the analysis unit 300.

When the check box of a flag is on, the AI analysis is executed on the waveform data that corresponds to the analysis result. In the example shown in FIG. 27, the check boxes for the analysis results of blast/abnormal lymphocyte, blast, abnormal lymphocyte, and atypical lymphocyte are on, and when flags indicating that these blood cells are present have been set through the calculation processing analysis, the AI analysis is executed with respect to these blood cells.

With reference back to FIG. 26, in step S73, on the basis of the flag provided to the analysis result obtained in step S71 and the rule referred to in step S72, the analysis unit 300 determines whether or not the specimen is an AI analysis target. When the specimen is an AI analysis target (S73: YES), the analysis unit 300 executes, in step S74, the AI analysis on the set data being a collection of a plurality of pieces of each waveform data. For example, when a flag indicating that a blast has been detected through the calculation processing analysis has been issued, the AI analysis is, according to the rule shown as an example in FIG. 27, executed on the set data being a collection of a plurality of pieces of each waveform data obtained in step S11.

On the other hand, when the specimen is not an AI analysis target (S73: NO), the analysis unit 300 skips step S74.

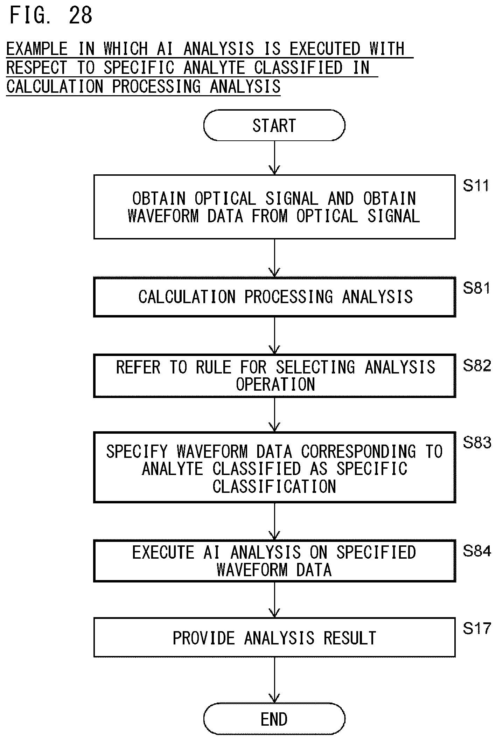

FIG. 28 is a flowchart showing an example in which the AI analysis is executed with respect to a specific analyte classified in the calculation processing analysis.

In FIG. 28, when compared with FIG. 15, steps S81 to S84 are added after step S11, and steps S12 to S16 are deleted. Hereinafter, changes from FIG. 15 will be described.

In step S81, the analysis unit 300 executes the calculation processing analysis on the waveform data obtained in step S11, to classify the analytes. In step S82, the analysis unit 300 refers to a rule that includes whether or not to perform the AI analysis for each type of analyte.

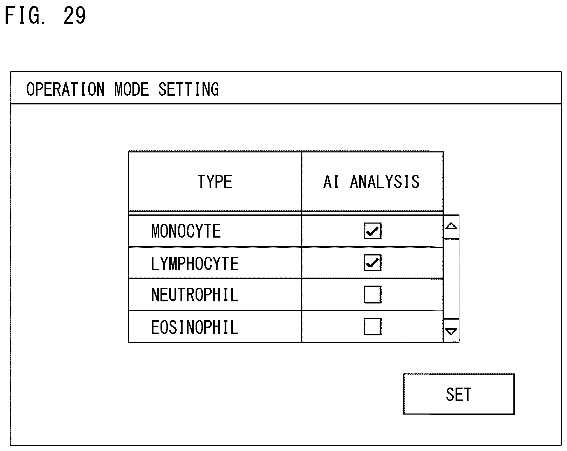

FIG. 29 is an exemplary drawing schematically showing a screen for setting whether or not to perform the AI analysis for each type of analyte.

The screen in FIG. 29 is displayed on a display part of the analysis unit 300, for example. The screen in FIG. 29 includes, for each type of analyte, a check box for setting the AI analysis. When the specimen analyzer 4000 is a blood cell analyzer, the types to be classified through the calculation processing analysis include eosinophil, neutrophil, lymphocyte, monocyte, and the like. The user operates a check box, to select whether or not to perform the AI analysis for each type of analyte, and operates a setting button. Accordingly, a rule is stored in a storage of the analysis unit 300.

When the check box for a type of an analyte is on, the AI analysis is executed on the set data being a collection of a plurality of pieces of the waveform data classified as the type. In the example shown in FIG. 29, the AI analysis is executed with respect to monocytes and lymphocytes.

With reference back to FIG. 28, in step S83, on the basis of the types of the analytes classified in step S81 and the rule referred to in step S82, the analysis unit 300 specifies waveform data that corresponds to the analyte (e.g., in the case of the rule shown in FIG. 29, monocyte and lymphocyte) classified as a specific classification. In step S84, the analysis unit 300 executes the AI analysis on the set data being a collection of a plurality of pieces of the specified waveform data.

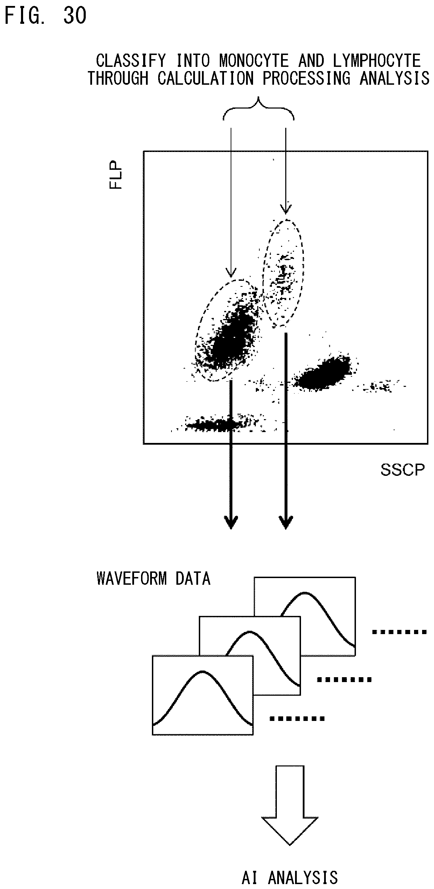

FIG. 30 is a diagram describing a classification method according to the calculation processing analysis and the AI analysis executed in the process shown in FIG. 28.

In the calculation processing analysis, a scattergram of which axes represent two types of representative values calculated from waveform data is used. For example, as shown in FIG. 30, when the AI analysis has been set to be executed with respect to monocytes and lymphocytes, plots in regions surrounded by broken lines that correspond to monocytes and lymphocytes on the scattergram are specified through the calculation processing analysis in step S81. Then, in step S83, waveform data that corresponds to each plot in each surrounded region is specified, and the AI analysis is executed, in step S84, on the set data being a collection of a plurality of pieces of the specified waveform data.

With reference back to FIG. 28, in step S17, the analysis unit 300 provides analysis results obtained through the calculation processing analysis and the AI analysis. At this time, the analysis unit 300 replaces, out of the analysis results obtained through the calculation processing analysis in step S81, the analysis result of the type having served as a target of the AI analysis, with an analysis result obtained through the AI analysis, and provides the analysis result. The analysis result obtained through the calculation processing analysis and the analysis result obtained through the AI analysis may be provided in combination.

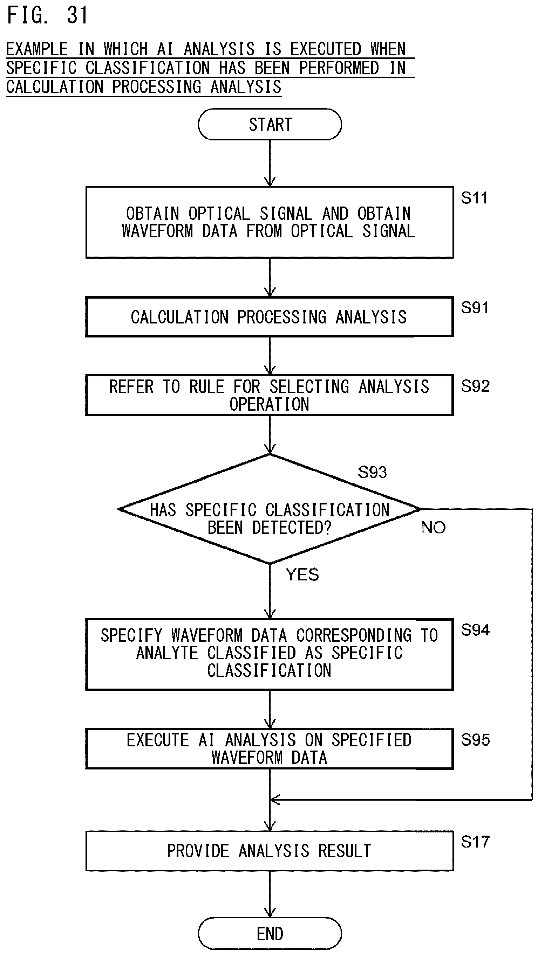

FIG. 31 is a flowchart showing an example in which the AI analysis is executed when a specific classification has been performed in the calculation processing analysis.

In FIG. 31, when compared with FIG. 15, steps S91 to S95 are added after step S11, and steps S12 to S16 are deleted. Hereinafter, changes from FIG. 15 will be described.

In step S91, the analysis unit 300 executes the calculation processing analysis on the waveform data obtained in step S11, to classify the analytes. In step S92, the analysis unit 300 refers to a rule that includes whether or not to perform the AI analysis with respect to an analyte of a specific type, e.g., a cell that is not present in peripheral blood of a healthy individual.

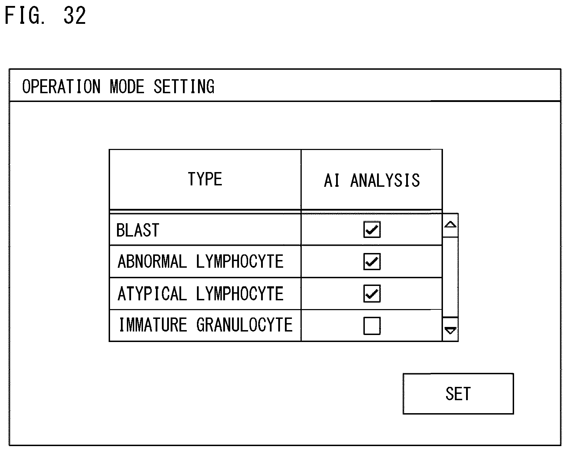

FIG. 32 is an exemplary drawing schematically showing a screen for setting whether or not to perform the AI analysis with respect to an analyte of a specific type.

The screen in FIG. 32 is displayed on a display part of the analysis unit 300, for example. The screen in FIG. 32 includes, for each specific type of analyte, a check box for setting the AI analysis. When the specimen analyzer 4000 is a blood cell analyzer, the types that are classified through the calculation processing analysis can include blast, abnormal lymphocyte, atypical lymphocyte, immature granulocyte, and the like. The user operates a check box to select whether or not to perform the AI analysis for each specific type of analyte, and operates a setting button. Accordingly, a rule is stored in a storage of the analysis unit 300.

When a check box for a specific type of analyte is on, the AI analysis is executed on the set data being a collection of a plurality of pieces of the waveform data classified as the type. In the example shown in FIG. 32, the AI analysis is executed with respect to blasts, abnormal lymphocytes, and atypical lymphocytes.

With reference back to FIG. 31, in step S93, on the basis of the type of each analyte classified in step S91 and the rule referred to in step S92, the analysis unit 300 determines whether or not the analyte (e.g., in the case of the rule shown in FIG. 32, blast, abnormal lymphocyte, and atypical lymphocyte) classified as a specific classification has been detected. When the analyte classified as the specific classification has been detected (S93: YES), the analysis unit 300 specifies, in step S94, waveform data that corresponds to the analyte classified as the specific classification. In step S95, the analysis unit 300 executes the AI analysis on the set data being a collection of a plurality of pieces of the specified waveform data.

In step S17, the analysis unit 300 provides analysis results obtained through the calculation processing analysis and the AI analysis. At this time, the analysis unit 300 replaces, out of the analysis results obtained through the calculation processing analysis in step S91, the analysis result of the type having served as a target of the AI analysis, with an analysis result obtained through the AI analysis, and provides the analysis result. The analysis result obtained through the calculation processing analysis and the analysis result obtained through the AI analysis may be provided in combination.

[Embodiment 5]

In Embodiment 5, an example in which the specimen analyzer 4000 executes counting and classification of blood cells in a blood specimen is shown.

FIG. 33 is a block diagram showing a functional configuration of the electric/control system of the analysis unit 300.

The analysis unit 300 includes the processor 3001, a RAM 3017, a bus 3003, a storage 3004, an IF part 3006, a display part 3011, and an operation part 3012. The analysis unit 300 is implemented by a personal computer, for example. The analysis unit 300 is connected to the measurement unit 400 via the IF part 3006.

The processor 3001 is implemented by a CPU, for example. The processor 3001 executes a program extracted to the RAM 3017 from the storage 3004. The RAM 3017 is a so-called main memory. The processor 3001 executes a program for analysis, thereby analyzing waveform data obtained in the measurement unit 400. The processor 3001 executes a program for control, thereby controlling the analysis unit 300 and the measurement unit 400.

The storage 3004 is implemented by a hard disk drive (HDD) or a solid state drive (SSD), for example. The storage 3004 stores waveform data received from the measurement unit 400, a program for controlling the analysis unit 300 and the measurement unit 400, and a program for analyzing waveform data. For example, the program for analyzing waveform data is configured to analyze waveform data on the basis of the calculation processing analysis and the AI analysis described above. The storage 3004 stores a rule for specifying waveform data to serve as a target of each of the AI analysis and the calculation processing analysis, and a rule for selecting an analysis operation.

The display part 3011 is implemented by a liquid crystal display, for example. The display part 3011 is connected to the processor 3001 via the bus 3003 and the IF part 3006. On the display part 3011, an analysis result obtained in the analysis unit 300 is displayed, for example.

The operation part 3012 is implemented by a pointing device and the like including a keyboard, a mouse, and a touch panel, for example. The user such as a doctor or a laboratory technician operates the operation part 3012, to input a measurement order to the specimen analyzer 4000, thereby being able to input a measurement instruction based on the measurement order. By operating the operation part 3012, the user can also input an instruction to display an analysis result. The analysis result includes, for example, a numerical value result, a graph, and a chart that are based on the analysis, flag information provided to the specimen, and the like.

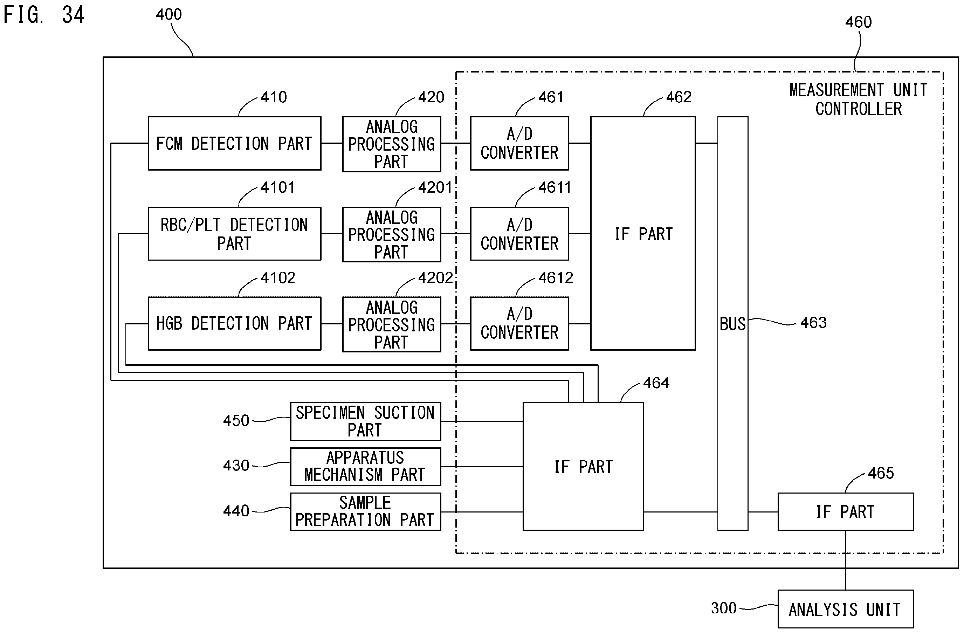

FIG. 34 is a block diagram showing a functional configuration of the electric/control system of the measurement unit 400 in a case where the specimen analyzer 4000 executes counting and classification of blood cells in a blood specimen.

The measurement unit 400 in FIG. 34 includes the FCM detection part 410, an RBC/PLT detection part 4101, an HGB detection part 4102, analog processing parts 420, 4201, 4202, an apparatus mechanism part 430, a sample preparation part 440, a specimen suction part 450, and a measurement unit controller 460.

The specimen suction part 450 suctions a specimen from a specimen container, and discharges the suctioned specimen into a reaction container (e.g., reaction chamber, reaction cuvette), for example. The sample preparation part 440 suctions a reagent for preparing a measurement sample, and discharges the reagent into the reaction container that contains the specimen, for example. The specimen and the reagent are mixed in the reaction container, whereby a measurement sample is prepared. The apparatus mechanism part 430 includes mechanisms in the measurement unit 400.

As shown in FIG. 2, the FCM detection part 410 performs measurement of blood cells according to flow cytometry. The RBC/PLT detection part 4101 is a detection part of an electrical resistance type, and performs measurement of blood cells according to a sheath flow DC detection method on the basis of an RBC/PLT measurement sample. The HGB detection part 4102 performs measurement of hemoglobin according to an SLS-hemoglobin method on the basis of a hemoglobin measurement sample. The analog processing parts 420, 4201, 4202 process analog optical signals outputted from the FCM detection part 410, the RBC/PLT detection part 4101, and the HGB detection part 4102, respectively.

Data obtained through A/D conversion of an analog signal obtained from each of the RBC/PLT detection part 4101 and the HGB detection part 4102 serves as a target of the calculation processing analysis. From the data based on the RBC/PLT detection part 4101, red blood cells and platelets in the blood specimen are counted. From the data based on the HGB detection part 4102, the hemoglobin content in the blood specimen is obtained.

The data obtained through A/D conversion of the analog signal obtained from each of the RBC/PLT detection part 4101 and the HGB detection part 4102 may serve as a target of the AI analysis. Alternatively, with respect to the data based on the RBC/PLT detection part 4101 and the HGB detection part 4102 as well, the AI analysis and the calculation processing analysis may be selectively used. Accordingly, the load on the analysis unit 300 which processes data can be reduced.

The measurement unit controller 460 includes A/D converters 461, 4611, 4612, an IF (interface) part 462, a bus 463, and IF parts 464, 465.

The A/D converters 461, 4611, 4612 respectively convert analog optical signals that are from measurement start to measurement end of the measurement sample and that have been outputted from the analog processing parts 420, 4201, 4202, into digital data. The digital data generated by the A/D converters 461, 4611, 4612 is transmitted to the analysis unit 300 via the IF parts 462, 465 and the bus 463. The apparatus mechanism part 430, the sample preparation part 440, and the specimen suction part 450 are controlled by the analysis unit 300 via the IF parts 464, 465 and the bus 463.

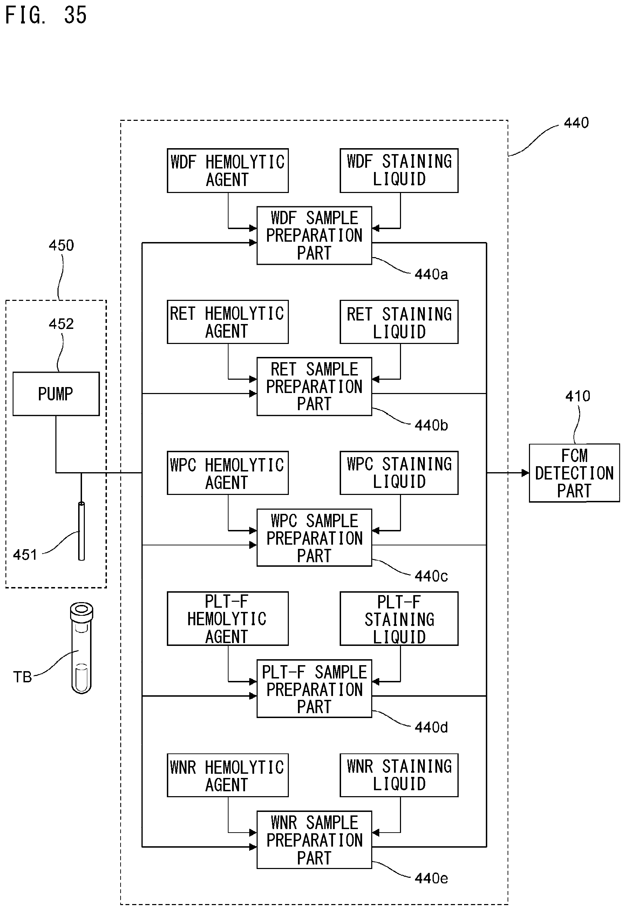

FIG. 35 is a block diagram showing a functional configuration of the fluidic system of the specimen suction part 450 and the sample preparation part 440 in the measurement unit 400 in FIG. 34.

The specimen suction part 450 includes: a nozzle 451 for suctioning a blood specimen (e.g., whole blood) from a collection tube TB; and a pump 452 for providing a negative pressure and a positive pressure to the nozzle. The nozzle 451 is moved upwardly and downwardly by the apparatus mechanism part 430 (see FIG. 34), to be inserted into the collection tube TB. When the pump 452 provides a negative pressure in a state where the nozzle 451 is inserted in the collection tube TB, the blood specimen is suctioned via the nozzle 451. The apparatus mechanism part 430 may include a hand member for inverting and stirring the collection tube TB before suctioning blood from the collection tube TB.

The sample preparation part 440 includes a WDF sample preparation part 440a, a RET sample preparation part 440b, a WPC sample preparation part 440c, a PLT-F sample preparation part 440d, and a WNR sample preparation part 440e. The sample preparation parts 440a to 440e each include a reaction chamber for mixing a specimen and a reagent (e.g., hemolytic agent and staining liquid). The sample preparation parts 440a to 440e are used in a WDF channel, a RET channel, a WPC channel, a PLT-F channel, and a WNR channel, respectively.

Here, the specimen analyzer 4000 includes a plurality of measurement channels so as to respectively correspond to a plurality of types of measurement samples that are prepared. The specimen analyzer 4000 includes the WDF channel, the RET channel, the WPC channel, the PLT-F channel, and the WNR channel, for example. The WDF channel is a channel for detecting neutrophils, lymphocytes, monocytes, and eosinophils. The RET channel is a channel for detecting reticulocytes. The WPC channel is a channel for detecting blasts and lymphocytic abnormal cells. The PLT-F channel is a channel for detecting platelets. The WNR channel is a channel for detecting white blood cells other than basophils, basophils, and nucleated red blood cells.

The sample preparation parts 440a to 440e each have connected thereto, via flow paths, a hemolytic agent container containing a hemolytic agent being a reagent corresponding to the measurement channel, and a staining liquid container containing a staining liquid being a reagent corresponding to the measurement channel. For example, the WDF sample preparation part 440a has connected thereto, via flow paths, a hemolytic agent container containing a WDF hemolytic agent (e.g., Lysercell WDF II; manufactured by Sysmex Corporation) being a WDF measurement reagent, and a staining liquid container containing a WDF staining liquid (e.g., Fluorocell WDF; manufactured by Sysmex Corporation) being a WDF measurement reagent. Here, a configuration example in which one sample preparation part is connected to a hemolytic agent container and a staining liquid container is shown. However, one sample preparation part need not necessarily be connected to both of a hemolytic agent container and a staining liquid container, and one reagent container may be used in common by a plurality of sample preparation parts. In addition, each sample preparation part and the corresponding reagent container need not be connected by a flow path. A configuration in which a reagent is suctioned by a nozzle from a reagent container, the nozzle is moved, and the suctioned reagent is discharged from the nozzle into a reaction chamber of the sample preparation part, may be adopted.

Through horizontal and up-down movement by the apparatus mechanism part 430, the nozzle 451 having suctioned a blood specimen is positioned above a reaction chamber of a sample preparation part that corresponds to a measurement order, among the sample preparation parts 440a to 440e. In this state, when the pump 452 provides a positive pressure, the blood specimen is discharged from the nozzle 451 to the corresponding reaction chamber. The sample preparation part 440 supplies a hemolytic agent and a staining liquid that correspond to the reaction chamber having discharged therein the blood specimen, and mixes the blood specimen, the hemolytic agent, and the staining liquid in the reaction chamber, thereby preparing a measurement sample.

A WDF measurement sample is prepared in the WDF sample preparation part 440a, a RET measurement sample is prepared in the RET sample preparation part 440b, a WPC measurement sample is prepared in the WPC sample preparation part 440c, a PLT-F measurement sample is prepared in the PLT-F sample preparation part 440d, and a WNR measurement sample is prepared in the WNR sample preparation part 440e. Each prepared measurement sample is supplied from the reaction chamber to the FCM detection part 410 via a flow path, and measurement of cells by flow cytometry is performed.

The measurement channels (WDF, RET, WPC, PLT-F, WNR) described above correspond to measurement items included in a measurement order. For example, the WDF channel corresponds to a measurement item regarding classification of white blood cells, the RET channel corresponds to a measurement item regarding reticulocytes, the PLT-F channel corresponds to a measurement item regarding platelets, and the WNR channel corresponds to a measurement item regarding the number of white blood cells and nucleated red blood cells. The measurement samples prepared in the measurement channels described above are measured by the FCM detection part 410.

The measurement result by the RBC/PLT detection part 4101 corresponds to a measurement item regarding the number of red blood cells. The measurement result by the HGB detection part 4102 corresponds to a measurement item regarding the hemoglobin content.

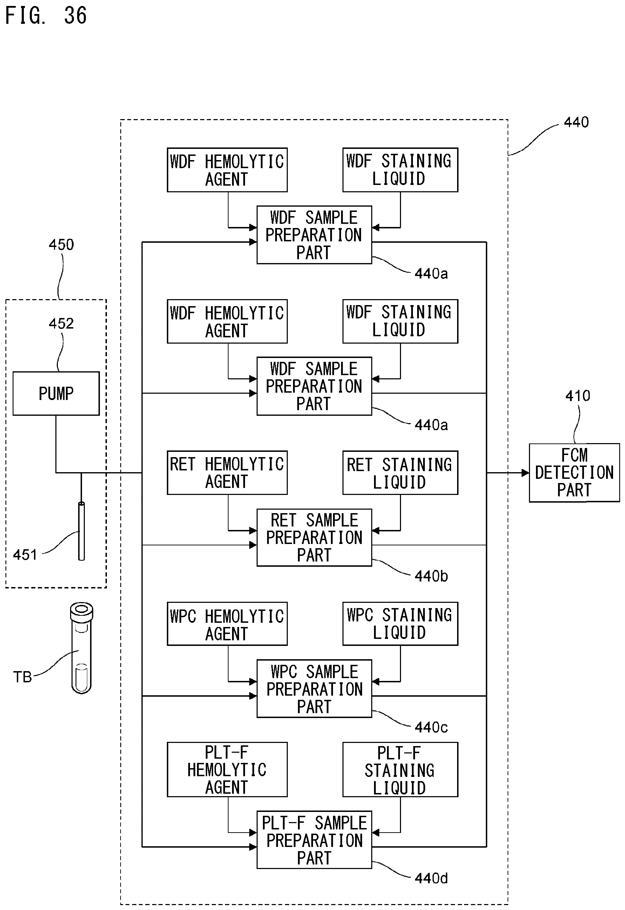

FIG. 36 is a block diagram showing another functional configuration of the fluidic system of the sample preparation part 440 shown in FIG. 35.

In the example shown in FIG. 36, the configuration of the measurement channels of the sample preparation part 440 has been changed in accordance with apportioning between the AI analysis and the calculation processing analysis. Specifically, in the sample preparation part 440 in FIG. 36, when compared with the sample preparation part 440 in FIG. 35, a WDF sample preparation part 440a for a WDF channel and a reagent (WDF hemolytic agent and WDF staining liquid) connected to the WDF sample preparation part 440a have been added, in place of the WNR sample preparation part 440e for the WNR channel and the reagent (WNR hemolytic agent and WNR staining liquid) connected to the WNR sample preparation part 440e. That is, the sample preparation part 440 in FIG. 36 includes two sets of the WDF sample preparation part 440a and the reagent that correspond to the WDF channel. The sample preparation part 440 may include three or more sets of the WDF sample preparation part 440a and the reagent that correspond to the WDF channel.

When the sample preparation part 440 is configured as shown in FIG. 36, classification of basophils and nucleated red blood cells to be performed in the WNR channel is performed in a WDF channel. The analysis unit 300 executes the AI analysis on the waveform data obtained from a measurement sample prepared in the WDF channel, thereby classifying neutrophils, lymphocytes, monocytes, eosinophils, basophils, and nucleated red blood cells. In this case, for example, out of waveform data obtained through measurement according to the WDF channel, waveform data that corresponds to neutrophils, lymphocytes, monocytes, eosinophils, basophils, and nucleated red blood cells is caused to be learned, as teacher data, by the AI algorithm 60 in advance. Accordingly, the AI algorithm 60 that can classify neutrophils, lymphocytes, monocytes, eosinophils, basophils, and nucleated red blood cells from the waveform data of the WDF channels is generated.

According to the configuration in FIG. 36, for example, in the respective reaction chambers of a plurality of the WDF sample preparation parts 440a, measurement samples of different specimens can be prepared in parallel. Accordingly, measurements regarding the WDF channels on different specimens can be executed in parallel.

In the configuration in FIG. 36, the reaction chamber and the reagent corresponding to the original measurement channel (the WNR channel) are replaced with a reaction chamber and a reagent corresponding to the replacement measurement channel (the WDF channel). In this case, the analysis regarding the original measurement channel needs to be performed in the analysis regarding the replacement measurement channel.

In the configuration in FIG. 36, analysis regarding the original measurement channel (the WNR channel) is executed as the AI analysis that is performed on the waveform data according to the replacement measurement channel (the WDF channel). Therefore, the original measurement channel (the WNR channel) can be replaced with the replacement measurement channel (the WDF channel). Thus, without increasing the total number of measurement channels provided to the specimen analyzer 4000, the number of measurement channels of a type that is desired to be additionally provided can be increased. When the number of WDF channels is increased, different specimens can be measured in a parallel manner in a plurality of the WDF channels, whereby the throughput of measurement according to the WDF channels is improved. Since apportioning between the AI analysis and the calculation processing analysis is performed, a remarkable effect that the arithmetic operation load on the AI analysis is reduced and that the throughput of specimen processing can also be improved is obtained.

In the present embodiment, for example, which of the AI analysis and the calculation processing analysis is to be performed may be determined in accordance with a measurement channel.

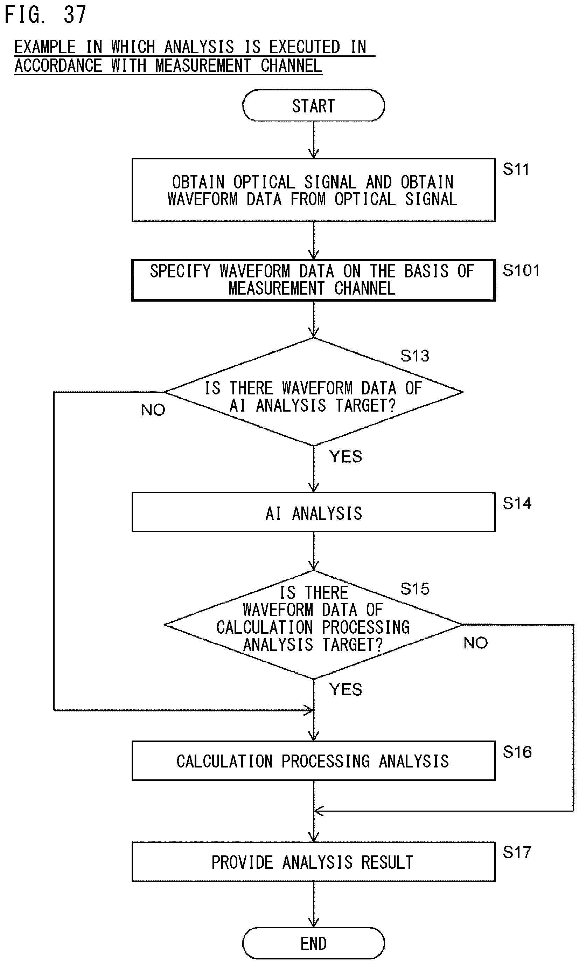

FIG. 37 is a flowchart showing an example in which analysis is executed in accordance with a measurement channel.

In FIG. 37, when compared with FIG. 15, step S101 is added in place of step S12. Hereinafter, changes from FIG. 15 will be described.

In step S101, the analysis unit 300 refers to a rule that includes which of the AI analysis and the calculation processing analysis is to be performed on the basis of a measurement channel, and on the basis of the rule referred to, the analysis unit 300 specifies waveform data to serve as a target of the AI analysis and waveform data to serve as a target of the calculation processing analysis, with respect to the waveform data obtained in step S 11.

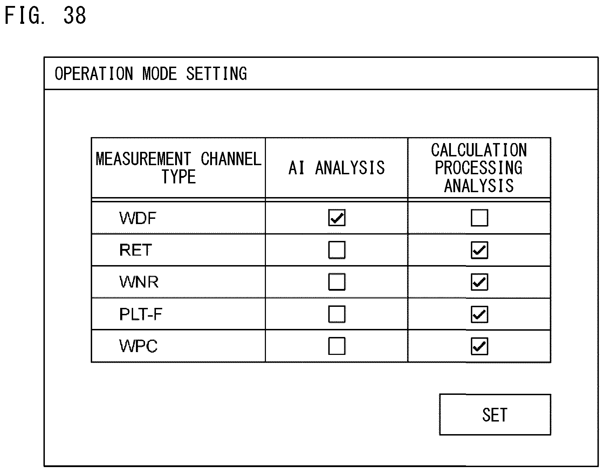

FIG. 38 is an exemplary drawing schematically showing a screen for setting the AI analysis or the calculation processing analysis for each measurement channel. The measurement channels shown as examples in FIG. 38 are those for a blood cell analyzer.

The screen in FIG. 38 is displayed on a display part of the analysis unit 300, for example. The screen in FIG. 38 includes, for each measurement channel, a check box for setting the AI analysis and a check box for setting the calculation processing analysis. The user operates a check box, to select which analysis out of the AI analysis and the calculation processing analysis is to be performed for each measurement channel, and operates a setting button. Accordingly, a rule is stored in a storage of the analysis unit 300.

With reference back to FIG. 37, when waveform data to serve as a target of the AI analysis is included in the waveform data specified in step S101 (S13: YES), the analysis unit 300 executes, in step S14, the AI analysis on the set data being a collection of a plurality of pieces of the waveform data to serve as a target of the AI analysis specified in step S101. When waveform data to serve as a target of the calculation processing analysis is included in the waveform data specified in step S101 (S15: YES), the analysis unit 300 executes, in step S16, the calculation processing analysis on the waveform data to serve as a target of the calculation processing analysis specified in step S101.

In the case of the example in FIG. 38, since the WDF channel is set as a target of the AI analysis, analysis of the measurement item (e.g., the types of white blood cells) associated with the WDF channel is executed as the AI analysis that is performed on the set data being a collection of a plurality of pieces of the waveform data obtained from a measurement sample prepared in the WDF channel. Meanwhile, the other channels are set to be targets of the calculation processing analysis, and thus, analyses of measurement items associated with the other channels are executed as the calculation processing analysis that is performed on waveform data obtained from measurement samples prepared in the other channels.

When the WDF channel is set to be a target of the AI analysis, the AI analysis may be executed with respect to all of the measurement items that correspond to the WDF channel, or the AI analysis may be executed with respect to some measurement items that correspond to the WDF channel and the calculation processing analysis may be executed with respect to the other measurement items.

In the analysis method described with reference to FIG. 37, through the AI analysis of the set data being a collection of a plurality of pieces of the waveform data obtained in measurement according to the WDF channel, for example, the analysis unit 300 may execute classification and counting of eosinophils, neutrophils, lymphocytes, and monocytes in addition to classification and counting of nucleated red blood cells (NRBC) and classification and counting of basophils (BASO). In the case of this example, the AI algorithm 60 has been caused to learn, with waveform data, so as to be able to classify nucleated red blood cells, basophils, eosinophils, neutrophils, lymphocytes, and monocytes. When such an AI algorithm 60 is used, the WNR channel can be replaced with the WDF channel.

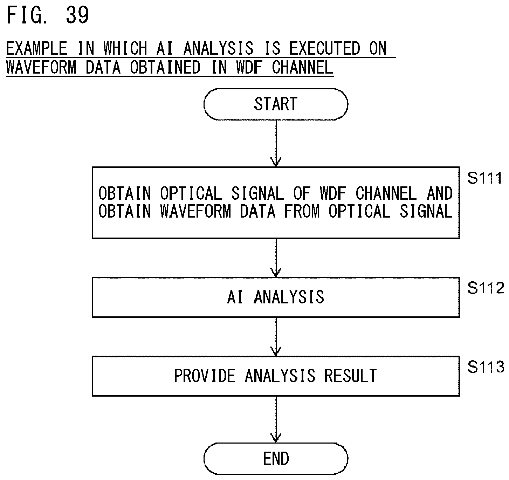

FIG. 39 is a flowchart showing an example in which the AI analysis is executed on waveform data obtained in the WDF channel.

In the example of the AI analysis shown in FIG. 39, through the AI analysis of the set data being a collection of a plurality of pieces of the waveform data obtained in measurement according to the WDF channel, for example, the analysis unit 300 also executes classification and counting of eosinophils, neutrophils, lymphocytes, and monocytes in addition to classification and counting of nucleated red blood cells (NRBC) and classification and counting of basophils (BASO). In the case of this example, the AI algorithm 60 has been caused to learn, with waveform data, so as to be able to classify nucleated red blood cells, basophils, eosinophils, neutrophils, lymphocytes, and monocytes. When such an AI algorithm 60 is used, the WNR channel can be replaced with the WDF channel.

In step S111, the measurement unit 400 obtains optical signals from a measurement sample prepared in the WDF channel, and obtains waveform data from each obtained optical signal. In step S112, the analysis unit 300 executes the AI analysis on the set data being a collection of a plurality of pieces of the waveform data obtained in step S111. In step S113, the analysis unit 300 provides an analysis result of the waveform data of the WDF channel, and analysis results of the waveform data of other channels in combination. How the analyses on the waveform data of the other channels are apportioned between and executed by the AI analysis and the calculation processing analysis is determined on the basis of one of the rules shown as examples in the embodiments described above, for example.

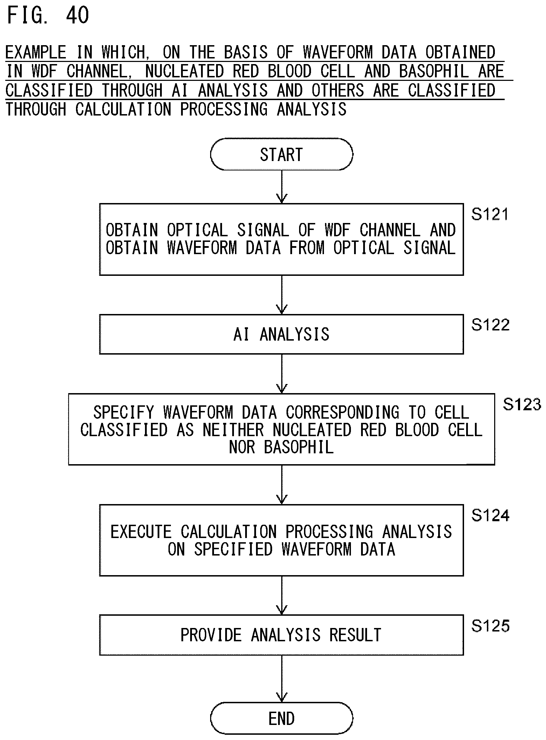

FIG. 40 is a flowchart showing an example in which, on the basis of waveform data obtained in the WDF channel, nucleated red blood cells and basophils are classified through the AI analysis, and the others are classified through the calculation processing analysis.

In the example in FIG. 40, the analysis unit 300 performs classification and counting of nucleated red blood cells and classification and counting of basophils, through the AI analysis performed on the set data being a collection of a plurality of pieces of the waveform data obtained in the WDF channel, for example. The analysis unit 300 executes the calculation processing analysis on waveform data that corresponds to cells that have been classified as neither nucleated red blood cells nor basophils, and executes classification and counting of eosinophils, neutrophils, lymphocytes, and monocytes. In the case of this example, the AI algorithm 60 has been caused to learn so as to be able to classify analytes into nucleated red blood cells, basophils, and analytes other than these, from waveform data, for example.

The analysis unit 300 executes the calculation processing analysis on waveform data that corresponds to cells that have been classified as neither nucleated red blood cells nor basophils. For example, the peak value of waveform data that corresponds to each cell that has been classified as neither a nucleated red blood cell nor a basophil is extracted, and the cell type is classified on the basis of a two-dimensional graph (scattergram) generated from peak values that correspond to side scattered light and peak values that correspond to fluorescence. For example, on the basis of the two-dimensional graph, which of an eosinophil, a neutrophil, a lymphocyte, a monocyte, and other than these the cell is, is classified. A cell that has been classified as other than an eosinophil, a neutrophil, a lymphocyte, and a monocyte through the analysis based on the two-dimensional graph is classified as debris, for example.

In step S121, the measurement unit 400 obtains optical signals from a measurement sample prepared in the WDF channel, and obtains waveform data from each obtained optical signal. In step S122, the analysis unit 300 executes the AI analysis on the set data being a collection of a plurality of pieces of the waveform data obtained in step S121. Accordingly, nucleated red blood cells and basophils are classified. In step S123, the analysis unit 300 specifies waveform data that corresponds to cells that are classified as neither nucleated red blood cells nor basophils.

In step S124, the analysis unit 300 executes the calculation processing analysis on the waveform data specified in step S123. Accordingly, lymphocytes, monocytes, eosinophils, and neutrophils are classified. In step S125, the analysis unit 300 provides an analysis result of the waveform data of the WDF channel and analysis results of the waveform data of other channels in combination.

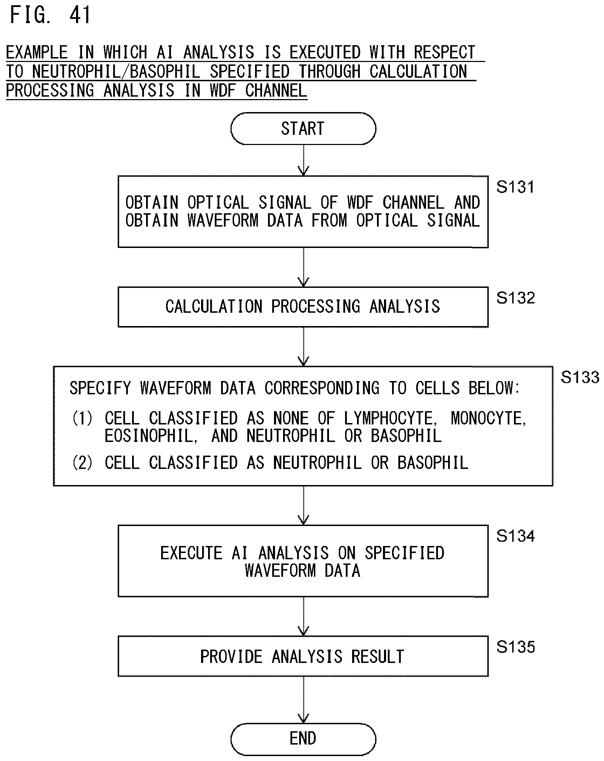

FIG. 41 is a flowchart showing an example in which the AI analysis is executed with respect to neutrophils/basophils specified through the calculation processing analysis in the WDF channel.

In the example in FIG. 41, the analysis unit 300 executes classification and counting of lymphocytes, classification and counting of monocytes, classification and counting of eosinophils, and classification and counting of neutrophils or basophils through the calculation processing analysis of waveform data obtained in the WDF channel, for example.

In the classification and counting of neutrophils or basophils, cells that are classified as either one of neutrophils and basophils are counted, for example. Subsequently, the analysis unit 300 executes the AI analysis on the set data being a collection of a plurality of pieces of waveform data that corresponds to cells that have been classified as none of lymphocytes, monocytes, eosinophils, and neutrophils or basophils, and cells that have been classified as either one of neutrophils and basophils. Accordingly, the analytes are classified as nucleated red blood cells, basophils, and cells other than these.

For example, from the counting result of cells classified as either neutrophils or basophils through the calculation processing analysis, a counting result of cells classified as basophils through the AI analysis is subtracted, whereby a counting result of neutrophils and a counting result of basophils are calculated. Cells that have been classified as neither nucleated red blood cells nor basophils through the AI analysis are classified as debris, for example.

In step S131, the measurement unit 400 obtains optical signals from a measurement sample prepared in the WDF channel, and obtains waveform data from each obtained optical signal. In step S132, the analysis unit 300 executes the calculation processing analysis on the waveform data obtained in step S131. Accordingly, groups of lymphocytes, monocytes, eosinophils, and neutrophils and basophils are classified. In step S133, the analysis unit 300 specifies waveform data that corresponds to (1) cells that have been classified as none of lymphocytes, monocytes, eosinophils, and neutrophils or basophils, and (2) cells that have been classified as neutrophils or basophils.

In step S134, the analysis unit 300 executes the AI analysis on the set data being a collection of a plurality of pieces of the waveform data specified in step S133. Accordingly, neutrophils and basophils are classified. In step S135, the analysis unit 300 provides an analysis result of the waveform data of the WDF channel and analysis results of the waveform data of other channels in combination.

[Embodiment 6]

In Embodiment 6, a configuration example of the specimen analyzer 4000 that includes a host processor and a parallel-processing processor is shown. In Embodiment 6, in a parallel-processing processor 3002, arithmetic operation regarding analysis of waveform data is executed by parallel processing, and on the basis of the result of the arithmetic operation by parallel processing, information regarding the type of each of analytes is generated.

According to Embodiment 6, even when data having a huge volume of several hundred megabytes to several gigabytes per specimen is analyzed, processing regarding waveform data can be executed in parallel by the parallel-processing processor provided separately from the host processor. Therefore, for example, even when data having a huge volume is processed by the AI algorithm 60, processing of the data is concluded in the specimen analyzer 4000. Therefore, for example, data need not necessarily be transmitted via the Internet or an intranet to an analysis server that stores the AI algorithm 60. Therefore, according to Embodiment 6, it is not necessary to transmit a large volume of data from the specimen analyzer 4000 to the analysis server, and to obtain an analysis result returning from the analysis server. Thus, the throughput of the specimen analyzer 4000 can be maintained at a high level while the classification accuracy of analytes in the specimen is improved.

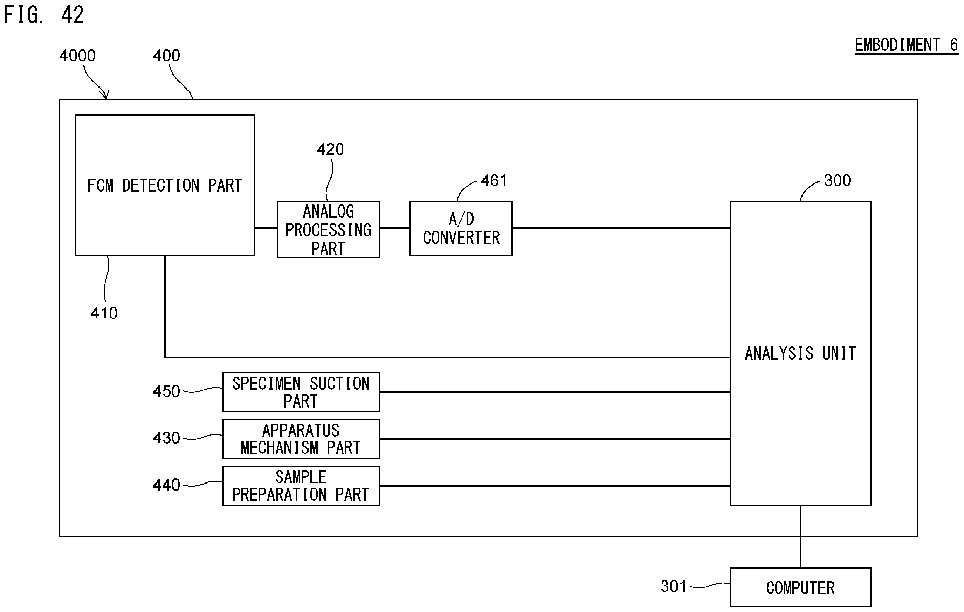

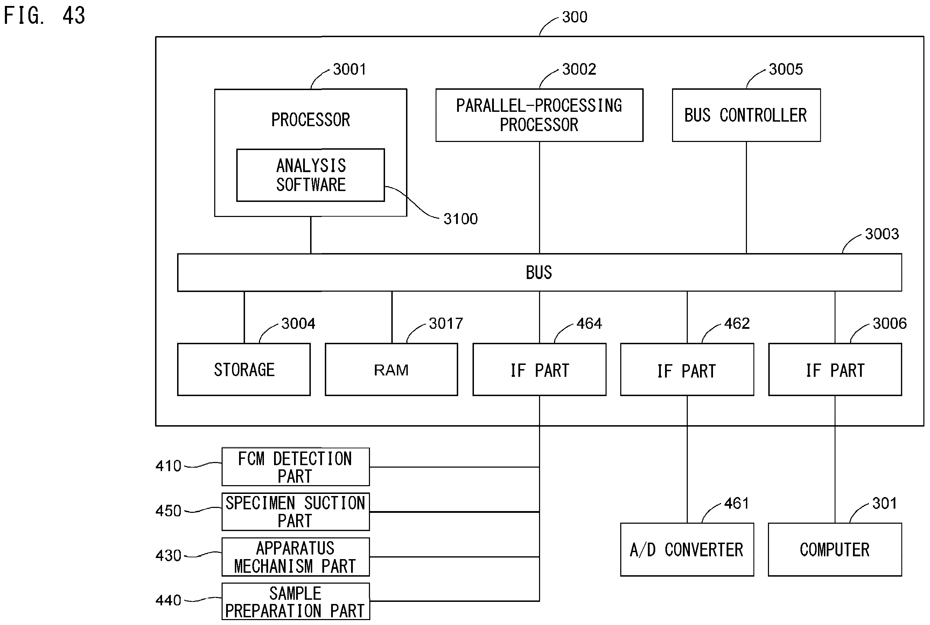

With reference to FIGS. 42 and 43, a configuration of the specimen analyzer 4000 of Embodiment 6 will be described. In the configuration example shown in FIGS. 42 and 43, the measurement unit 400 includes the FCM detection part 410 for measuring a specimen (e.g., blood specimen, urine specimen, body fluid, bone marrow aspirate).

FIG. 42 is a block diagram showing a functional configuration of the electric/control system of the specimen analyzer 4000, according to Embodiment 6.

The specimen analyzer 4000 of Embodiment 6 includes the measurement unit 400, and the analysis unit 300 provided in the measurement unit 400. The analysis unit 300 of Embodiment 6 is connected to the A/D converter 461, the apparatus mechanism part 430, the sample preparation part 440, and the specimen suction part 450 in the measurement unit 400, and a computer 301 connected to the measurement unit 400.

FIG. 43 is a block diagram showing a functional configuration of the electric/control system of the analysis unit 300, according to Embodiment 6.

When compared with the analysis unit 300 shown in FIG. 33, the analysis unit 300 of Embodiment 6 includes the parallel-processing processor 3002, a bus controller 3005, and the IF parts 462, 464.

The parallel-processing processor 3002 is configured to be able to process, instead of a master processor, arithmetic processes by the AI algorithm 60. By using the parallel-processing processor 3002 suitable for the processes of a matrix operation executed by the AI algorithm 60, it is possible to improve the TAT necessary for the AI analysis. However, although the TAT is improved by the parallel-processing processor 3002, if the data amount of the analysis target is increased, the computer load necessary for the AI analysis is increased. With regard to this, as described above, since data analysis is apportioned between the calculation processing analysis and the AI analysis, the computer load can be reduced, and further improvement of the test efficiency can be realized.

Using the parallel-processing processor 3002, the processor 3001 executes, by the AI algorithm 60, an analysis process on the set data being a collection of a plurality of pieces of waveform data. That is, the processor 3001 executes analysis software 3100, thereby executing the AI analysis, based on the AI algorithm 60, of the set data being a collection of a plurality of pieces of waveform data. The analysis software 3100 is used in order to analyze waveform data corresponding to each analyte in a specimen, on the basis of the AI algorithm 60.

The analysis software 3100 may be stored in the storage 3004. In this case, the processor 3001 extracts the analysis software 3100 stored in the storage 3004 to the RAM 3017 and executes the analysis software 3100, thereby executing the AI analysis, based on the AI algorithm 60, of the set data being a collection of a plurality of pieces of waveform data.

In the present embodiment, for example, the AI analysis is executed by the processor 3001 and the parallel-processing processor 3002, and the calculation processing analysis is executed by the processor 3001 without using the parallel-processing processor 3002.

The processor 3001 is a CPU (Central Processing Unit), for example. For example, Core i9, Core i7, or Core i5 manufactured by Intel Corporation, or Ryzen 9, Ryzen 7, Ryzen 5, or Ryzen 3 manufactured by AMD, or the like may be used as the processor 3001.

The processor 3001 controls the parallel-processing processor 3002. The parallel-processing processor 3002 executes parallel processing regarding, for example, a matrix operation in accordance with control by the processor 3001. That is, the processor 3001 is a master processor of the parallel-processing processor 3002, and the parallel-processing processor 3002 is a slave processor of the processor 3001. The processor 3001 is also referred to as a host processor or a main processor. The processor 3001 executes the matrix operation according to the AI algorithm 60, through parallel processing performed by the parallel-processing processor 3002.

The parallel-processing processor 3002 executes in parallel, a plurality of arithmetic processes being at least a part of processes regarding analysis of waveform data. The parallel-processing processor 3002 is a GPU (Graphics Processing Unit), an FPGA (Field Programmable Gate Array), or an ASIC (Application Specific Integrated Circuit), for example. When the parallel-processing processor 3002 is an FPGA, the parallel-processing processor 3002 may have programmed therein in advance an arithmetic process regarding the trained AI algorithm 60, for example. When the parallel-processing processor 3002 is an ASIC, the parallel-processing processor 3002 may have incorporated therein in advance a circuit for executing the arithmetic process regarding the trained AI algorithm 60, or may have built therein a programmable module in addition to such an incorporated circuit, for example.

As the parallel-processing processor 3002, GeForce, Quadro, TITAN, Jetson, or the like manufactured by NVIDIA Corporation may be used, for example. In the case of the Jetson series, Jetson Nano, Jetson Tx2, Jetson Xavier, or Jetson AGX Xavier is used, for example.

The processor 3001 executes calculation processing regarding control of the measurement unit 400, for example. The processor 3001 executes calculation processing regarding control signals transmitted/received between the apparatus mechanism part 430, the sample preparation part 440, and the specimen suction part 450, for example. The processor 3001 executes calculation processing regarding transmission/reception of information to/from the computer 301, for example.

The computer 301 has a function of displaying an analysis result transmitted from the analysis unit 300 on the basis of the processing performed by the processor 3001, for example. The computer 301 transmits a measurement order to the analysis unit 300, for example. The measurement order is transmitted from a host computer to the computer 301, for example. It is also possible for the user to input a measurement order via an input device of the computer 301.

The processor 3001 executes processes regarding reading-out of program data from the storage 3004, extracting a program to the RAM 3017, and transmission/reception of data to/from the RAM 3017, for example. The above-described processes executed by the processor 3001 are required to be executed in a predetermined sequential order, for example. For example, when processes needed for control of the apparatus mechanism part 430, the sample preparation part 440, and the specimen suction part 450 are assumed to be A, B, and C, respectively, the processes are required to be executed in a sequential order of B, A, and C, in some cases. The processor 3001 often executes such continuous processes that depend on a sequential order, and thus, even when the number of arithmetic units (each may be referred to as a "processor core", a "core", or the like) is increased, the processing speed is not always increased.

Meanwhile, the parallel-processing processor 3002 executes a large number of regular calculation processes such as arithmetic operations on matrix data including a large number of elements, for example. In the present embodiment, the parallel-processing processor 3002 executes parallel processing in which at least a part of arithmetic processes of analyzing the set data being a collection of a plurality of pieces of waveform data in accordance with the AI algorithm 60 are parallelized. The AI algorithm 60 includes a large number of matrix operations, for example. For example, the AI algorithm 60 may include at least 100 matrix operations, or may include at least 1000 matrix operations.

The parallel-processing processor 3002 has a plurality of arithmetic units, and the respective arithmetic units can simultaneously execute matrix operations. That is, the parallel-processing processor 3002 can execute, in parallel, matrix operations by a plurality of respective arithmetic units, as parallel processing. For example, a matrix operation included in the AI algorithm 60 can be divided into a plurality of arithmetic processes that are not dependent on a sequential order with each other. The thus divided arithmetic processes can be executed in parallel by a plurality of arithmetic units, respectively. These arithmetic units may be each referred to as a "processor core", a "core", or the like.

As a result of execution of such parallel processing, speed up of arithmetic processing in the entirety of the specimen analyzer 4000 can be realized. A process such as a matrix operation included in the AI algorithm 60 may be referred to as "Single Instruction Multiple Data (SIMD) processing", for example. The parallel-processing processor 3002 is suitable for such an SIMD operation, for example. Such a parallel-processing processor 3002 may be referred to as a vector processor.

As described above, the processor 3001 is suitable for executing diverse and complicated processes. Meanwhile, the parallel-processing processor 3002 is suitable for executing in parallel a large number of regular processes. Through parallel execution of a large number of regular arithmetic processes, the TAT required for calculation processing is shortened.

The parallel processing to be executed by the parallel-processing processor 3002 is not limited to matrix operations. For example, when the parallel-processing processor 3002 executes a learning process with respect to the AI algorithm 60, differential operations or the like regarding the learning process can be the target of the parallel processing.

As for the number of arithmetic units of the processor 3001, a dual core (the number of cores: 2), a quad core (the number of cores: 4), or an octa core (the number of cores: 8) is adopted, for example. Meanwhile, the parallel-processing processor 3002 has, for example, at least ten arithmetic units (the number of cores: 10), and can execute in parallel ten matrix operations. The parallel-processing processor 3002 that has several tens of arithmetic units also exists. The parallel-processing processor 3002 that has, for example, at least 100 arithmetic units (the number of cores: 100) and that can execute in parallel 100 matrix operations also exists. The parallel-processing processor 3002 that has several hundred arithmetic units also exists. The parallel-processing processor 3002 that has, for example, at least 1000 arithmetic units (the number of cores: 1000) and that can execute in parallel 1000 matrix operations also exists. The parallel-processing processor 3002 that has several thousand arithmetic units also exists.

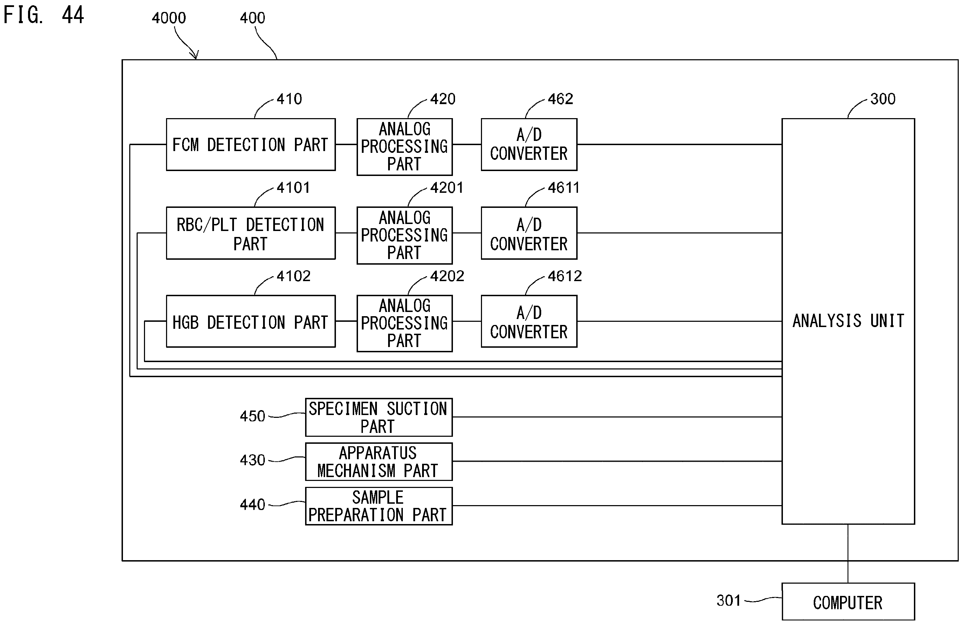

FIG. 44 is a block diagram showing another functional configuration of the electric/control system of the specimen analyzer 4000, according to Embodiment 6. The specimen analyzer 4000 in FIG. 44 executes counting and classification of blood cells in a blood specimen.

When compared with the specimen analyzer 4000 in FIG. 42, the specimen analyzer 4000 in FIG. 44 includes the RBC/PLT detection part 4101, the HGB detection part 4102, the analog processing parts 4201, 4202, and the A/D converters 4611, 4612. The sample preparation part 440 is configured so as to be similar to that in other embodiments described above.

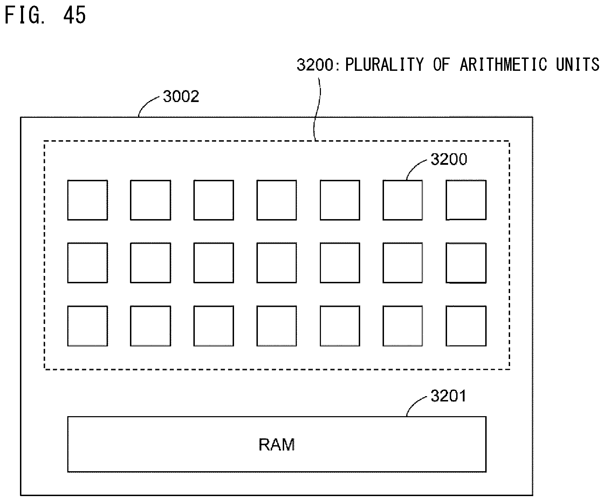

FIG. 45 shows a configuration example of the parallel-processing processor 3002.

The parallel-processing processor 3002 includes a plurality of arithmetic units 3200 and a RAM 3201. The respective arithmetic units 3200 execute arithmetic processes on matrix data in parallel. The RAM 3201 stores data regarding arithmetic processes executed by the arithmetic units 3200. The RAM 3201 is a memory that has a capacity of at least 1 gigabyte. The RAM 3201 may be a memory that has a capacity of 2 gigabytes, 4 gigabytes, 6 gigabytes, 8 gigabytes, 10 gigabytes, or more. Each arithmetic unit 3200 obtains data from the RAM 3201 and executes an arithmetic process. The arithmetic unit 3200 may be referred to as a "processor core", a "core", or the like.

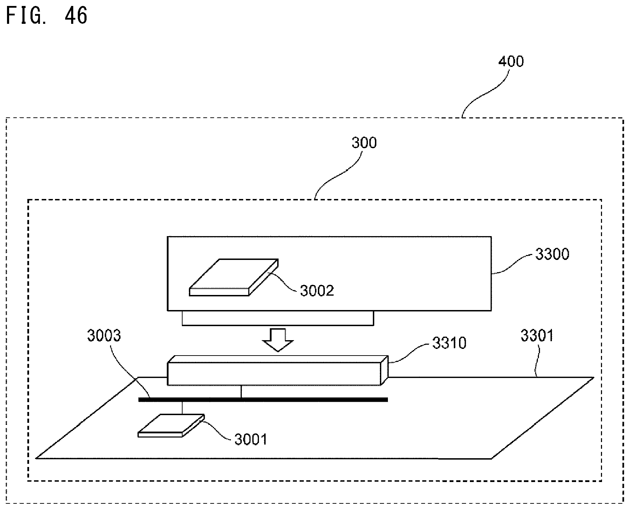

FIGS. 46 to 48 each schematically show an installation example of the parallel-processing processor 3002.

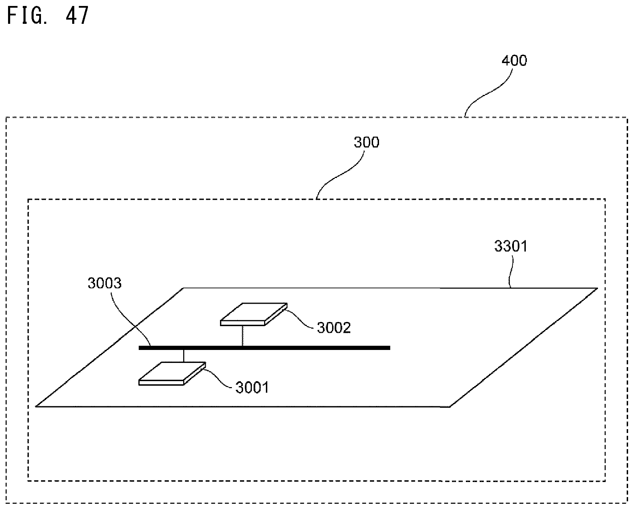

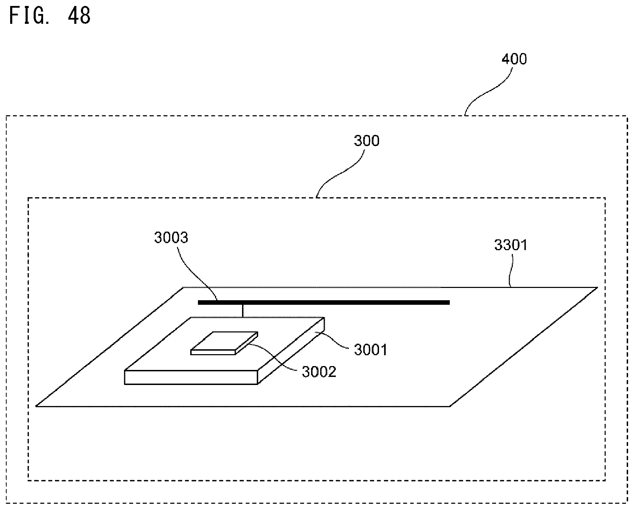

In the example shown in FIG. 46, the processor 3001 is installed on a substrate 3301. The parallel-processing processor 3002 is installed on a graphic board 3300, and the graphic board 3300 is connected to the substrate 3301 via a connector 3310, for example. The processor 3001 is connected to the parallel-processing processor 3002 via the bus 3003. In the example shown in FIG. 47, the parallel-processing processor 3002 is directly installed on the substrate 3301, and connected to the processor 3001 via the bus 3003. In the example shown in FIG. 48, the processor 3001 and the parallel-processing processor 3002 are integrally provided. In this case, the parallel-processing processor 3002 is built in the processor 3001 installed on the substrate 3301.

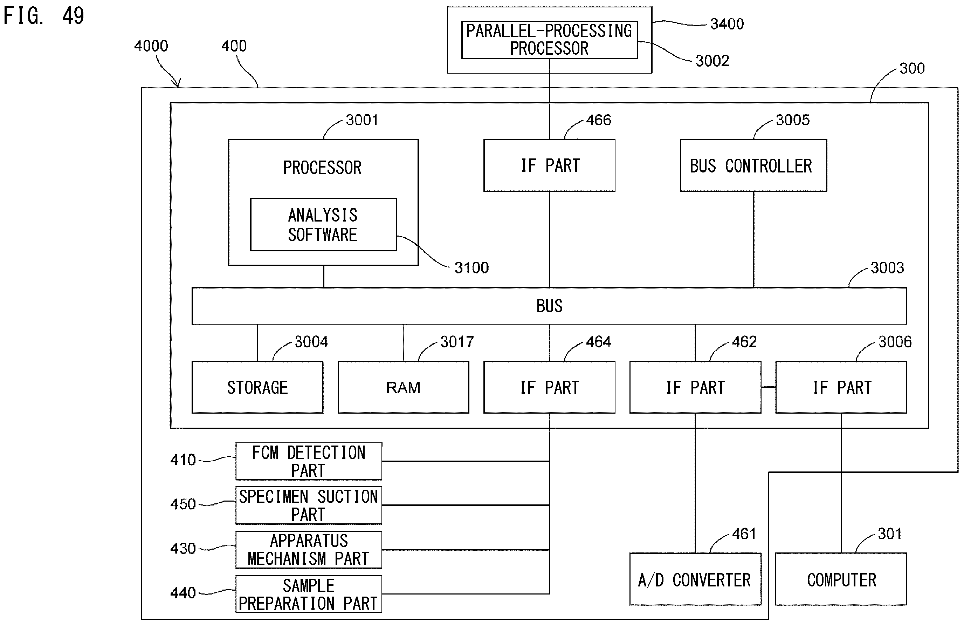

FIG. 49 shows another installation example of the parallel-processing processor 3002.

In the example shown in FIG. 49, the parallel-processing processor 3002 is installed to the measurement unit 400 by means of an external apparatus 3400 connected to the measurement unit 400. The parallel-processing processor 3002 is mounted to the external apparatus 3400 being a USB device, for example. The external apparatus 3400 is connected to the bus 3003 via an IF part 466, whereby the parallel-processing processor 3002 is installed to the specimen analyzer 4000. The USB device may be a small device such as a USB dongle, for example. The IF part 466 is a USB interface having a transfer rate of several hundred Mbps, for example, and more preferably, is a USB interface having a transfer rate of several Gbps to several tens of Gbps, or higher. As the external apparatus 3400 having the parallel-processing processor 3002 mounted thereon, Neural Compute Stick 2 manufactured by Intel Corporation may be used, for example.

A plurality of USB devices each having the parallel-processing processor 3002 mounted thereon may be connected to the IF part 466, whereby a plurality of the parallel-processing processors 3002 may be installed to the specimen analyzer 4000. The parallel-processing processor 3002 mounted on one USB device has a smaller number of arithmetic units 3200 than a GPU or the like in some cases. Therefore, if a plurality of USB devices are connected to the measurement unit 400, scale-up of the number of cores can be realized.

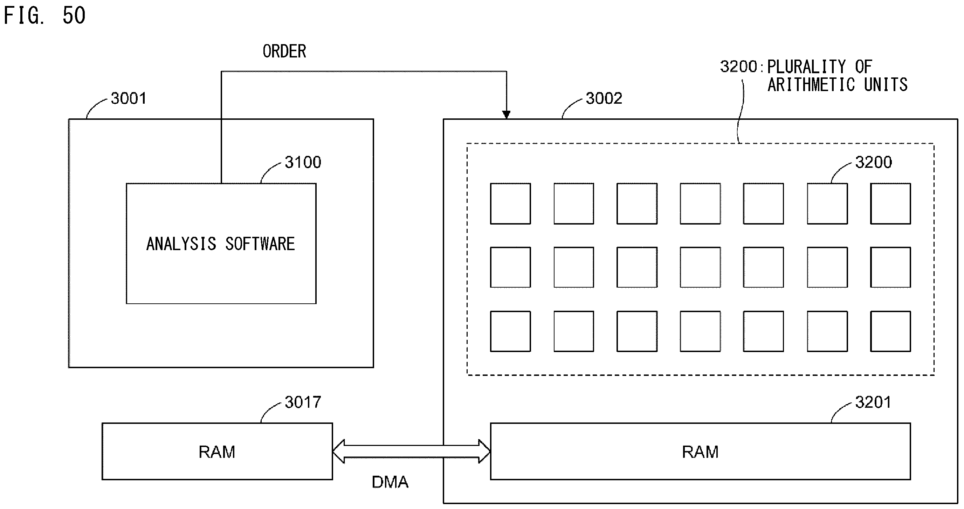

Next, with reference to FIGS. 50 to 52, an outline of arithmetic processes executed by the parallel-processing processor 3002 on the basis of control of the analysis software 3100 which operates on the processor 3001 will be described.

FIG. 50 shows a configuration example of the parallel-processing processor 3002 which executes arithmetic processes.

The parallel-processing processor 3002 includes a plurality of the arithmetic units 3200 and the RAM 3201. The processor 3001, which executes the analysis software 3100, issues an order to the parallel-processing processor 3002, and causes the parallel-processing processor 3002 to execute at least a part of arithmetic processes necessary for analysis, by the AI algorithm 60, of the set data being a collection of a plurality of pieces of waveform data. The processor 3001 orders the parallel-processing processor 3002 to execute arithmetic processes regarding waveform data analysis based on the AI algorithm 60.

All or at least a part of waveform data is stored in the RAM 3017. The data stored in the RAM 3017 is transferred to the RAM 3201 of the parallel-processing processor 3002 by a DMA (Direct Memory Access) method, for example. The plurality of arithmetic units 3200 of the parallel-processing processor 3002 respectively execute, in parallel, arithmetic processes with respect to the data stored in the RAM 3201. Each of the plurality of arithmetic units 3200 obtains necessary data from the RAM 3201, to execute an arithmetic process. Data corresponding to the arithmetic result is stored into the RAM 3201 of the parallel-processing processor 3002. The data corresponding to the arithmetic result is transferred from the RAM 3201 to the RAM 3017 by a DMA method, for example.

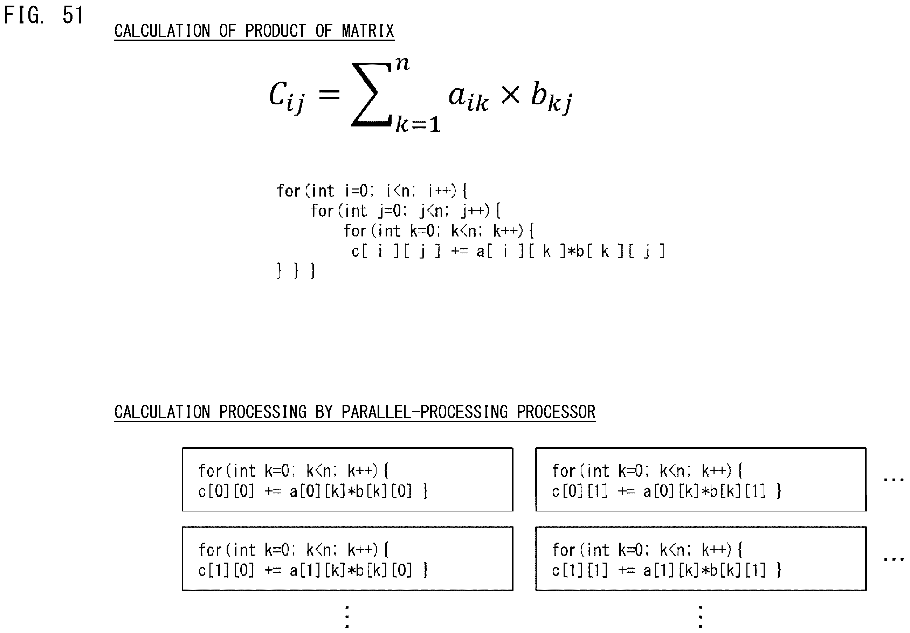

FIG. 51 shows an outline of a matrix operation executed by the parallel-processing processor 3002.

Prior to analyzing the set data being a collection of a plurality of pieces of waveform data in accordance with the AI algorithm 60, calculation of the product of matrixes (matrix operation) is executed. The parallel-processing processor 3002 executes in parallel a plurality of arithmetic processes regarding the matrix operation, for example.

The drawing in the upper part of FIG. 51 shows a calculation formula of the product of matrixes. In the example of this calculation formula, a matrix c is obtained by the product of a matrix a of n rows × n columns and a matrix b of n rows × n columns. As in the example in FIG. 8 described above, the AI algorithm 60 calculates the product of matrixes in order to obtain the relevance degree between pieces of waveform data. As shown as an example in the drawing in the upper part of FIG. 51, the calculation formula is described in a hierarchical loop syntax. The drawing in the lower part of FIG. 51 shows an example of arithmetic processes executed in parallel in the parallel-processing processor 3002. The calculation formulas shown as an example in the drawing in the lower part of FIG. 51 can be divided into n × n arithmetic processes, n × n being the number of combinations of a loop variable i for the first hierarchical level and a loop variable j for the second hierarchical level, for example. Arithmetic processes thus divided are arithmetic processes that are not dependent on each other, and thus can be executed in parallel.

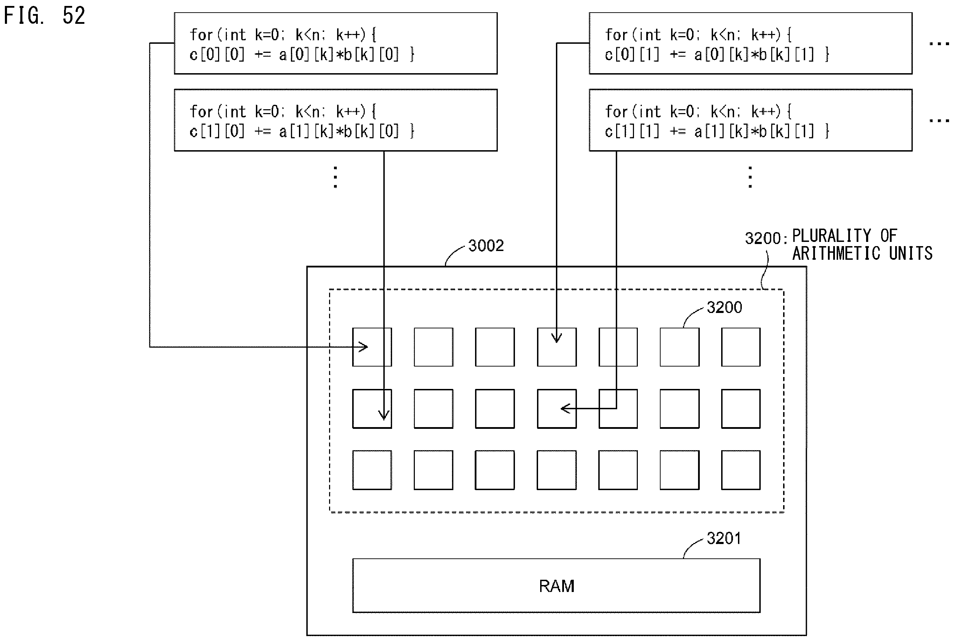

FIG. 52 is a conceptual diagram showing that a plurality of arithmetic processes shown as an example in the drawing in the lower part of FIG. 51 are executed in parallel in the parallel-processing processor 3002.

As shown in FIG. 52, each of the plurality of arithmetic processes is assigned to one of the plurality of arithmetic units 3200 of the parallel-processing processor 3002. The respective arithmetic units 3200 execute in parallel the assigned arithmetic processes. That is, the respective arithmetic units 3200 simultaneously execute the divided arithmetic processes.

Through the arithmetic operation shown as an example in FIGS. 51 and 52 performed by the parallel-processing processor 3002, information regarding the relevance degree between pieces of waveform data forming the set data is obtained, for example. For example, on the basis of the results of the arithmetic operations, the processor 3001, which executes the analysis software 3100, performs analysis regarding the cell type of a cell corresponding to each piece of the waveform data. In the analysis regarding the cell type of each cell, for example, on the basis of information regarding the relevance degree above, information regarding the probability at which a cell corresponding to each piece of waveform data belongs to each of a plurality of cell types is obtained.

The arithmetic operations of the probability at which an analyte in the specimen belongs to each of a plurality of classification types may be performed by a processor different from the parallel-processing processor 3002. For example, the arithmetic results by the parallel-processing processor 3002 may be transferred from the RAM 3201 to the RAM 3017, and on the basis of the arithmetic results read out from the RAM 3017, the processor 3001 may perform arithmetic operations on the information regarding the probability at which the analyte corresponding to each piece of waveform data belongs to each of a plurality of classification types. Alternatively, the arithmetic results by the parallel-processing processor 3002 may be transferred from the RAM 3201 to the analysis unit 300, and a processor installed in the analysis unit 300 may perform arithmetic operations on the information regarding the probability at which the analyte corresponding to each piece of waveform data belongs to each of a plurality of classification types.

The processes shown in FIGS. 51 and 52 may be applied to an arithmetic process (also referred to as a filtering process) regarding a convolution layer in the AI algorithm 60, for example.

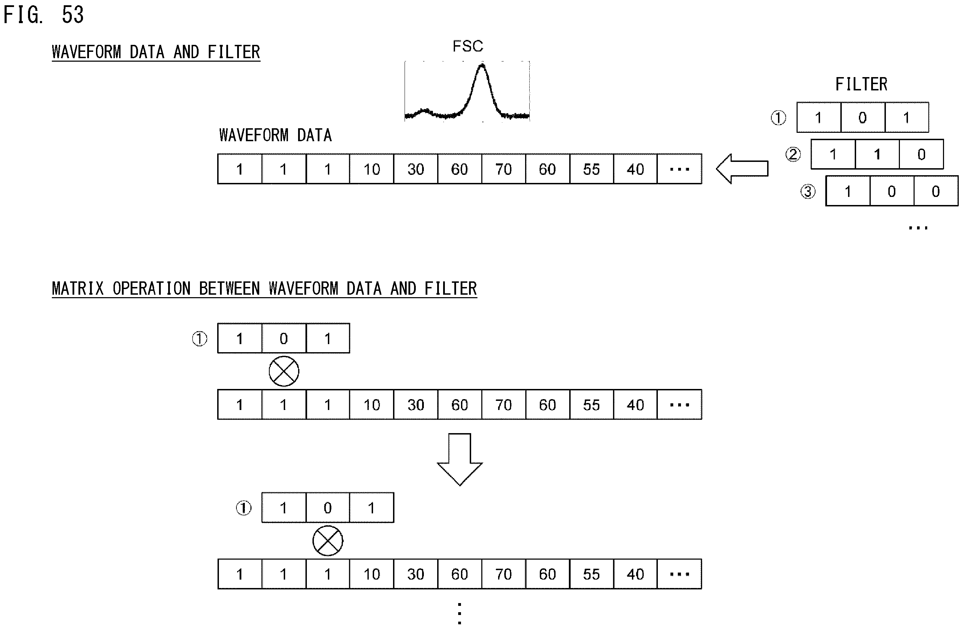

FIG. 53 schematically shows an outline of an arithmetic process regarding a convolution layer.

The drawing in the upper part of FIG. 53 shows waveform data obtained on the basis of forward scattered light, as waveform data that is inputted to the AI algorithm 60. The waveform data of the present embodiment is one-dimensional matrix data, for example. To put it more simply, the waveform data is array data in which elements are arranged in a line. Here, for convenience of description, the number of elements of the waveform data is assumed to be n (n is an integer of 1 or greater). The drawing in the upper part of FIG. 53 shows a plurality of filters. Each filter is generated through a learning process of the AI algorithm 60. Each of the plurality of filters is one-dimensional matrix data indicating features of the waveform data. Although each filter shown in the drawing in the upper part of FIG. 53 is matrix data of 1 row × 3 columns, for example, the number of columns is not limited to three. A matrix operation is performed on each filter and the waveform data that is inputted to the AI algorithm 60, whereby features corresponding to the cell type regarding the waveform data are calculated.

The drawing in the lower part of FIG. 53 shows an outline of a matrix operation between waveform data and a filter. The matrix operation is executed while each filter is shifted with respect to the elements of the waveform data, one by one. Calculation of the matrix operation is executed according to Formula 1 below.

$$\sum_{P=0}^{L-1}\sum_{q=0}^{M-1} h_{pq}x_{i+p,j+q} \qquad \text{... (Formula 1)}$$

In Formula 1, the suffixes of x are variables that indicate the row number and the column number of the waveform data. The suffixes of h are variables that indicate the row number and the column number of the filter. In the example shown in FIG. 53, waveform data is one-dimensional matrix data, and the filter is matrix data of 1 row × 3 columns, and thus, L=1, M=3, p=0, q=0, 1, 2, i=0, and j=0, 1,..., n-1.

The parallel-processing processor 3002 executes in parallel the matrix operation represented by Formula 1, by means of the plurality of respective arithmetic units 3200. On the basis of the arithmetic processes executed by the parallel-processing processor 3002, classification information 82 regarding the type of each analyte in the specimen is generated. The generated classification information 82 is used in generation and display of a test result (analysis result) of the specimen.

The computer 301 is connected to the processor 3001 via the IF part 3006 and the bus 3003, and can receive analysis results obtained by the processor 3001 and the parallel-processing processor 3002. The IF part 3006 is a USB interface, for example. The computer 301 receives, via the IF part 3006, the analysis results obtained by the analysis unit 300, and displays the analysis results on a display device of the computer 301.

The computer 301 may include an operation part implemented by a pointing device including a keyboard, a mouse, or a touch panel. The user such as a doctor or a laboratory technician operates the operation part to input a measurement order to the specimen analyzer 4000, thereby being able to input a measurement instruction in accordance with the measurement order. The user can input an instruction for displaying a test result, to the computer 301 via the operation part. By operating the operation part, the user can view various types of information regarding the test result, such as a numerical value result, a graph, a chart, and flag information provided to the specimen that are based on the analysis.

<Operation of specimen analyzer>

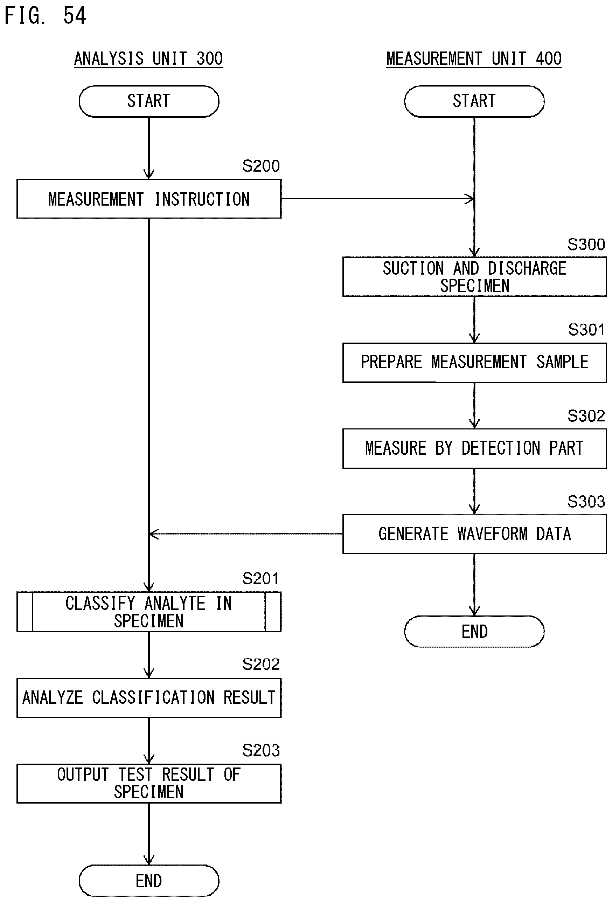

With reference to FIGS. 54 to 56, a specimen analysis operation performed by the specimen analyzer 4000 will be described.

FIG. 54 is a flowchart showing analysis operations performed by the analysis unit 300 and the measurement unit 400.

In step S200, when the processor 3001 of the analysis unit 300 has received a measurement order, the processor 3001 instructs the measurement unit 400 to execute measurement. For example, through the instruction issued to the measurement unit 400, the analysis unit 300 controls operation of each detection part (the FCM detection part 410, the RBC/PLT detection part 4101, the HGB detection part 4102), the specimen suction part 450, and the sample preparation part 440 of the measurement unit 400. The measurement unit 400 starts measurement of a specimen in accordance with the instruction from the analysis unit 300.

In step S300, in accordance with the measurement instruction from the analysis unit 300, the specimen suction part 450 suctions a specimen from a collection tube and discharges the suctioned specimen into a reaction chamber. The measurement instruction from the analysis unit 300 includes information of a measurement channel with respect to which measurement is requested by the measurement order. On the basis of the information of the measurement channel included in the measurement instruction, the specimen suction part 450 discharges the specimen into the reaction chamber of the corresponding measurement channel.

In step S301, in accordance with the measurement instruction from the analysis unit 300, the sample preparation part 440 prepares a measurement sample. Specifically, on the basis of the information of the measurement channel included in the measurement instruction, the sample preparation part 440 supplies a reagent (hemolytic agent and staining liquid) to the reaction chamber having the specimen discharged therein, and mixes the specimen and the reagent. Accordingly, a measurement sample (e.g., WDF measurement sample, RET measurement sample, WPC measurement sample, PLT-F measurement sample, WNR measurement sample) is prepared. The sample preparation part 440 supplies a reagent to a reaction chamber having the specimen discharged therein, and mixes the specimen and the reagent, to prepare an RBC/PLT measurement sample. The sample preparation part 440 supplies a reagent to a reaction chamber having the specimen discharged therein, and mixes the specimen and the reagent, to prepare a hemoglobin measurement sample.

In step S302, in accordance with the measurement instruction from the analysis unit 300, the FCM detection part 410 measures the prepared measurement sample. Specifically, in accordance with the measurement instruction from the analysis unit 300, the apparatus mechanism part 430 sends the measurement sample in the reaction chamber of the sample preparation part 440 to the FCM detection part 410. The measurement sample sent from the reaction chamber is caused to flow in the flow cell 4113, and is irradiated with laser light by the light source 4111. When an analyte contained in the measurement sample passes through the flow cell 4113, light is applied to the analyte. Then, forward scattered light, side scattered light, and fluorescence generated from the analyte are detected by the light receiving elements 4116, 4121, 4122, respectively, and analog optical signals according to the received light intensities are outputted. Each optical signal is processed by the analog processing part 420, and then is outputted to the A/D converter 461.

The RBC/PLT detection part 4101 performs measurement of blood cells by a sheath flow DC detection method on the basis of the RBC/PLT measurement sample. The HGB detection part 4102 performs measurement of hemoglobin by an SLS-hemoglobin method on the basis of the hemoglobin measurement sample. An analog signal detected by the RBC/PLT detection part 4101 is processed by the analog processing part 4201, and then outputted to the A/D converter 4611. An analog signal detected by the HGB detection part 4102 is processed by the analog processing part 4202, and then outputted to the A/D converter 4612.

In step S303, as described above, the A/D converter 461 generates digital data by sampling at a predetermined rate each analog optical signal, and generates waveform data corresponding to each of analytes on the basis of the digital data. The waveform data generated by the A/D converter 461 is transferred directly to a RAM by, for example, DMA transfer, not via the processor 3001 of the analysis unit 300. Accordingly, waveform data based on a forward scattered light signal, waveform data corresponding to side scattered light, and waveform data corresponding to fluorescence, which have been obtained from each analyte, are taken into the RAM 3017.

The A/D converter 4611 generates digital data by sampling at a predetermined rate the analog signal from the RBC/PLT detection part 4101. The A/D converter 4612 generates digital data by sampling at a predetermined rate the analog signal from the HGB detection part 4102. These pieces of digital data may also be taken into the RAM 3017.

In step S201, the processor 3001 of the analysis unit 300 causes the parallel-processing processor 3002 to execute the AI analysis on the set data being a collection of a plurality of pieces of the waveform data by using the AI algorithm 60. In addition to the AI analysis above, the processor 3001 may, as necessary, execute the calculation processing analysis with respect to a representative value, of the waveform data, corresponding to a feature of the analyte. When the AI analysis and the calculation processing analysis are executed while being apportioned, the apportioning is performed as described above. Accordingly, the analyte in the specimen is classified. Although the process of the AI analysis in step S201 will be described later, the processor 3001 obtains, as a result of the process using the parallel-processing processor 3002, classification information 82 of each individual analyte in the specimen, and obtains a label value 83 as a classification result, for example.

In step S202, the processor 3001 analyzes the classification result including the label value 83 by using a program stored in the storage 3004, and generates a test result (analysis result) of the specimen. In step S202, for example, on the basis of the classification result of each individual analyte, the number of the analytes is counted for each type of analyte.

For example, in a case of an example in which a test of blood cells in a blood specimen is performed, if, in one specimen, there are N classification results provided with a label value "1" which indicates neutrophil, a counting result that the number of neutrophils = N is obtained as a test result of the specimen. The processor 3001 obtains the counting result regarding the measurement item corresponding to the measurement channel on the basis of the classification result, and stores the counting result, together with identification information of the specimen, into the storage 3004.

Here, the measurement item corresponding to the measurement channel is an item of which the counting result is requested by the measurement order. For example, a measurement item corresponding to the WDF channel includes a measurement item of the number of cells of the five classifications of white blood cells, i.e., monocytes, neutrophils, lymphocytes, eosinophils, and basophils. A measurement item corresponding to the RET channel includes a measurement item of the number of reticulocytes. A measurement item corresponding to PLT-F includes a measurement item of the number of platelets. A measurement item corresponding to WPC includes a measurement item of the number of hematopoietic progenitor cells. A measurement item corresponding to WNR includes a measurement item of the number of white blood cells and nucleated red blood cells.

The counting result is not limited to that of an item (also referred to as "reportable item") for which measurement as listed above is requested, and can also include a counting result of another cell of which measurement can be performed in the same measurement channel. For example, in the case of the WDF channel, immature granulocytes (IG) and abnormal cells are also included in the counting result in addition to the five classifications of white blood cells.

Further, the processor 3001 analyzes the obtained counting result to generate a test result of the specimen, and stores the result into the storage 3004. The analysis of the counting result includes performing determination as to, for example, whether the counting result is in a normal value range, whether any abnormal cell has been detected, whether deviation from the previous test result is in an allowable range, and the like.

In step S203, the analysis unit 300 causes, for example, a display part of the computer 301 to display the generated test result.

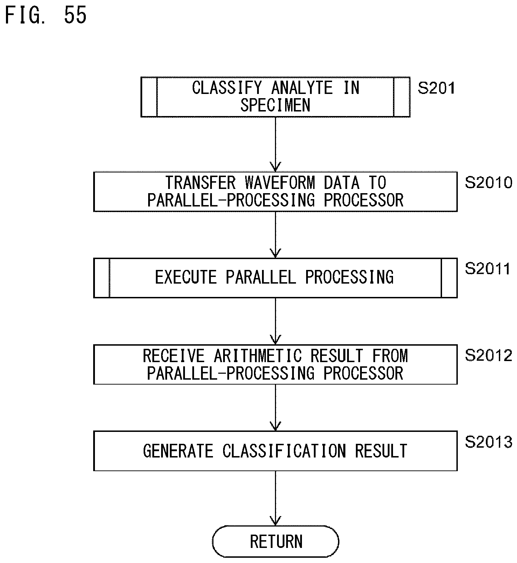

FIG. 55 is a flowchart showing details of the AI analysis performed in step S201 in FIG. 54.

Step S201 is executed by the processor 3001 in accordance with operation of the analysis software 3100.

In step S2010, the processor 3001 causes the waveform data taken into the RAM 3017 in step S303, to be transferred to the parallel-processing processor 3002. For example, the waveform data is DMA-transferred from the RAM 3017 to the RAM 3201. At this time, for example, the processor 3001 controls the bus controller 3005 to DMA-transfer the waveform data from the RAM 3017 to the RAM 3201.

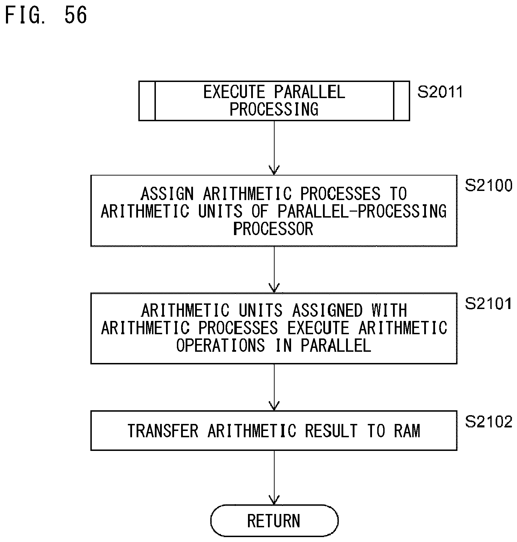

In step S2011, the processor 3001 instructs the parallel-processing processor 3002 to execute parallel processing on the waveform data. The processor 3001 instructs the execution of parallel processing by calling a kernel function of the parallel-processing processor 3002, for example. The process executed by the parallel-processing processor 3002 will be described later with reference to FIG. 56. The processor 3001 instructs the parallel-processing processor 3002 to execute a matrix operation regarding the AI algorithm 60, for example. The waveform data corresponding to each of the analytes in the specimen is inputted to the AI algorithm 60. The set data being a collection of a plurality of pieces of the waveform data inputted to the AI algorithm 60 is subjected to arithmetic operations performed by the parallel-processing processor 3002.

In step S2012, the processor 3001 receives results (classification result) of arithmetic operations executed by the parallel-processing processor 3002. The arithmetic results are DMA-transferred from the RAM 3201 to the RAM 3017, for example. In step S2013, on the basis of the arithmetic results by the parallel-processing processor 3002, the processor 3001 generates a classification result of the type of each analyte.

FIG. 56 is a flowchart showing details of step S2011 in FIG. 55.

Step S2011 is executed by the parallel-processing processor 3002 on the basis of an instruction from the processor 3001.

In step S2100, the processor 3001, which executes the analysis software 3100, causes the parallel-processing processor 3002 to execute assignment of arithmetic processes to the arithmetic units 3200. For example, the processor 3001 causes the parallel-processing processor 3002 to execute assignment of arithmetic processes to the arithmetic units 3200, by calling a kernel function of the parallel-processing processor 3002. For example, a matrix operation regarding the AI algorithm 60 is divided into a plurality of arithmetic processes, and the respective divided arithmetic processes are assigned to the arithmetic units 3200. Waveform data corresponding to each of the analytes in the specimen is inputted to the AI algorithm 60. A matrix operation corresponding to the waveform data is divided into a plurality of arithmetic processes, to be assigned to the arithmetic units 3200.

In step S2101, the arithmetic processes are processed in parallel by a plurality of arithmetic units 3200. The arithmetic processes are executed on the plurality of pieces of waveform data. In step S2102, arithmetic results generated through the parallel processing by the plurality of arithmetic units 3200 are transferred from the RAM 3201 to the RAM 3017. The arithmetic results are DMA-transferred from the RAM 3201 to the RAM 3017, for example.

In step S201 in FIG. 54, the processor 3001 of the analysis unit 300 may use the AI algorithm 60 on digital data based on the analog signal from the RBC/PLT detection part 4101, thereby obtaining classification information 82 of a measurement item (e.g., the number of red blood cells, hematocrit value, or the like) that corresponds to the RBC/PLT channel. Further, the processor 3001 may use the AI algorithm 60 on digital data based on the analog signal from the HGB detection part 4102, thereby obtaining classification information 82 of a measurement item (e.g., hemoglobin content, or the like) with respect to the HGB channel.

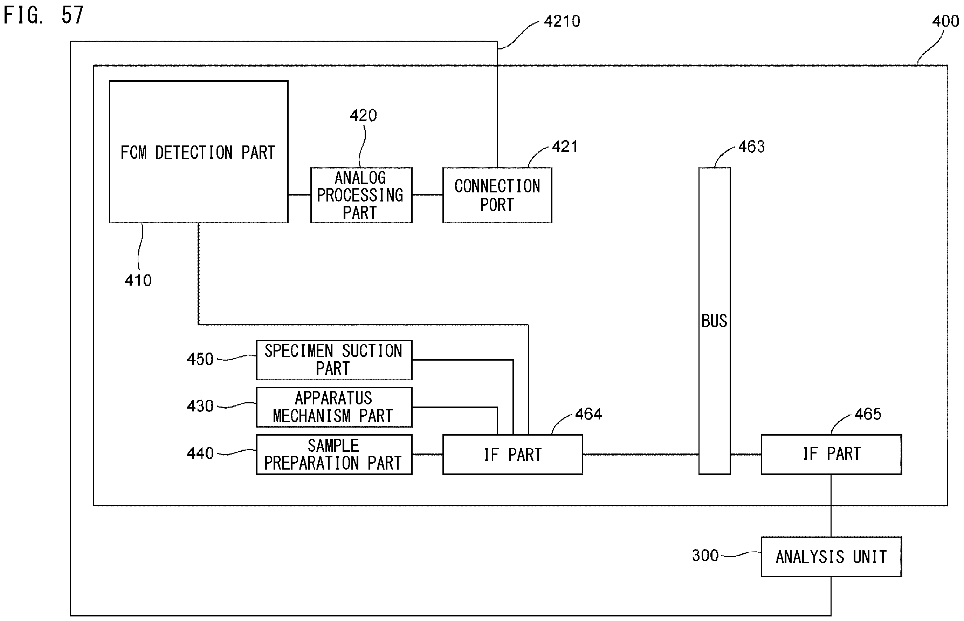

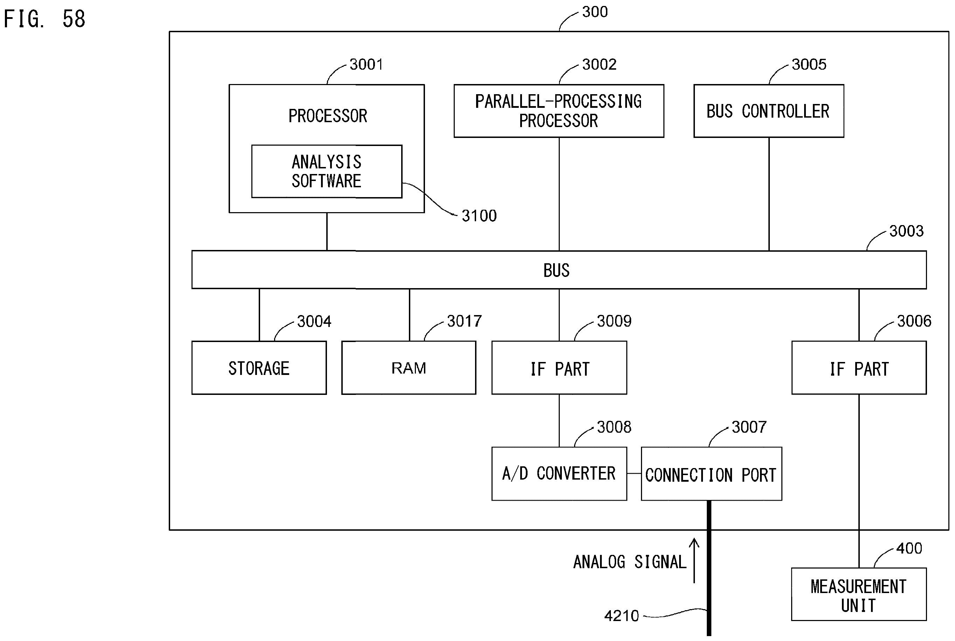

Next, with reference to FIGS. 57 and 58, another configuration example of the specimen analyzer 4000 composed of the measurement unit 400 and the analysis unit 300 will be described.

FIG. 57 is a block diagram showing another functional configuration of the electric/control system of the measurement unit 400.

In the example shown in FIG. 57, each analog optical signal processed by the analog processing part 420 is transmitted to the analysis unit 300 via a connection port 421. A connection cable 4210 is connected to the connection port 421. The other configurations shown in FIG. 57 have configurations and functions similar to those of the measurement unit 400 in the embodiments described above.

FIG. 58 is a block diagram showing another functional configuration of the electric/control system of the analysis unit 300.

In the example shown in FIG. 58, the analysis unit 300 is connected to the measurement unit 400 via the IF part 3006. The bus 3003 is a transmission line having a data transfer rate of not less than several hundred MB/s, for example. The bus 3003 may be a transmission line having a data transfer rate of not less than 1 GB/s. The bus 3003 performs data transfer on the basis of the PCI-Express or PCI-X standard, for example. Configurations of the processor 3001, the parallel-processing processor 3002, the storage 3004, and the RAM 3017, and processes executed by these are similar to the configurations and the processes described above.

The analysis unit 300 includes a connection port 3007, an A/D converter 3008, and an IF part 3009.

The connection port 3007 is connected to the connection port 421 (see FIG. 57) of the measurement unit 400 via the connection cable 4210. The connection cable 4210 includes transmission paths of which the number corresponds to the types of analog signals transmitted from the measurement unit 400 to the analysis unit 300, for example. For example, the connection cable 4210 is implemented by twisted-pair cables, and has pairs of wires, the number of the pairs corresponding to the types of analog signals transmitted to the analysis unit 300. For noise reduction during signal transmission, the connection cable 4210 has a length of 1 meter or less, for example.

The A/D converter 3008 is connected to the connection port 3007. The A/D converter 3008 samples each analog optical signal outputted from the measurement unit 400 as described above, to generate waveform data corresponding to each analyte in the specimen. The generated waveform data is stored into the storage 3004 or the RAM 3017 via the IF part 3009 and the bus 3003. The transmission path from the connection port 3007 to the A/D converter 3008 may also have wires of which the number corresponds to the types of optical signals transmitted to the analysis unit 300.

The processor 3001 and the parallel-processing processor 3002 execute arithmetic processes on the waveform data stored in the storage 3004 or the RAM 3017. The analysis software 3100, which operates on the processor 3001, is similar to the analysis software 3100 shown in FIG. 49. By executing the analysis software 3100, the processor 3001 generates, through operations similar to those described above, classification information 82 regarding the type of each analyte in the specimen.

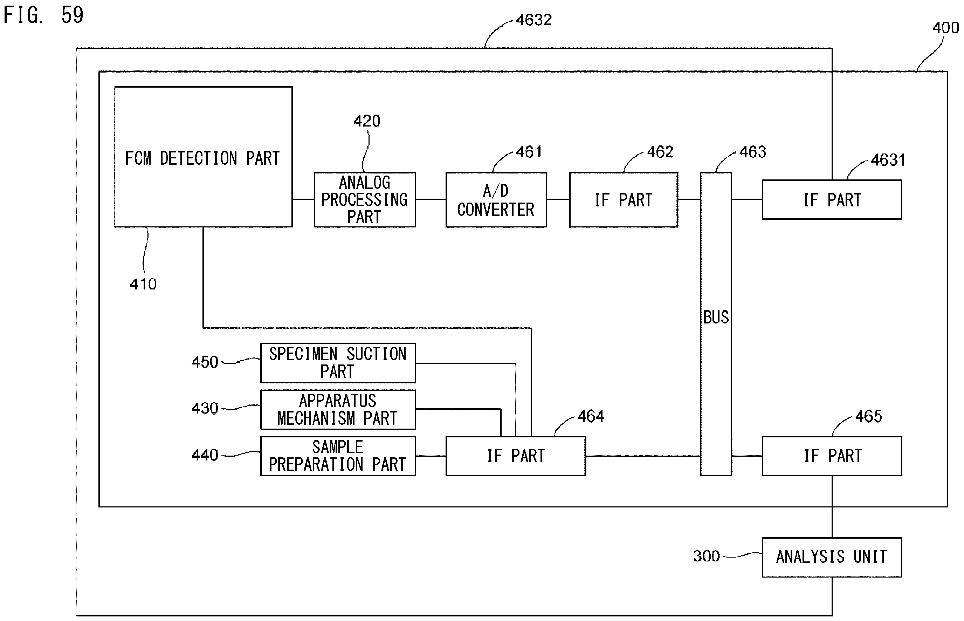

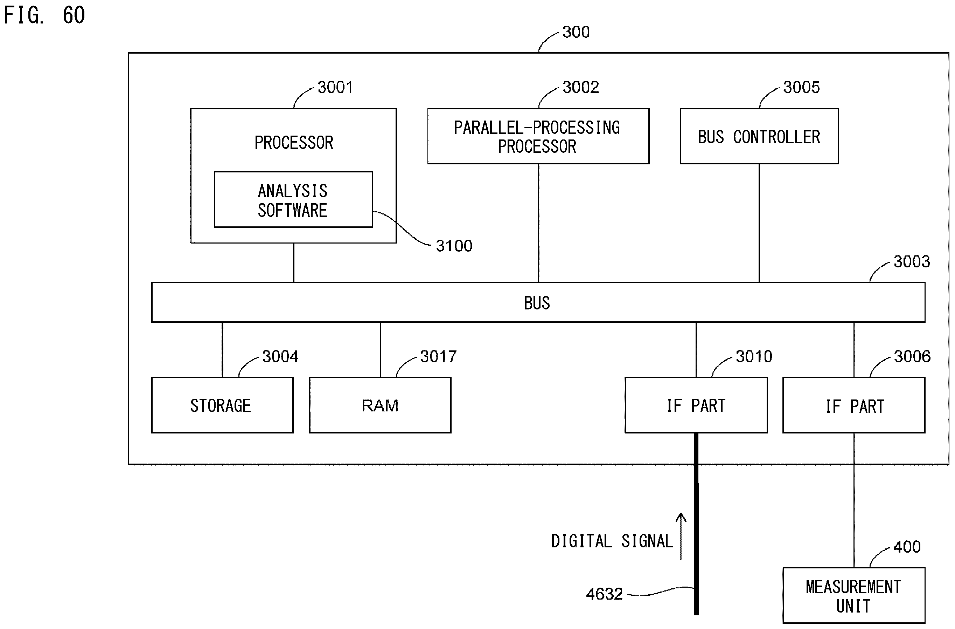

Next, with reference to FIGS. 59 and 60, another configuration example of the specimen analyzer 4000 composed of the measurement unit 400 and the analysis unit 300 will be described.

FIG. 59 is a block diagram showing another functional configuration of the electric/control system of the measurement unit 400.

The measurement unit 400 shown in FIG. 59 includes an IF part 4631 for transmitting waveform data generated by the A/D converter 461, to the analysis unit 300. A transmission line 4632 is connected to the IF part 4631. The other configurations and functions are similar to those of the measurement unit 400 described above.

The IF part 4631 is an interface serving as a dedicated line having a communication band of not less than 1 gigabit/second, for example. For example, the IF part 4631 is an interface according to Gigabit Ethernet, USB 3.0, or Thunderbolt 3. When the IF part 4631 is of Gigabit Ethernet, the transmission line 4632 is a LAN cable. When the IF part 4631 is of USB 3.0, the transmission line 4632 is a USB cable according to USB 3.0. The transmission line 4632 is a dedicated transmission line for transmitting digital data between the measurement unit 400 and the analysis unit 300, for example.

FIG. 60 is a block diagram showing another functional configuration of the electric/control system of the analysis unit 300.

The analysis unit 300 shown in FIG. 60 includes an IF part 3010. The other configurations and functions are similar to those of the analysis unit 300 described above. The analysis unit 300 may be connected to a plurality of the measurement units 400 via a plurality of the IF parts 3010 and a plurality of the IF parts 3006.

The analysis software 3100, which operates on the processor 3001, has functions similar to those of the analysis software 3100 described above. The analysis software 3100 analyzes the type of each analyte in the specimen through operations similar to those in the related description above.

In the configuration shown in FIGS. 59 and 60, the A/D converter 461 of the measurement unit 400 generates digital waveform data on the basis of each analog optical signal generated in the FCM detection part 410. The waveform data is sent to the analysis unit 300 via the IF part 462, the bus 463, the IF part 4631, and the transmission line 4632.

The measurement unit 400 and the analysis unit 300 are connected to each other in a one-to-one relationship via the transmission line 4632, for example. The transmission line 4632 in this case is a transmission line that provides no transmission of data related to an apparatus other than components (e.g., the measurement unit 400 and the analysis unit 300) forming the specimen analyzer 4000. The transmission line 4632 is a transmission line different from an intranet or the Internet, for example. Accordingly, even when waveform data generated in the measurement unit 400 is transmitted to the analysis unit 300, bottleneck in the communication speed of transmission of digital data can be avoided.

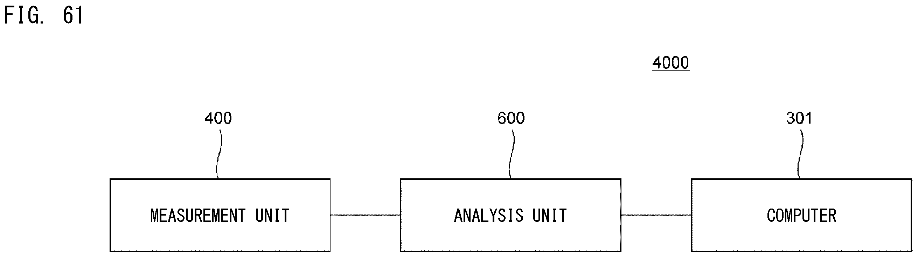

Next, with reference to FIGS. 61 to 65, another configuration example of the specimen analyzer 4000 will be described.

FIG. 61 is a block diagram showing another functional configuration of the electric/control system of the specimen analyzer 4000.

In the present configuration example, an analysis unit 600 is provided between the measurement unit 400 and the computer 301. That is, in the configuration in FIGS. 61 to 65, the specimen analyzer 4000 includes the measurement unit 400, the computer 301, and the analysis unit 600. The analysis unit 600 analyzes the type of each measured cell. As described later, a parallel-processing processor 6002 of the present configuration example is installed to the specimen analyzer 4000 in the form of being incorporated in the analysis unit 600.

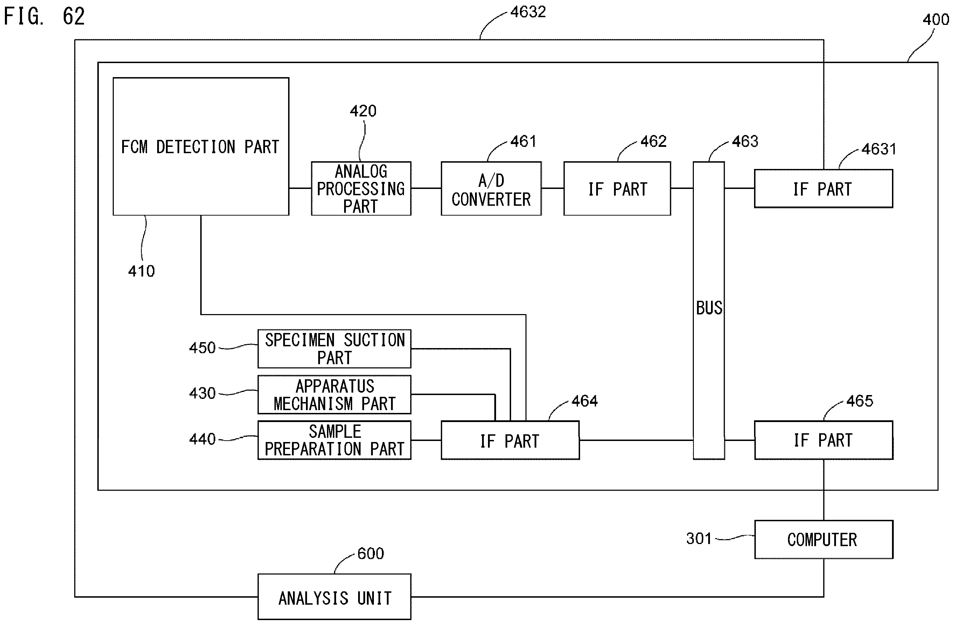

FIG. 62 is a block diagram showing another functional configuration of the electric/control system of the measurement unit 400.

In the measurement unit 400 in FIG. 62, when compared with the configuration in FIG. 59, the computer 301 is connected to the IF part 465, and the analysis unit 600 is provided between the IF part 4631 and the computer 301. The analysis unit 600 is communicably connected to the IF part 4631 and the computer 301. The analysis unit 600 may be connected to a plurality of the measurement units 400. The analysis unit 600 may be connected to a plurality of the computers 301.

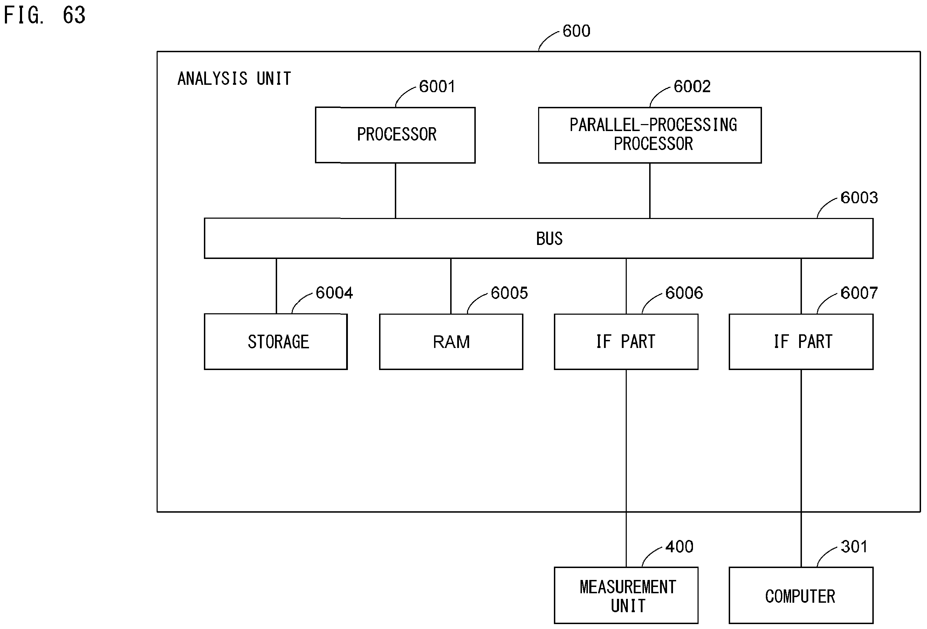

FIG. 63 is a block diagram showing another functional configuration of the electric/control system of the analysis unit 600.

The analysis unit 600 includes a processor 6001, the parallel-processing processor 6002, a bus 6003, a storage 6004, a RAM 6005, and IF parts 6006, 6007. Each component of the analysis unit 600 is connected to the bus 6003.

The bus 6003 is a transmission line having a data transfer rate of not less than several hundred MB/s, for example. The bus 6003 may be a transmission line having a data transfer rate of not less than 1 GB/s. The bus 6003 performs data transfer on the basis of the PCI-Express or PCI-X standard, for example. The analysis unit 600 may be connected to a plurality of the measurement units 400 via a plurality of the IF parts 6006. When a plurality of the measurement units 400 are provided, an analysis unit 600 may be connected to each of the measurement units 400. In this case, for example, a plurality of the measurement units 400 and a plurality of the analysis units 600 are connected in a one-to-one relationship, respectively.

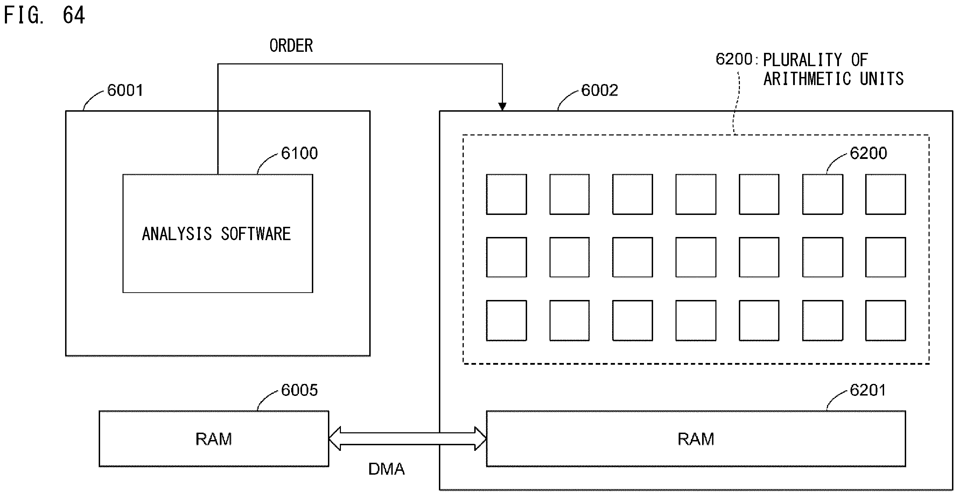

FIG. 64 shows a configuration example of the parallel-processing processor 6002 which executes arithmetic processes.

The processor 6001 and the parallel-processing processor 6002 have configurations and functions similar to those of the processor 3001 and the parallel-processing processor 3002 described above. The parallel-processing processor 6002 includes a plurality of arithmetic units 6200 and a RAM 6201. Analysis software 6100, which analyzes the type of each analyte in the specimen, operates on the processor 6001. The analysis software 6100 operating on the processor 6001 has functions similar to those of the analysis software 3100 shown in FIG. 50. The analysis software 6100 analyzes the type of the analyte in the specimen through operations similar to those described in FIG. 50. The analysis software 6100 transmits classification information 82 of the analyte in the specimen to the computer 301 via the IF part 6007.

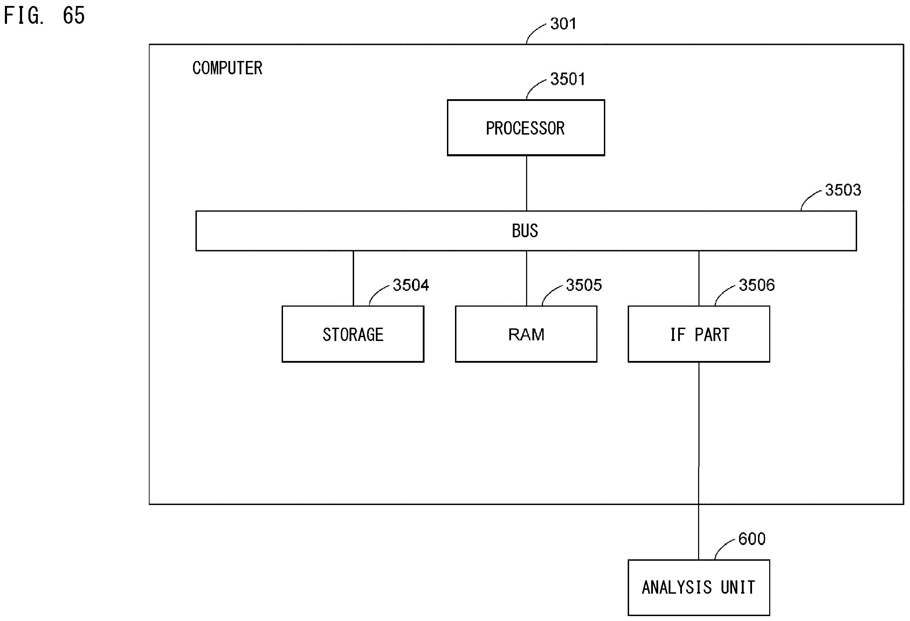

FIG. 65 is a block diagram showing a functional configuration of the electric/control system of the computer 301.

The computer 301 in FIG. 65 has a configuration similar to that in which the parallel-processing processor 6002 is omitted from the analysis unit 600 in FIG. 63. The computer 301 includes a processor 3501, a bus 3503, a storage 3504, a RAM 3505, and an IF part 3506.

The analysis software 3100 need not necessarily operate on the processor 3501. The computer 301 receives, via the IF part 3506, analysis results obtained by the analysis unit 600. The IF part 3506 is of Ethernet or USB, for example. The IF part 3506 may be an interface capable of performing wireless communication.

In the configuration of FIGS. 62 to 65, each analog optical signal of a cell generated in the FCM detection part 410 is converted to digital waveform data by the A/D converter 461 in the measurement unit 400. The waveform data is sent to the analysis unit 600 via the IF part 462, the bus 463, the IF part 4631, and the transmission line 4632.

As described above, the IF part 4631 is a dedicated interface that connects the measurement unit 400 and the analysis unit 600, and the IF part 4631 connects the measurement unit 400 and the analysis unit 600 in a one-to-one relationship. In other words, the transmission line 4632 is a transmission line that provides no transmission of data related to an apparatus other than components (e.g., the measurement unit 400 and the analysis unit 300) forming the specimen analyzer 4000, for example. The transmission line 4632 is a transmission line different from an intranet or the Internet. Accordingly, even when waveform data generated in the measurement unit 400 is transmitted to the analysis unit 600, bottleneck in the communication speed of transmission of the waveform data can be avoided.

In this case, steps S200 to S202 in FIG. 54 are executed by the analysis unit 600, and step S203 is executed by the computer 301.

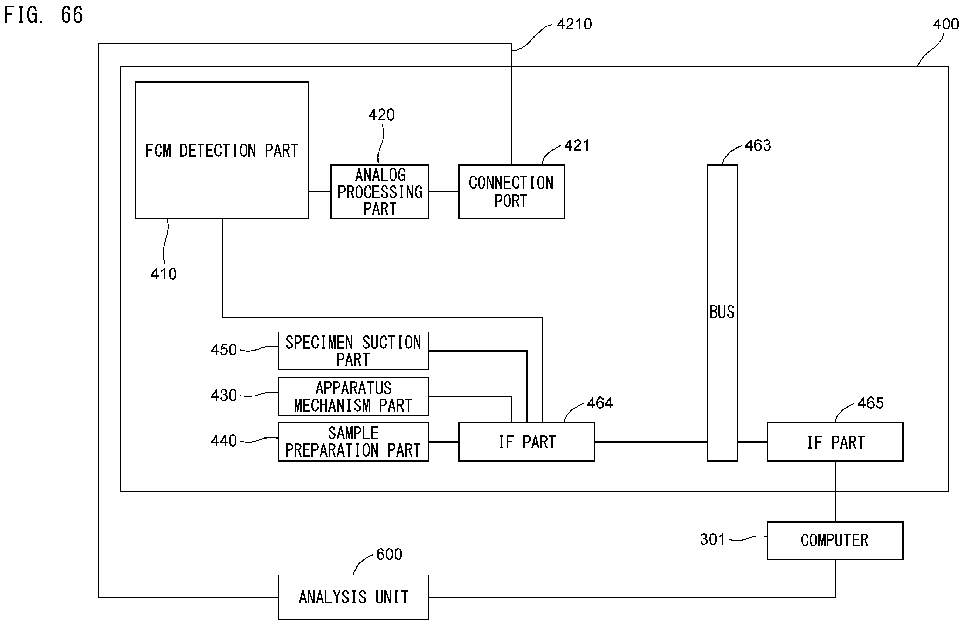

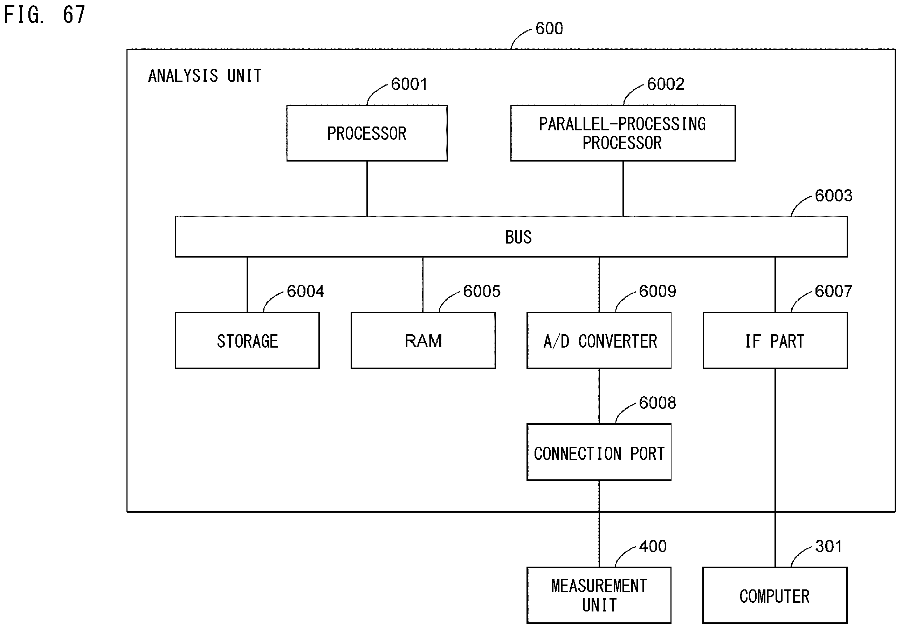

Next, with reference to FIGS. 66 and 67, another configuration example of the specimen analyzer 4000 in FIG. 61 will be described. The specimen analyzer 4000 of this example includes the measurement unit 400, the computer 301, and the analysis unit 600.

In the measurement unit 400 in FIG. 66, when compared with the configuration in FIG. 57, the computer 301 is connected to the IF part 465 and the analysis unit 600 is provided between the connection port 421 and the computer 301. The analysis unit 600 is communicably connected to the connection port 421 and the computer 301. The measurement unit 400 transmits each analog optical signal to the analysis unit 600 via the connection cable 4210.

When compared with the configuration in FIG. 63, the analysis unit 600 in FIG. 67 includes a connection port 6008 and an A/D converter 6009 in place of the IF part 6006.

The analog optical signal transmitted from the measurement unit 400 via the connection cable 4210 is inputted to the A/D converter 6009 via the connection port 6008. The A/D converter 6009 generates waveform data from the optical signal through a process similar to that of the A/D converter 461.

The analysis unit 600 may be connected to a plurality of the measurement units 400 via a plurality of the connection ports 6008. When a plurality of the measurement units 400 are provided, an analysis unit 600 may be connected to each of the measurement units 400. In this case, for example, a plurality of the measurement units 400 and a plurality of the analysis units 600 are connected in a one-to-one relationship, respectively.

In the configuration in FIGS. 66 and 67, in step S303 in FIG. 54, the analysis unit 600 generates waveform data on the basis of each analog optical signal transmitted from the measurement unit 400. Steps S200 to S202 in FIG. 54 are executed by the analysis unit 600, and step S203 is executed by the computer 301.

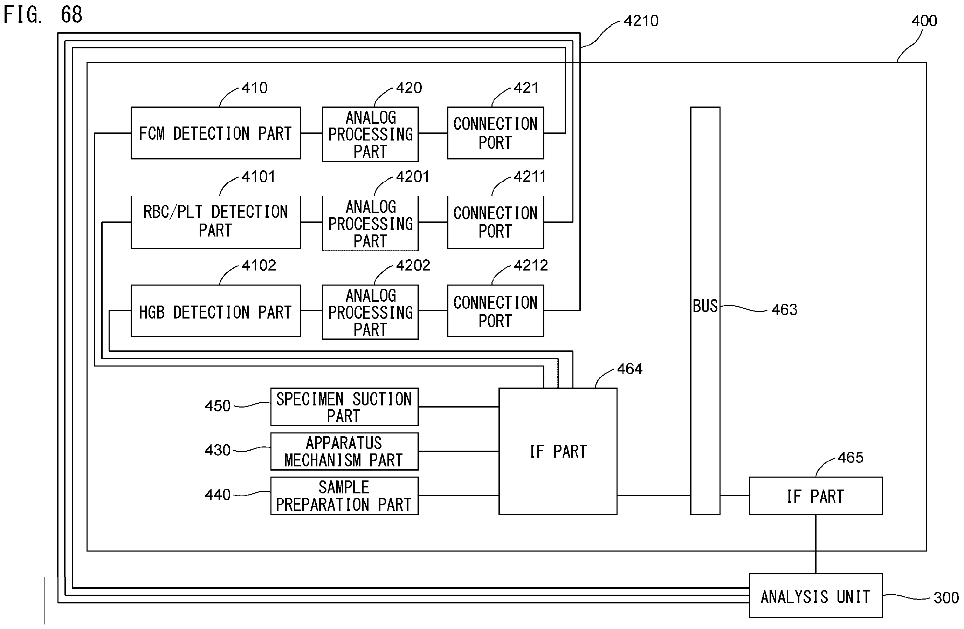

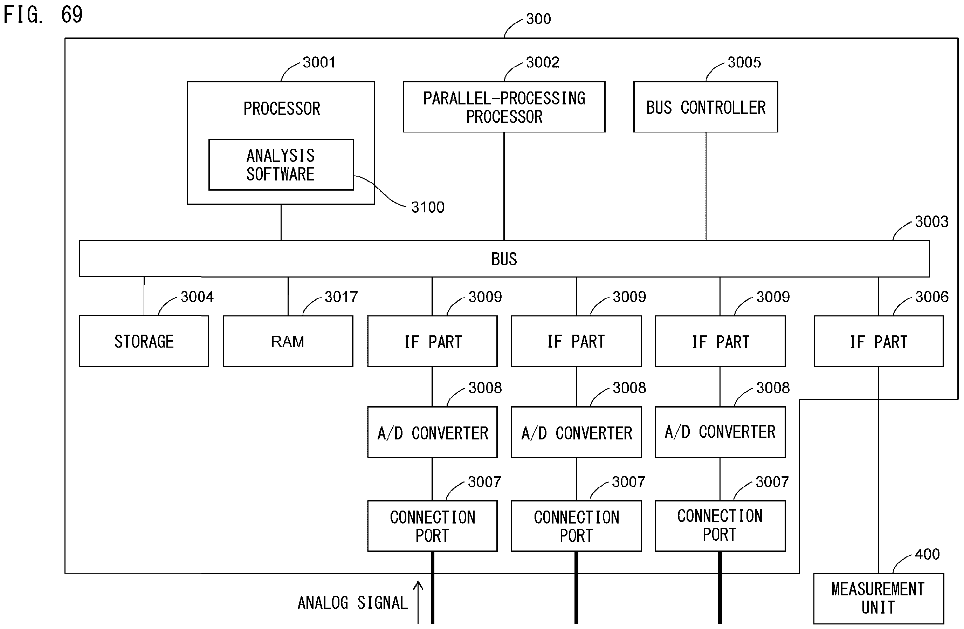

Next, with reference to FIGS. 68 and 69, another configuration example of the measurement unit 400 and the analysis unit 300 of the specimen analyzer 4000 will be described.

When compared with the configuration in FIG. 34, the measurement unit 400 in FIG. 68 includes the connection ports 421, 4211, 4212 in place of the A/D converters 461, 4611, 4612 and the IF part 462. The analog optical signals obtained in the detection parts are transmitted to the analysis unit 300 via the connection cables 4210, respectively.

When compared with the configuration in FIG. 58, the analysis unit 300 in FIG. 69 includes three sets each composed of the connection port 3007, the A/D converter 3008, and the IF part 3009. The three connection ports 3007 are connected to the connection ports 421, 4211, 4212 in FIG. 68, respectively.

In the configuration in FIGS. 68 and 69, in step S303 in FIG. 54, the analysis unit 300 generates waveform data on the basis of each analog optical signal transmitted from the measurement unit 400.

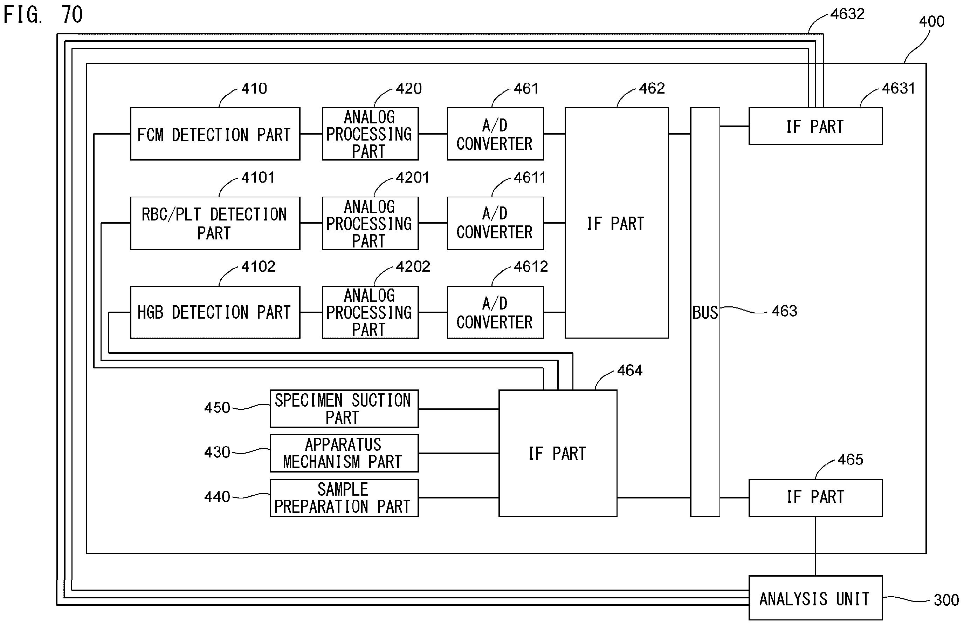

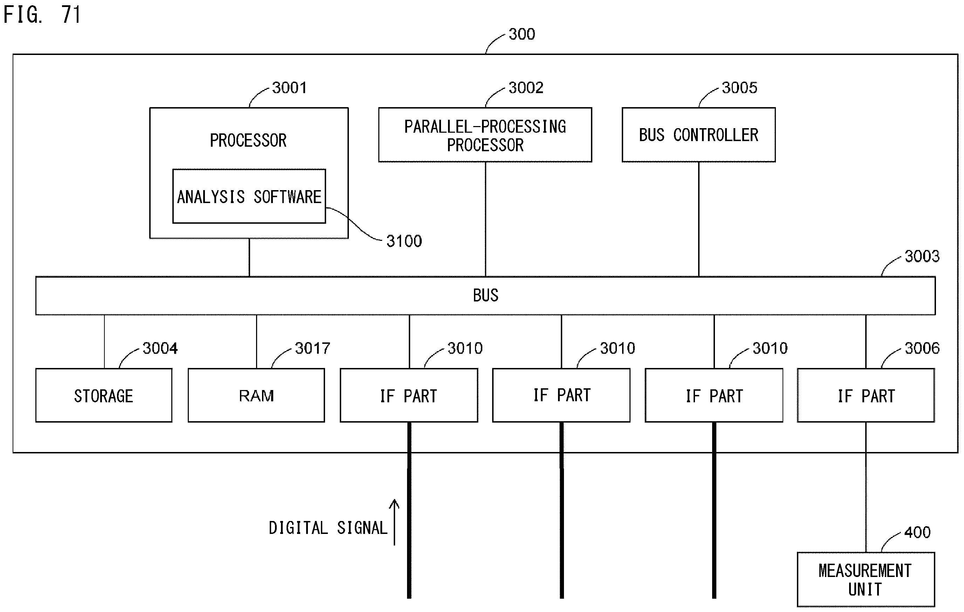

Next, with reference to FIGS. 70 and 71, another configuration example of the measurement unit 400 and the analysis unit 300 of the specimen analyzer 4000 will be described.

When compared with the configuration in FIG. 34, the measurement unit 400 in FIG. 70 includes the IF part 4631. The A/D converters 461, 4611, 4612 generate waveform data on the basis of the analog optical signals obtained in the corresponding detection parts, respectively. Pieces of the waveform data corresponding to the respective detection parts are transmitted to the analysis unit 300 via the transmission lines 4632, respectively.

When compared with the configuration in FIG. 60, the analysis unit 300 in FIG. 71 includes three IF parts 3010. The three IF parts 3010 are connected to the transmission lines 4632 in FIG. 70, respectively.

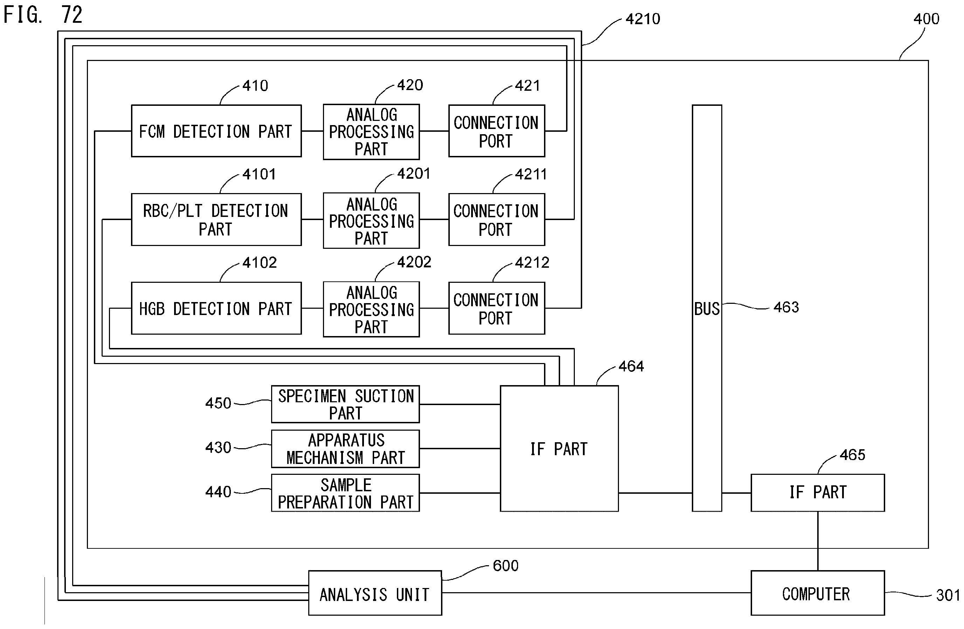

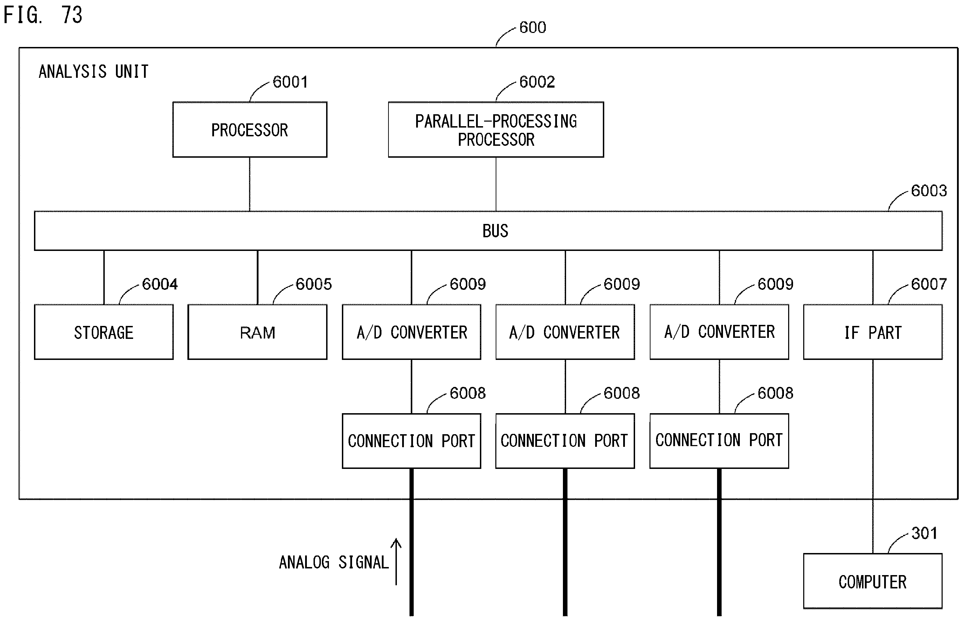

Next, with reference to FIGS. 72 and 73, another configuration example of the measurement unit 400 and the analysis unit 300 of the specimen analyzer 4000 will be described.

In the measurement unit 400 in FIG. 72, when compared with the configuration in FIG. 68, the computer 301 is connected to the IF part 465, and the analysis unit 600 is disposed between the connection ports 421, 4211, 4212 and the computer 301. The analysis unit 600 is communicably connected to the connection ports 421, 4211, 4212 and the computer 301. The analysis unit 600 and the computer 301 are connected so as to be able to transmit/receive digital data to/from each other.

When compared with the configuration in FIG. 67, the analysis unit 600 in FIG. 73 includes three sets of the connection port 6008 and the A/D converter 6009. The three connection ports 6008 are connected to the connection ports 421, 4211, 4212 in FIG. 72, respectively.

Next, the data sizes of waveform data and digital data will be described.

In the present embodiment, for example, with respect to one analyte in the specimen, sampling is performed for each of an analog optical signal (FSC) based on forward scattered light, an analog optical signal (SSC) based on side scattered light, and an analog optical signal (FL) based on fluorescence.

Examples of the sampling rate include sampling at 1024 points at a 10 nanosecond interval, sampling at 128 points at an 80 nanosecond interval, sampling at 64 points at a 160 nanosecond interval, and the like. The data amount is 2 bytes per sampling, for example. With respect to each of FSC, SSC, and FL, data (in the case of the rate of 1024 points, 2 bytes×1024=2048 bytes) of an amount corresponding to the sampling rate is obtained. This data amount is the data amount per analyte in the specimen.

In a single measurement, FSC, SSC, and FL are measured with respect to at least 100 analytes, for example.

In a single measurement, FSC, SSC, and FL may be measured with respect to at least 1000 analytes, for example. In a single measurement, FSC, SSC, and FL may be measured with respect to about 10000 to about 140000 analytes, for example. Therefore, when the number of analytes measured in a single measurement is 100000 and the sampling rate is 1024, the data amount of digital data of each of FSC, SSC, and FL is 2 bytes$\times$1024$\times$100000=204,800,000 bytes, and the data amount in total of FSC, SSC, and FL is 614,400,000 bytes.

Further, FSC, SSC, and FL are measured for each measurement channel. When the number of analytes measured in a single measurement is 100000, the sampling rate is 1024, and the number of measurement channels is 5, the data amount of each of FSC, SSC, and FL is 2 bytes$\times$1024$\times$100000$\times$5=1,024,000,000 bytes, and the data amount in total of FSC, SSC, and FL is 3,072,000,000 bytes.

Thus, the volume of digital data is several hundred megabytes to several gigabytes per specimen, for example, and is at least 1 gigabyte depending on the number of analytes, the sampling rate, and the number of measurement channels.

According to the present embodiment, when digital data having a huge volume of several hundred megabytes to several gigabytes per specimen is analyzed, the analysis process using the AI algorithm 60 is concluded inside the specimen analyzer 4000 as described above, and the digital data is not transmitted, via the Internet or an intranet, to an analysis server provided outside the specimen analyzer 4000. Therefore, decrease in the throughput associated with increase in communication load caused by transmission of the digital data from the specimen analyzer 4000 to the analysis server can be avoided.

[Embodiment 7]

<Configuration of waveform data analysis system>

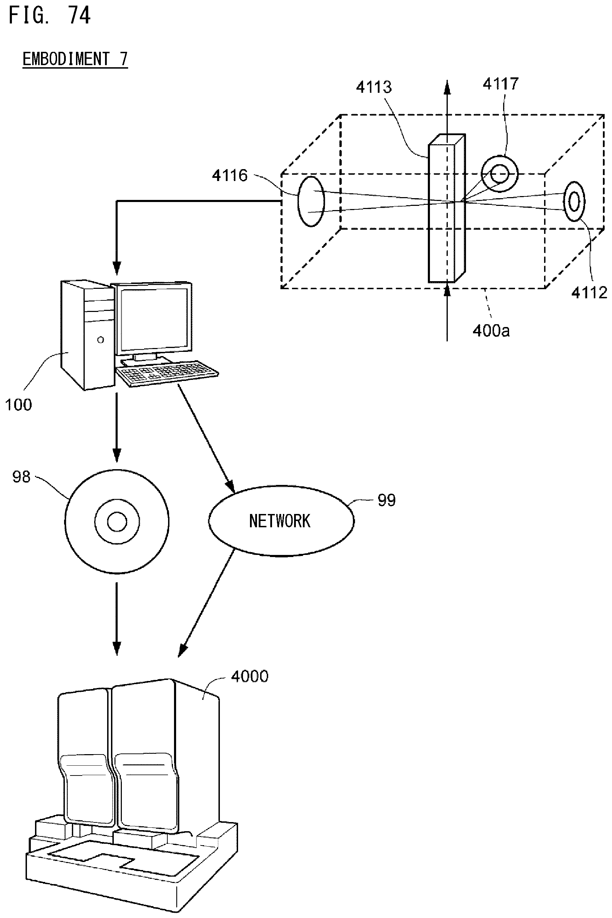

FIG. 74 schematically shows a configuration of a waveform data analysis system according to Embodiment 7.

The configuration of a measurement unit 400a is similar to that of the measurement unit 400 described above. In the measurement unit 400a, a measurement sample prepared on the basis of a specimen is sent to the flow cell 4113. The light source 4111 applies light to the measurement sample supplied to the flow cell 4113, and the light receiving elements 4116, 4121, 4122 detect forward scattered light, side scattered light, and fluorescence generated from each analyte in the measurement sample. The measurement unit 400a generates waveform data from optical signals based on forward scattered light, side scattered light, and fluorescence outputted from the light receiving elements 4116, 4121, 4122, and transmits the generated waveform data to a deep learning apparatus 100.

The deep learning apparatus 100 is a vendor-side apparatus. The deep learning apparatus 100 receives training waveform data obtained by the measurement unit 400a. The generation method of the training waveform data has been described above. The AI algorithm 60 stored in the deep learning apparatus 100 is a deep learning algorithm, for example. The deep learning apparatus 100 causes the AI algorithm 60 configured as a neural network before being trained, to learn by using training data, and provides the user with the AI algorithm 60 having been trained by the training data. The AI algorithm 60 configured as a learned neural network is provided to the specimen analyzer 4000 from the deep learning apparatus 100 through a storage medium 98 or a communication network 99. The storage medium 98 is a computer-readable non-transitory tangible storage medium such as a DVD-ROM or a USB memory, for example.

The deep learning apparatus 100 is implemented as a general-purpose computer, for example, and performs a deep learning process on the basis of a flowchart described later.

The specimen analyzer 4000 executes the AI analysis on waveform data corresponding to each analyte, by using the AI algorithm 60 configured as a learned neural network.

<Hardware configuration of deep learning apparatus>

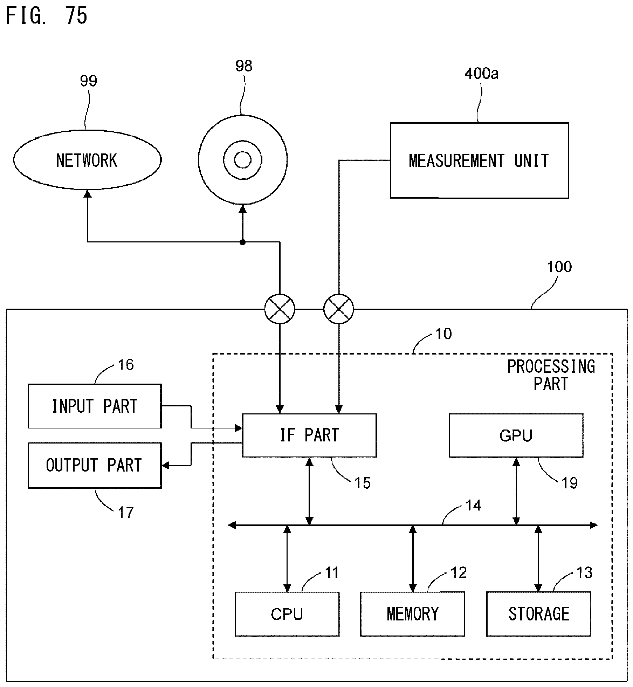

FIG. 75 is a block diagram showing a functional configuration of the electric/control system of the deep learning apparatus 100.

The deep learning apparatus 100 includes a processing part 10, an input part 16, and an output part 17.

The input part 16 and the output part 17 are connected to the processing part 10 via an IF part 15. The input part 16 is an input device such as a keyboard or a mouse, for example. The output part 17 is a display device such as a liquid crystal display, for example.

The processing part 10 includes a CPU 11, a memory 12, a storage 13, a bus 14, the IF part 15, and a GPU 19.

The CPU 11 performs data processing described later. The memory 12 is used as a work area for the data processing. The storage 13 stores a program and processing data described later. The bus 14 transmits data between components. The IF part 15 performs input/output of data to/from an external apparatus. The GPU 19 functions as an accelerator that assists arithmetic processes (e.g., parallel arithmetic processes) performed by the CPU 11. That is, in the description below, the processes performed by the CPU 11 also include processes performed by the CPU 11 using the GPU 19 as an accelerator. The GPU 19 has a function equivalent to that of the parallel-processing processor 3002, 6002 described above. Instead of the GPU 19, a chip suitable for calculation in a neural network may be used. Examples of such a chip can include FPGA, ASIC, and Myriad X (Intel).

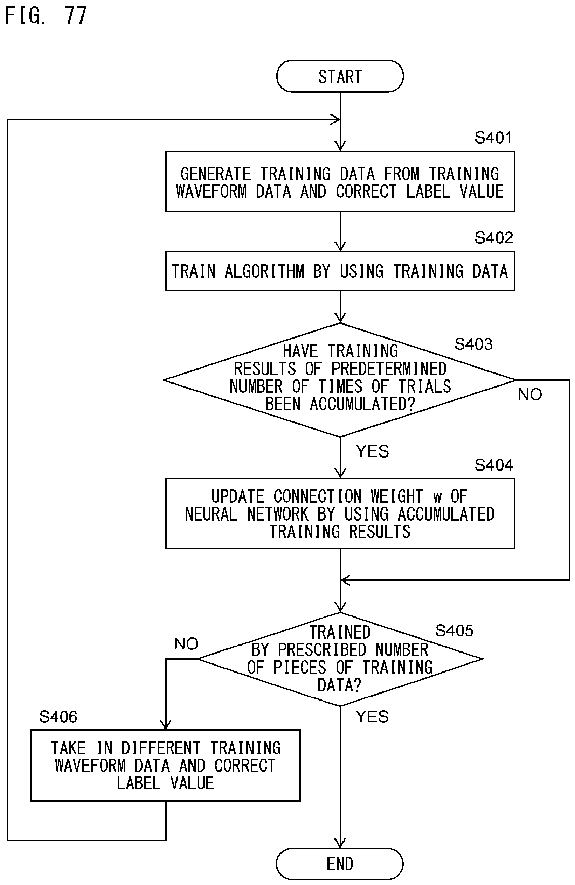

In order to perform the process of each step described later with reference to FIG. 77, the processing part 10 has previously stored, in the storage 13, a program and the AI algorithm 60 configured as a neural network before being trained according to the present embodiment, in an executable form, for example. The executable form is a form generated through conversion of a programming language by a compiler, for example. The processing part 10 uses the program stored in the storage 13, to perform a training process of the AI algorithm 60 before being trained.

In the description below, unless otherwise specified, the processes performed by the processing part 10 mean processes performed by the CPU 11 on the basis of the program and the AI algorithm 60 stored in the storage 13 or the memory 12. The CPU 11 temporarily stores necessary data (intermediate data being processed, etc.) using the memory 12 as a work area, and stores, as appropriate in the storage 13, data to be saved for a long time such as arithmetic results.

<Hardware configuration of analyzer>

The configuration of the specimen analyzer 4000 (see FIG. 74) is similar to that described above, and the specimen analyzer 4000 processes waveform data on the basis of an algorithm provided from the deep learning apparatus 100. The specimen analyzer 4000 may also have the function of the deep learning apparatus 100, to cause the AI algorithm 60 to learn by using training data. In this case, the deep learning apparatus 100 is not necessary.

In order to perform the process of each step described in the waveform data analysis process below, the specimen analyzer 4000 has previously stored, in the storage 3004 or the storage 6004, a program and the AI algorithm 60 configured as a trained neural network according to the present embodiment, in an executable form, for example. The specimen analyzer 4000 performs processes by using the program and the AI algorithm 60 stored in the storage 3004.

The AI algorithm 60 stored in the storage 3004, 6004 may be updated via a communication network. The deep learning apparatus 100 transmits the AI algorithm 60 to the specimen analyzer 4000 via a communication network (e.g., Internet, intranet). The specimen analyzer 4000 updates, by the received AI algorithm 60, the AI algorithm 60 already stored in the storage 3004, 6004.

<Function block and processing procedure>

(Deep learning process)

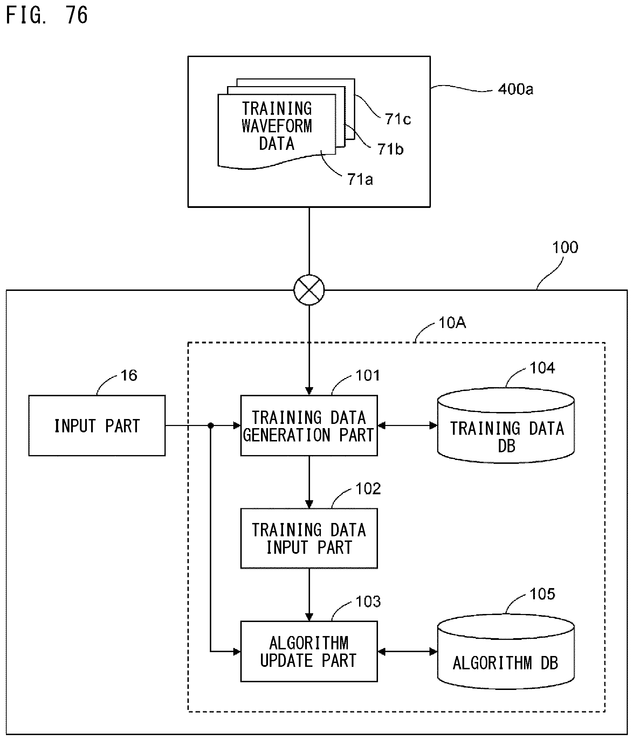

FIG. 76 is a block diagram showing a functional configuration of the electric/control system of the deep learning apparatus 100.

The processing part 10A of the deep learning apparatus 100 includes a training data generation part 101, a training data input part 102, and an algorithm update part 103. A program that causes a computer to execute a deep learning process is installed in the storage 13 or the memory 12 of the processing part 10 shown in FIG. 75, and this program is executed by the CPU 11 and the GPU 19, whereby each function block of the processing part 10A is realized.

A training data database (DB) 104 and an algorithm database (DB) 105 are stored in the storage 13 or the memory 12 of the processing part 10 shown in FIG. 75. The training waveform data 71a, 71b, 71c is obtained in advance by the measurement unit 400a, for example, and is stored in advance in the training data database 104. The AI algorithm 60 before being trained is stored in the algorithm database 105.

FIG. 77 is a flowchart showing a process performed by the deep learning apparatus 100.

The processes of steps S401, S404, and S406 are executed by the training data generation part 101. The process of step S402 is executed by the training data input part 102. The processes of steps S403 and S405 are executed by the algorithm update part 103.

First, the processing part 10A obtains the training waveform data 71a, 71b, 71c. The pieces of the training waveform data 71a, 71b, 71c are pieces of waveform data based on forward scattered light, side scattered light, and fluorescence, respectively. The training waveform data 71a, 71b, 71c may be obtained, for example, from the measurement unit 400a, from the storage medium 98, or via the communication network 99, through operation by an operator. When the training waveform data 71a, 71b, 71c is obtained, information (correct label value 73) regarding which cell type the training waveform data 71a, 71b, 71c indicates is also obtained. The information regarding the cell type may be associated with the training waveform data 71a, 71b, 71c or may be inputted by the operator through the input part 16.

In step S401, the processing part 10A generates training data from the training waveform data 71a, 71b, 71c and the correct label values 73. In step S402, the processing part 10A inputs the training data to the AI algorithm 60, and obtains a trial result. The trial result is accumulated every time each of a plurality of pieces of the training data is inputted to the AI algorithm 60.

In the cell type analysis method according to the present embodiment, a convolutional neural network is used, and a stochastic gradient descent method is used. Therefore, in step S403, the processing part 10A determines whether or not training results of a previously-set predetermined number of times of trials have been accumulated. When the predetermined number of training results have been accumulated (S403: YES), the processing part 10A advances the process to step S404. On the other hand, when the predetermined number of training results have not been accumulated (S403: NO), the processing part 10A skips the process of step S404.

When the predetermined number of training results have been accumulated (S403: YES), the processing part 10A updates, in step S404, a connection weight w of the neural network forming the AI algorithm 60, by using the training results accumulated in step S402. In addition, in step S404, the processing part 10A updates the weighting matrixes $W_q$, $W_k$, and $W_v$ (see FIG. 8) forming the AI algorithm 60, by using the accumulated training results. In the cell type analysis method according to the present embodiment, since the stochastic gradient descent method is used, the connection weight w of the neural network and the weighting matrixes $W_q$, $W_k$, $W_v$ are updated at the stage where the predetermined number of times of training results have been accumulated. Specifically, these update processes are processes of performing calculation by the gradient descent method, represented by Formula 12 and Formula 13 described later.

In step S405, the processing part 10A determines whether or not the AI algorithm 60 has been trained by a prescribed number of pieces of training data. When the AI algorithm 60 has been trained by the prescribed number of pieces of training data (S405: YES), the deep learning process ends. On the other hand, when the AI algorithm 60 has not been trained by the prescribed number of pieces of training data (S405: NO), the processing part 10A takes in different pieces of the training waveform data 71a, 71b, 71c and the correct label values 73 in step S406, and returns the process to step S401.

Through the processes as above, the processing part 10A trains the AI algorithm 60 and obtains the trained AI algorithm 60.

(Structure of neural network)

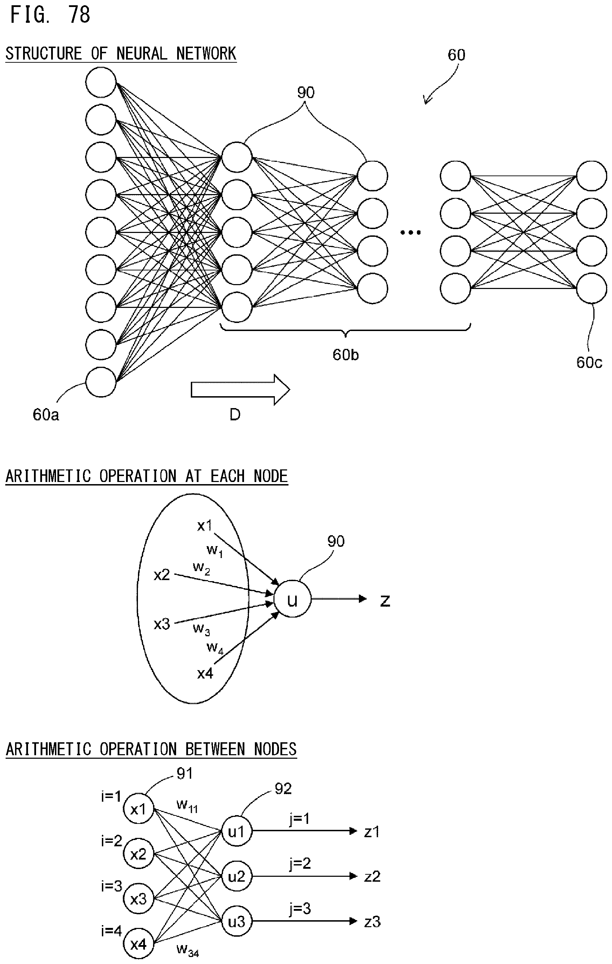

The upper part of FIG. 78 is a schematic diagram showing, as an example, a structure of a neural network forming the AI algorithm 60. As described above, a convolutional neural network is used in the present embodiment. The neural network of the AI algorithm 60 includes an input layer 60a, a middle layer 60b, and an output layer 60c, and the middle layer 60b is between the input layer 60a and the output layer 60c and is composed of a plurality of layers. The number of layers forming the middle layer 60b is, for example, 5 or greater, preferably 50 or greater, and more preferably 100 or greater.

In the examples shown in FIGS. 5 and 11, the AI algorithm 60 is shown to have, as functions, the relevance degree calculation part 61 and the classification determination part 62, but the AI algorithm 60 composed of the relevance degree calculation part 61 and the classification determination part 62 includes, as a whole, the input layer 60a, the middle layer 60b composed of a plurality of layers, and the output layer 60c, as shown in the upper part of FIG. 78.

In the neural network of the AI algorithm 60, a plurality of nodes 90 arranged in a layered manner are connected between the layers. Accordingly, information is propagated only in one direction indicated by an arrow D in the drawing, from the input layer 60a to the output layer 60c.

(Arithmetic operation at each node)

The middle part of FIG. 78 is a schematic diagram showing arithmetic operations performed at each node 90. Each node 90 receives a plurality of inputs, and calculates one output (z). In the case of the example shown in the middle part of FIG. 78, the node 90 receives four inputs. The total input (u) received by the node 90 is represented by Formula 2 below, for example. Here, in the present embodiment, one-dimensional matrix data is used as the analysis data 71, 81. Therefore, when variables of the arithmetic expression correspond to two-dimensional matrix data, a process of converting the variables so as to correspond to one-dimensional matrix data is performed.

$$u = w_1 x_1 + w_2 x_2 + w_3 x_3 + w_4 x_4 + b \qquad \ldots \text{(Formula 2)}$$

Each input is multiplied by a different weight. In Formula 2, b is a value referred to as bias. The output (z) of the node serves as an output of a predetermined function f with respect to the total input (u) represented by Formula 2, and is represented by Formula 3 below. The function f is referred to as an activation function.

$$z = f(u) \qquad \text{... (Formula 3)}$$

The lower part of FIG. 78 is a schematic diagram showing arithmetic operations between nodes. In the neural network, nodes 90 that each output, with respect to the total input (u) of the corresponding node 90 represented by Formula 2, a result (z) represented by Formula 3 are arranged in a layered manner. The outputs of nodes 90 in the previous layer serve as inputs to nodes 90 in the next layer. In the example shown in the lower part of FIG. 78, the outputs of nodes 91 in the left layer in the drawing serve as inputs to nodes 92 in the right layer in the drawing. Each node 92 receives outputs from the nodes 91. The connection between each node 91 and each node 92 is multiplied by a different weight. When the respective outputs from the plurality of nodes 91 are defined as x1 to x4, the inputs to the respective three nodes 92 are represented by Formula 4-1 to Formula 4-3 below.

$$u_1 = w_{11}x_1 + w_{12}x_2 + w_{13}x_3 + w_{14}x_4 + b_1 \qquad \text{... (Formula 4-1)}$$

$$u_2 = w_{21}x_1 + w_{22}x_2 + w_{23}x_3 + w_{24}x_4 + b_2 \qquad \text{... (Formula 4-2)}$$

$$u_3 = w_{31}x_1 + w_{32}x_2 + w_{33}x_3 + w_{34}x_4 + b_3 \qquad \text{... (Formula 4-3)}$$

When these Formula 4-1 to Formula 4-3 are generalized, Formula 4-4 below is obtained. Here, i = 1,..., I, and j=1,..., J. I is the total number of inputs, and J is the total number of outputs.

$$u_j = \sum_{i=1}^{I} w_{ji}x_i + b_j \qquad \text{... (Formula 4-4)}$$

When Formula 4-4 is applied to the activation function, an output represented by Formula 5 below is obtained.

$$z_j = f(u_j) \quad (j = 1, 2, 3) \qquad \text{... (Formula 5)}$$

(Activation function)

In the cell type analysis method according to the embodiment, a rectified linear unit function is used as the activation function. The rectified linear unit function is represented by Formula 6 below.

$$f(u) = \max(u, 0) \qquad \text{... (Formula 6)}$$

Formula 6 is a function obtained by setting u=0 to the part u<0 in the linear function with z=u. In the example shown in the lower part of FIG. 78, the output from the node of j=1 is represented by Formula 6 below.

$$z_1 = \max\big((w_{11}x_1 + w_{12}x_2 + w_{13}x_3 + w_{14}x_4 + b_1), 0\big)$$

(Neural network learning)

If a function expressed by use of a neural network is defined as y(x:w), the function y(x:w) varies when a parameter w of the neural network is varied. Adjusting the function y(x:w) such that the neural network selects a more suitable parameter w with respect to the input x is referred to as neural network training/learning. It is assumed that a plurality of pairs of inputs and outputs of a function expressed by use of a neural network are given. When a desirable output for an input x is defined as d, the pairs of the input/output are given as {(x1,d1), (x2,d2),..., (xn,dn)}. The set of pairs expressed as (x,d) is referred to as training data. Specifically, as shown in FIG. 10, the set of the waveform data 71a, 71b, 71c and the correct label values 73 are the training data.

The neural network learning means adjusting the weight w such that, with respect to any input/output pair (xn,dn), the output y(xn:w) of the neural network when given the input xn becomes close to the output dn as much as possible, as shown in the formula below.

$$y(x_n : w) \approx d_n$$

An error function is a measure for measuring closeness between the training data and the function expressed by use of the neural network. The error function is also referred to as a loss function. An error function E(w) used in the cell type analysis method according to the embodiment is represented by Formula 7 below. Formula 7 is referred to as cross entropy.

$$E(w) = -\sum_{n=1}^{N} \sum_{k=1}^{K} d_{nk} \log y_k(x_n : w)$$

$$\ldots \text{(Formula 7)}$$

A method for calculating the cross entropy of Formula 7 will be described. In the output layer 60c (the upper part of FIG. 78) of the neural network used in the cell type analysis method according to the embodiment, i.e., in the last layer of the neural network, an activation function for classifying the input x into a finite number of classes in accordance with the contents, is used. The activation function is referred to as a softmax function, and is represented by Formula 8 below. It is assumed that, in the output layer 60c, nodes are arranged by the same number as the number of classes k. It is assumed that the total input u of each node k (k=1,..., K) of an output layer L is given as uk$^{(L)}$ from the outputs of the previous layer L-1. Accordingly, the output of the k-th node in the output layer is represented by Formula 8 below.

$$y_k \equiv z_k^{(L)} = \frac{\exp\left(u_k^{(L)}\right)}{\sum_{j=1}^{K} \exp\left(u_j^{(L)}\right)}$$

$$\ldots \text{(Formula 8)}$$

Formula 8 is the softmax function. The sum of output y1,..., yK determined by Formula 8 is always 1.
When each class is expressed as C1,..., CK, output yK of node k in the output layer L (i.e., uk$^{(L)}$) represents the probability at which the given input x belongs to class CK. The input x is classified to a class at which the probability represented by Formula 9 below is highest.

$$p(C_k|x) = y_k = z_k^{(L)}$$

$$\ldots \text{(Formula 9)}$$

In the neural network learning, a function expressed by the neural network is considered as a model of the posterior probability of each class. The likelihood of the weight w with respect to the training data is evaluated under such a probability model, and a weight w that maximizes the likelihood is selected.
It is assumed that the target output by the softmax function of Formula 8 is 1 only when the output is a correct class, and otherwise, the target output is 0. When the target output is expressed in a vector form dn=[dn1,..., dnK], if, for example, the correct class of input xn is C3, only target output dn3 becomes 1, and the other target outputs become 0. When coding is performed in this manner, the posterior distribution is represented by Formula 10 below.

$$p(d|x) = \prod_{k=1}^{K} p(C_k|x)^{d_k}$$

... (Formula 10)

Likelihood L(w) of the weight w with respect to the training data {(xn,dn)}(n=1,..., N) is represented by Formula 11 below. When the logarithm of the likelihood L(w) is taken and the sign is inverted, the error function of Formula 7 is derived.

$$L(w) = \prod_{n=1}^{N} p(d_n|x_n:w)$$
$$= \prod_{n=1}^{N} \prod_{k=1}^{K} p(C_k|x_n)^{d_{nk}}$$
$$= \prod_{n=1}^{N} \prod_{k=1}^{K} \left(y_k(x:w)\right)^{d_{nk}}$$

... (Formula 11)

Learning means minimizing the error function E(w) calculated on the basis of the training data, with respect to the parameter w of the neural network. In the cell type analysis method according to the embodiment, the error function E(w) is represented by Formula 7.

Minimizing the error function E(w) with respect to the parameter w has the same meaning as finding a local minimum point of the error function E(w). The parameter w is a weight of connection between nodes. The local minimum point of the weight w is obtained by iterative calculation of repeatedly updating the parameter w from an arbitrary initial value used as a starting point. An example of such calculation is the gradient descent method.

In the gradient descent method, a vector represented by Formula 12 below is used.

$$\nabla E = \frac{\partial E}{\partial w} = \left[\frac{\partial E}{\partial w_1}, \dots, \frac{\partial E}{\partial w_M}\right]^T$$

... (Formula 12)

In the gradient descent method, a process of moving the value of the current parameter w in the negative gradient direction (i.e., -VE) is repeated many times. When the current weight is w(t) and the weight after the moving is w(t+1), the arithmetic operation according to the gradient descent method is represented by Formula 13 below. The value t means the number of times the parameter w is moved.

$$w^{(t+1)} = w^{(t)} - \epsilon \nabla E$$

... (Formula 13)

The symbol used in Formula 13 and shown in Formula 14 below is a constant that determines the magnitude of the update amount of the parameter w, and is referred to as a learning coefficient.

$$\epsilon$$

... (Formula 14)

As a result of repetition of the arithmetic operation represented by Formula 13, an error function E(w(t)) decreases in association with increase of the value t, and the parameter w reaches a local minimum point.

It should be noted that the arithmetic operation according to Formula 13 may be performed on all of the training data (n=1,..., N), or may be performed on only a part of the training data. The gradient descent method performed on only a part of the training data is referred to as a stochastic gradient descent method. In the cell type analysis method according to the embodiment, the stochastic gradient descent method is used.

[Effects of embodiments]

In the following description of effects, when the specimen analyzer 4000 is a blood cell analyzer or a urine analyzer, first and second data are digital data (waveform data) corresponding to the intensity of an optical signal based on light generated from each analyte (cell or particle). The optical signal in this case is an analog signal outputted from the light receiving element on the basis of each of forward scattered light, side scattered light, and fluorescence. The optical signal is a signal that has a region corresponding to each of analytes in a specimen and in which the presence of the analyte in the specimen is reflected. The waveform data (first and second data) is generated so as to correspond to the region of the optical signal. In other words, the waveform data corresponds to an optical signal obtained while an analyte is passing through the position of light applied by the light source 4111. A representative value corresponding to a feature of an analyte is a value such as the peak value, the area, or the width obtained from the waveform data corresponding to the analyte, for example. A first analysis and a second analysis denote operations of determining the type of the analyte (cell or particle).

The measurement unit 400 obtains the waveform data 81a, 81b, 81c (first data) corresponding to an optical signal obtained from an analyte. The matrix S (set data) composed of a plurality of pieces of the first data is inputted to the AI algorithm 60 (artificial intelligence algorithm) capable of calculating the relevance degree between the pieces of the first data. The type of the analyte is determined by use of the relevance degree above.

With this configuration, since the set data is processed by the AI algorithm 60 to obtain the relevance degree between the pieces of the first data, it is possible to determine which of a plurality of types certain first data is more relevant to. Therefore, it is possible to reduce the number of pieces of the first data for which it is difficult to determine which of a plurality of types the analyte belongs to, and thus, the classification accuracy of the type of the analyte by the AI algorithm 60 can be improved.

The determination of the type of the analyte may be performed by the AI algorithm 60 as shown in the above embodiments, or may be performed by another AI algorithm other than the AI algorithm 60. When the determination of the type of the analyte is performed by the other AI algorithm, the AI algorithm 60 does not include the classification determination part 62 and calculates the relevance degree by the relevance degree calculation part 61, for example. Meanwhile, the other AI algorithm includes the classification determination part 62, and on the basis of the first data and the relevance degree, or on the basis of the first data corrected with the relevance degree, determines the type of the analyte by means of the classification determination part 62.

The AI algorithm 60 need not necessarily include the classification determination part 62. When the AI algorithm 60 includes only the relevance degree calculation part 61, for example, a representative value is obtained from the waveform data of each analyte included in the matrix S corrected by the relevance degree calculation part 61, and on the basis of the obtained representative value, determination of the type of each analyte is performed through the calculation processing analysis.

The type of the analyte is determined on the basis of the waveform data 81a, 81b, 81c (first data) and the relevance degree.

With this configuration, for example, the first data corresponding to each of analytes is corrected on the basis of the relevance degree, whereby the first data capable of improving the classification accuracy can be formed.

The first data forming the matrix S (set data) is corrected by the AI algorithm 60 on the basis of the relevance degree.

With this configuration, in the AI algorithm 60, both of calculation of the relevance degree and correction based on the relevance degree are performed. Therefore, for example, it is not necessary to perform these processes across a plurality of computers.

The type of the analyte is determined on the basis of the first data corrected by the AI algorithm 60.

With this configuration, the type of the analyte can be determined smoothly and with high accuracy by use of the corrected first data.

The AI algorithm 60 calculates the relevance degree between pieces of waveform data respectively corresponding to a plurality of analytes. On the basis of the calculated relevance degree, the AI algorithm 60 corrects the waveform data inputted to the AI algorithm 60 such that the features of pieces of the waveform data having a high relevance degree become closer to each other. For example, when waveform data that is near the boundary between a group A and a group B has been determined to have higher relevance to the group B, the waveform data is corrected so as to be close to the group B. In addition, pieces of waveform data are corrected such that a plurality of pieces of waveform data belonging to the group A become close to each other, and a plurality of pieces of waveform data belonging to the group B become close to each other.

With this configuration, through the correction based on the relevance degree, it is possible to reduce the number of pieces of waveform data for which classification of the type is difficult due to ambiguity of the group to which the analyte belongs. Since the number of pieces of waveform data for which classification of the type is difficult can be reduced, in the AI algorithm 60 capable of determining the relevance degree, classification accuracy of the analyte is improved when compared with an algorithm that does not determine the relevance degree.

The measurement unit 400 obtains waveform data (second data) corresponding to the optical signal obtained from the analyte. The analysis unit 300, 600 executes the AI analysis (first analysis) on the matrix S (set data) being a collection of a plurality of pieces of the waveform data 81a, 81b, 81c (first data), and executes the second analysis, on the waveform data (second data), of processing a representative value corresponding to a feature of the analyte.

With this configuration, when compared with a case where the AI analysis is performed on all of the waveform data, the AI analysis is performed only on the first data. Accordingly, the load on the analysis unit 300, 600 can be reduced.

The second analysis in this case need not necessarily be the calculation processing analysis. For example, a classification result may be obtained by processing the representative value by the AI analysis.

The first analysis is the AI analysis by the artificial intelligence algorithm, and the second analysis is the calculation processing analysis (non-AI analysis) of processing the representative value corresponding to the feature of the analyte.

With this configuration, since the analysis process of the waveform data is apportioned between the AI analysis and the calculation processing analysis, when compared with a case where data corresponding to the optical signal is all analyzed by using only an artificial intelligence algorithm, the load on the analysis unit 300, 600 can be reduced.

In the calculation processing analysis (second analysis), the analysis unit 300, 600 specifies the representative value on the basis of the waveform data (second data), and processes the specified representative value.

With this configuration, the representative value can be smoothly specified on the basis of the second data, and thus, in the second analysis, processing can be smoothly performed by using the specified representative value.

As shown in FIG. 13, in the calculation processing analysis (second analysis), the representative value such as the peak value, the area, or the width is specified on the basis of the magnitude of the waveform data (second data).

With this configuration, the representative value can be smoothly specified.

In the calculation processing analysis (second analysis), the representative value to serve as a target of the calculation processing analysis is specified on the basis of the waveform data (second data).

With this configuration, the optical signal includes a region corresponding to each of analytes. Therefore, on the basis of the waveform data corresponding to each region in the optical signal, a representative value such as the peak value, the area, or the width corresponding to each analyte can be smoothly specified.

When the specimen analyzer 4000 is a blood cell analyzer or a urine analyzer, the optical signal has a region corresponding to each of analytes in the specimen. In the AI analysis, the analysis unit 300, 600 inputs, to the AI algorithm 60, the set data being a collection of a plurality of pieces of the waveform data (first data) corresponding to the respective regions of the optical signal. Since the optical signal includes regions corresponding to the respective analytes, when the set data being a collection of a plurality of pieces of the waveform data corresponding to the respective regions of the optical signal is inputted to the AI algorithm 60, the AI analysis can be smoothly executed.

As described above, when the optical signal has a region corresponding to each of analytes in the specimen, the measurement unit 400 obtains waveform data (first and second data) on the basis of a signal greater than a predetermined threshold corresponding to the intensity of the optical signal, for example. With this configuration, waveform data corresponding to each of the analytes can be accurately obtained.

As shown in FIG. 15, on the basis of a rule for specifying data to serve as a target of each of the AI analysis (first analysis) and the calculation processing analysis (second analysis), the analysis unit 300 specifies waveform data (first data) to serve as a target of the AI analysis and waveform data (second data) to serve as a target of the calculation processing analysis.

With this configuration, it is possible to smoothly determine which of the first analysis and the second analysis is to be performed on the data corresponding to the optical signal.

As shown in FIG. 16, in accordance with a measurement item included in a measurement order for a specimen, the analysis unit 300 specifies waveform data (first data) to serve as a target of the AI analysis and waveform data (second data) to serve as a target of the calculation processing analysis.

With this configuration, for example, analysis of a measurement item for which highly accurate analysis is difficult to be executed by the calculation processing analysis can be executed by the AI analysis, and analysis of a normal measurement item can be executed by the calculation processing analysis. Accordingly, highly accurate analysis and reduction of the load on the analysis unit 300 can be realized.

As shown in FIG. 22, in accordance with the type of a measurement order for the specimen, the analysis unit 300 specifies waveform data (first data) to serve as a target of the AI analysis, and waveform data (second data) to serve as a target of the calculation processing analysis.

With this configuration, for example, which of the AI analysis and the calculation processing analysis is to be executed can be determined in accordance with the type of the measurement order such as normal measurement (Normal), remeasurement (Rerun) in which the same measurement order is executed again, or measurement (Reflex) in which a measurement order is reset, i.e., in accordance with the purpose or the like of the measurement based on the measurement order.

As shown in FIG. 20, in accordance with an analysis mode of the specimen analyzer 4000, the analysis unit 300 specifies waveform data (first data) to serve as a target of the AI analysis and waveform data (second data) to serve as a target of the calculation processing analysis.

With this configuration, for example, when either one of the AI analysis mode and the calculation processing analysis mode is set in advance to the specimen analyzer 4000, labor of setting an analysis mode for each specimen or measurement item can be omitted.

As shown in FIGS. 26 and 28, the analysis unit 300 determines whether or not the AI analysis (first analysis) needs to be executed, in accordance with an analysis result of the calculation processing analysis (second analysis).

With this configuration, for example, when further detailed analysis is necessary according to the analysis result of the calculation processing analysis, the AI analysis is executed, whereby highly accurate analysis can be performed.

As shown in FIGS. 26 and 28, the analysis unit 300 determines whether or not the AI analysis (first analysis) needs to be executed, in accordance with whether or not a predetermined analyte has been detected through the calculation processing analysis (second analysis).

With this configuration, for example, when a blast, an abnormal lymphocyte, an atypical lymphocyte, or the like has been detected through the calculation processing analysis, a further detailed test can be performed through the AI analysis.

As shown in FIG. 31, the analysis unit 300 specifies, as a target of the AI analysis (first analysis), waveform data (first data) that corresponds to an analyte classified as a predetermined type through the calculation processing analysis (second analysis).

In an analysis result of the calculation processing analysis, for example, as shown in FIG. 30, distribution regions of cells classified as, for example, monocytes and lymphocytes are close to each other. Therefore, when the AI analysis is executed on the cells classified as, for example, monocytes and lymphocytes through the calculation processing analysis, highly accurate classification can be performed.

The data amount of the representative value processed in the calculation processing analysis (second analysis) is smaller than the data amount of the set data being a collection of a plurality of pieces of the waveform data (first data) inputted to the AI algorithm 60 in the AI analysis (first analysis).

With this configuration, in the calculation processing analysis, the data amount of the processing target is smaller than that of the AI analysis. Therefore, the load on the computer that performs analysis is smaller than that in the AI analysis. Accordingly, the TAT (Turn Around Time) of the analysis of the measurement result can be shortened.

Various modifications can be made as appropriate to the embodiments of the present disclosure, without departing from the scope of the technological idea defined by the claims.


[Remarks]

The present disclosure includes following items 1-53.


Item 1: An analysis method for analyzing an analyte in a specimen, the analysis method comprising:

obtaining first data corresponding to an optical signal obtained from the analyte;
inputting set data composed of a plurality of pieces of the first data, to an artificial intelligence algorithm capable of calculating a relevance degree between the pieces of the first data; and
determining a type of the analyte by using the relevance degree.


Item 2: The analysis method of item 1, wherein
the type of the analyte is determined on the basis of the first data and the relevance degree.
Item 3: The analysis method of item 1, wherein
the first data forming the set data is corrected by the artificial intelligence algorithm on the basis of the relevance degree.
Item 4: The analysis method of item 1, wherein
the type of the analyte is determined on the basis of the first data corrected by the artificial intelligence algorithm.
Item 5: The analysis method of item 1, wherein
the first data is corrected by the artificial intelligence algorithm such that a difference between the first data that belongs to a first group formed on the basis of the relevance degree and the first data that belongs to a second group formed on the basis of the relevance degree becomes large.

Item 6: The analysis method of item 1, wherein
the first data is corrected by the artificial intelligence algorithm such that a plurality of pieces of the first data that belong to one group formed on the basis of the relevance degree become close to each other.

Item 7: The analysis method of item 1, wherein
the relevance degree is calculated through a matrix operation.

Item 8: The analysis method of item 1, wherein
the artificial intelligence algorithm is a deep learning algorithm.

Item 9: The analysis method of item 1, comprising:

obtaining second data corresponding to the optical signal obtained from the analyte;
executing, on the first data, a first analysis by the artificial intelligence algorithm; and
executing, on the second data, a second analysis of processing a representative value corresponding to a feature of the analyte.

Item 10: The analysis method of item 9, wherein

the first analysis is an AI analysis by the artificial intelligence algorithm, and
the second analysis is a non-AI analysis of processing the representative value corresponding to the feature of the analyte.

Item 11: The analysis method of item 10, wherein
in the second analysis, the representative value is specified on the basis of the second data, and the specified representative value is processed.

Item 12: The analysis method of item 10, wherein
in the second analysis, the representative value is specified on the basis of a magnitude of the second data.

Item 13: The analysis method of item 10, wherein
in the second analysis, a peak value of the second data is specified as the representative value.

Item 14: The analysis method of item 10, wherein
the first data and the second data are specified on the basis of a rule for specifying data to serve as a target of each of the first analysis and the second analysis.

Item 15: The analysis method of item 10, wherein
the first data and the second data are specified in accordance with a measurement item included in a measurement order for the specimen.

Item 16: The analysis method of item 10, wherein
the first data and the second data are specified in accordance with a type of a measurement order for the specimen.

Item 17: The analysis method of item 10, wherein
the first data and the second data are specified in accordance with an analysis mode of an apparatus that measures the specimen.

Item 18: The analysis method of item 10, wherein
whether or not the first analysis needs to be executed is determined in accordance with an analysis result of the second analysis.

Item 19: The analysis method of item 10, wherein
whether or not the first analysis needs to be executed is determined in accordance with whether or not a predetermined analyte has been detected through the second analysis.

Item 20: The analysis method of item 10, wherein
the first data that corresponds to an analyte classified as a predetermined type through the second analysis is specified as a target of the first analysis.

Item 21: The analysis method of item 10, wherein
a data amount of the representative value processed in the second analysis is smaller than a data amount of the set data inputted to the artificial intelligence algorithm in the first analysis.

Item 22: The analysis method of item 1, wherein
arithmetic processes by the artificial intelligence algorithm are executed as parallel processing by a parallel-processing processor.

Item 23: The analysis method of item 1, wherein
a matrix operation by the artificial intelligence algorithm is executed as parallel processing by a parallel-processing processor.

Item 24:The analysis method of item 1, wherein
a matrix operation for calculating the relevance degree is executed as parallel processing by a parallel-processing processor.

Item 25:The analysis method of item 10, wherein
a process regarding the first analysis is executed by a parallel-processing processor, and a process regarding the second analysis is executed by a host processor of the parallel-processing processor.

Item 26:The analysis method of item 22, wherein
the parallel-processing processor is a GPU.

Item 27:A specimen analyzer configured to analyze an analyte in a specimen, the specimen analyzer comprising:

a measurement unit configured to obtain an optical signal from the analyte; and
an analysis unit configured to analyze set data composed of a plurality of pieces of first data corresponding to the optical signal, wherein
the analysis unit analyzes the set data by using an artificial intelligence algorithm configured to determine a type of the analyte on the basis of a relevance degree between the pieces of the first data.

Item 28:The specimen analyzer of item 27, wherein
the artificial intelligence algorithm determines the type of the analyte on the basis of the first data and the relevance degree.

Item 29:The specimen analyzer of item 27, wherein
the artificial intelligence algorithm corrects the first data forming the set data, on the basis of the relevance degree.

Item 30:The specimen analyzer of item 27, wherein
the artificial intelligence algorithm determines the type of the analyte on the basis of the first data corrected by the artificial intelligence algorithm.

Item 31: The specimen analyzer of item 27, wherein
the artificial intelligence algorithm corrects the first data such that a difference between the first data that belongs to a first group formed on the basis of the relevance degree and the first data that belongs to a second group formed on the basis of the relevance degree becomes large.

Item 32:The specimen analyzer of item 27, wherein
the artificial intelligence algorithm corrects the first data such that a plurality of pieces of the first data that belong to one group formed on the basis of the relevance degree become close to each other.

Item 33:The specimen analyzer of item 27, wherein
the artificial intelligence algorithm calculates the relevance degree through a matrix operation.

Item 34:The specimen analyzer of item 27, wherein
the artificial intelligence algorithm is a deep learning algorithm.

Item 35:The specimen analyzer of item 27, wherein

the measurement unit obtains second data corresponding to the optical signal obtained from the analyte, and
the analysis unit executes, on the first data, a first analysis by the artificial intelligence algorithm and executes, on the second data, a second analysis of processing a representative value corresponding to a feature of the analyte.

Item 36:The specimen analyzer of item 35, wherein

the first analysis is an AI analysis by the artificial intelligence algorithm, and
the second analysis is a non-AI analysis of processing the representative value corresponding to the feature of the analyte.

Item 37:The specimen analyzer of item 36, wherein
in the second analysis, the analysis unit specifies the representative value on the basis of the second data, and processes the specified representative value.

Item 38:The specimen analyzer of item 36, wherein
in the second analysis, the analysis unit specifies the representative value on the basis of a magnitude of the second data.

Item 39:The specimen analyzer of item 36, wherein
in the second analysis, the analysis unit specifies a peak value of the second data as the representative value.

Item 40:The specimen analyzer of item 36, wherein
the analysis unit specifies the first data and the second data on the basis of a rule for specifying data to serve as a target of each of the first analysis and the second analysis.

Item 41:The specimen analyzer of item 36, wherein
the analysis unit specifies the first data and the second data in accordance with a measurement item included in a measurement order for the specimen.

Item 42:The specimen analyzer of item 36, wherein
the analysis unit specifies the first data and the second data in accordance with a type of a measurement order for the specimen.

Item 43:The specimen analyzer of item 36, wherein
the analysis unit specifies the first data and the second data in accordance with an analysis mode of an apparatus that measures the specimen.

Item 44:The specimen analyzer of item 36, wherein
the analysis unit determines whether or not the first analysis needs to be executed, in accordance with an analysis result of the second analysis.

Item 45:The specimen analyzer of item 36, wherein
the analysis unit determines whether or not the first analysis needs to be executed, in accordance with whether or not a predetermined analyte has been detected through the second analysis.

Item 46:The specimen analyzer of item 36, wherein
the analysis unit specifies, as a target of the first analysis, the first data that corresponds to an analyte classified as a predetermined type through the second analysis.

Item 47:The specimen analyzer of item 36, wherein
a data amount of the representative value processed in the second analysis is smaller than a data amount of the set data inputted to the artificial intelligence algorithm in the first analysis.

Item 48:The specimen analyzer of item 27, wherein

the analysis unit further includes a parallel-processing processor, and
the parallel-processing processor executes, as parallel processing, arithmetic processes by the artificial intelligence algorithm.

Item 49:The specimen analyzer of item 27, wherein

the analysis unit further includes a parallel-processing processor, and
the parallel-processing processor executes, as parallel processing, a matrix operation by the artificial intelligence algorithm.

Item 50:The specimen analyzer of item 27, wherein

the analysis unit further includes a parallel-processing processor, and
the parallel-processing processor executes, as parallel processing, a matrix operation for calculating the relevance degree.

Item 51:The specimen analyzer of item 36, wherein

the analysis unit further includes a parallel-processing processor,
the parallel-processing processor executes a process regarding the first analysis, and
a host processor of the parallel-processing processor executes a process regarding the second analysis.

Item 52:The specimen analyzer of item 48, wherein
the parallel-processing processor is a GPU.

Item 53:A program for causing a computer to execute a process of analyzing an analyte in a specimen,

the program comprising a process of analyzing set data composed of a plurality of pieces of data corresponding to an optical signal obtained from the analyte, wherein
the process

calculates a relevance degree between the pieces of the data, and
analyzes the set data by using an artificial intelligence algorithm configured to determine a type of the analyte on the basis of the relevance degree.

**Claims**

1.  An analysis method for analyzing an analyte in a specimen, the analysis method comprising:

    obtaining first data corresponding to an optical signal obtained from the analyte;
    inputting set data composed of a plurality of pieces of the first data, to an artificial intelligence algorithm capable of calculating a relevance degree between the pieces of the first data; and
    determining a type of the analyte by using the relevance degree.

2.  The analysis method of claim 1, wherein
    the type of the analyte is determined on the basis of the first data and the relevance degree.

3.  The analysis method of claim 1, wherein
    the first data forming the set data is corrected by the artificial intelligence algorithm on the basis of the relevance degree.

4.  The analysis method of claim 1, wherein
    the type of the analyte is determined on the basis of the first data corrected by the artificial intelligence algorithm.

5.  The analysis method of claim 1, wherein
    the first data is corrected by the artificial intelligence algorithm such that a difference between the first data that belongs to a first group formed on the basis of the relevance degree and the first data that belongs to a second group formed on the basis of the relevance degree becomes large.

6.  The analysis method of claim 1, wherein
    the first data is corrected by the artificial intelligence algorithm such that a plurality of pieces of the first data that belong to one group formed on the basis of the relevance degree become close to each other.

7.  The analysis method of claim 1, wherein
    the relevance degree is calculated through a matrix operation.

8.  The analysis method of claim 1, wherein
    the artificial intelligence algorithm is a deep learning algorithm.

9.  The analysis method of claim 1, comprising:

    obtaining second data corresponding to the optical signal obtained from the analyte;
    executing, on the first data, a first analysis by the artificial intelligence algorithm; and
    executing, on the second data, a second analysis of processing a representative value corresponding to a feature of the analyte.

10. The analysis method of claim 9, wherein

    the first analysis is an AI analysis by the artificial intelligence algorithm, and
    the second analysis is a non-AI analysis of processing the representative value corresponding to the feature of the analyte.

11. The analysis method of claim 10, wherein
    in the second analysis, the representative value is specified on the basis of the second data, and the specified representative value is processed.

12. The analysis method of claim 10, wherein
    in the second analysis, the representative value is specified on the basis of a magnitude of the second data.

13. The analysis method of claim 10, wherein
    in the second analysis, a peak value of the second data is specified as the representative value.

14. The analysis method of claim 10, wherein

the first data and the second data are specified on the basis of a rule for specifying data to serve as a target of each of the first analysis and the second analysis.

15. The analysis method of claim 10, wherein
the first data and the second data are specified in accordance with a measurement item included in a measurement order for the specimen.

16. The analysis method of claim 10, wherein
the first data and the second data are specified in accordance with a type of a measurement order for the specimen.

17. The analysis method of claim 10, wherein
the first data and the second data are specified in accordance with an analysis mode of an apparatus that measures the specimen.

18. The analysis method of claim 10, wherein
whether or not the first analysis needs to be executed is determined in accordance with an analysis result of the second analysis.

19. The analysis method of claim 10, wherein
whether or not the first analysis needs to be executed is determined in accordance with whether or not a predetermined analyte has been detected through the second analysis.

20. The analysis method of claim 10, wherein
the first data that corresponds to an analyte classified as a predetermined type through the second analysis is specified as a target of the first analysis.

21. The analysis method of claim 10, wherein
a data amount of the representative value processed in the second analysis is smaller than a data amount of the set data inputted to the artificial intelligence algorithm in the first analysis.

22. The analysis method of claim 1, wherein
arithmetic processes by the artificial intelligence algorithm are executed as parallel processing by a parallel-processing processor.

23. The analysis method of claim 1, wherein
a matrix operation by the artificial intelligence algorithm is executed as parallel processing by a parallel-processing processor.

24. The analysis method of claim 1, wherein
a matrix operation for calculating the relevance degree is executed as parallel processing by a parallel-processing processor.

25. The analysis method of claim 10, wherein
a process regarding the first analysis is executed by a parallel-processing processor, and a process regarding the second analysis is executed by a host processor of the parallel-processing processor.

26. The analysis method of claim 22, wherein
the parallel-processing processor is a GPU.

27. A specimen analyzer configured to analyze an analyte in a specimen, the specimen analyzer comprising:

a measurement unit configured to obtain an optical signal from the analyte; and
an analysis unit configured to analyze set data composed of a plurality of pieces of first data corresponding to the optical signal, wherein
the analysis unit analyzes the set data by using an artificial intelligence algorithm configured to determine a type of the analyte on the basis of a relevance degree between the pieces of the first data.

28. The specimen analyzer of claim 27, wherein

the artificial intelligence algorithm determines the type of the analyte on the basis of the first data and the relevance degree.

29. The specimen analyzer of claim 27, wherein
the artificial intelligence algorithm corrects the first data forming the set data, on the basis of the relevance degree.

30. The specimen analyzer of claim 27, wherein
the artificial intelligence algorithm determines the type of the analyte on the basis of the first data corrected by the artificial intelligence algorithm.

31. The specimen analyzer of claim 27, wherein
the artificial intelligence algorithm corrects the first data such that a difference between the first data that belongs to a first group formed on the basis of the relevance degree and the first data that belongs to a second group formed on the basis of the relevance degree becomes large.

32. The specimen analyzer of claim 27, wherein
the artificial intelligence algorithm corrects the first data such that a plurality of pieces of the first data that belong to one group formed on the basis of the relevance degree become close to each other.

33. The specimen analyzer of claim 27, wherein
the artificial intelligence algorithm calculates the relevance degree through a matrix operation.

34. A program for causing a computer to execute a process of analyzing an analyte in a specimen,

the program comprising a process of analyzing set data composed of a plurality of pieces of data corresponding to an optical signal obtained from the analyte, wherein
the process

calculates a relevance degree between the pieces of the data, and
analyzes the set data by using an artificial intelligence algorithm configured to determine a type of the analyte on the basis of the relevance degree.

# FIG. 1

## CONFIGURATION EXAMPLE OF EMBODIMENT 1

4000

400    300

## MODIFICATION OF CONFIGURATION OF EMBODIMENT 1

400

300    4000

FIG. 2

FIG. 3

ANALOG SIGNAL
80a

4116

ANALOG SIGNAL
80b

4121

ANALOG SIGNAL
80c

4122

461

A/D
CONVERTER

APPLI-
CATION
LIGHT

4113

POINT WHERE SIGNAL LEVEL IS NOT LESS
THAN PREDETERMINED THRESHOLD

ANALOG SIGNAL

...

START    END
POINT   POINT
SAMPLING

DIGITAL SIGNAL
(SAMPLING RATE: EXAMPLE OF 1024 POINTS AT 10 NANOSECOND INTERVAL)

INDEX          INDEX          INDEX                    INDEX

| 1 | 10 | 15 | ·· | 12 | 7 | 2 | 9 | 13 | ·· | 10 | 9 | 3 | 8 | ·· | 12 | 10 | | ·· | N | 6 | ·· | 12 | 9 |

t=0ns 10ns    10240ns 0ns 10ns    10240ns 0ns          10240ns

WAVEFORM       WAVEFORM       WAVEFORM                 WAVEFORM
DATA           DATA           DATA                     DATA

# FIG. 4

# FIG. 5

81

t=0ns        10240ns

| 10 | 10 | ··· | 200 | 100 | — 81a |

| 50 | 200 | ··· | 50 | 10 | — 81b |

| 100 | 200 | ··· | 50 | 10 | — 81c |

81

t=0ns        10240ns

| 10 | 10 | ··· | 200 | 100 | — 81a |

| 50 | 200 | ··· | 50 | 10 | — 81b |

| 100 | 200 | ··· | 50 | 10 | — 81c |

·········

MATRIX S

60

CLASSIFICATION DETERMINATION PART

62

62c

RELEVANCE DEGREE CALCULATION PART

61

62a     62b

CLASSIFICATION RESULT

| INDEX: 1 | 3 |
| INDEX: 2 | 2 |
| ... | ... |
| INDEX: N | 3 |

83

CLASSIFICATION INFORMATION

INDEX: 1

| LYMPHOCYTE | 10% |
| MONOCYTE | 80% |
| ... | ... |

82

INDEX: 2

| LYMPHOCYTE | 85% |
| MONOCYTE | 10% |
| ... | ... |

82

·····

FIG. 6

| CELL TYPE | LABEL VALUE |
|---|---|
| NOT APPLICABLE (NONE) | 0 |
| NEUTROPHIL (NEUT) | 1 |
| LYMPHOCYTE (LYMPH) | 2 |
| MONOCYTE (MONO) | 3 |
| EOSINOPHIL (EO) | 4 |
| BASOPHIL (BASO) | 5 |
| IMMATURE GRANULOCYTE (IG) | 6 |
| ABNORMAL CELL (BLOOD-DERIVED CELL OTHER THAN FIVE CLASSIFICATIONS) | 7 |

FIG. 7

SET DATA (MATRIX S) COMPOSED OF PLURALITY OF PIECES OF ANALYSIS DATA

| | WAVEFORM DATA CORRESPONDING TO FORWARD SCATTERED LIGHT SIGNAL | | | WAVEFORM DATA CORRESPONDING TO SIDE SCATTERED LIGHT SIGNAL | | | WAVEFORM DATA CORRESPONDING TO FLUORESCENCE SIGNAL | | |
|---|---|---|---|---|---|---|---|---|---|
| INDEX #1 → | 10 | ... | 100 | 50 | ... | 100 | 100 | ... | 10 |
| | ... | ... | ... | ... | ... | ... | ... | ... | ... |
| INDEX #N → | 15 | ... | 70 | 30 | ... | 60 | 20 | ... | 150 |

EP 4 439 571 A1

FIG. 8

（1）

$$Q = TW_q \qquad K = SW_k \qquad V = SW_v$$

$W_q$, $W_k$, $W_v$ ARE WEIGHTING MATRIX GENERATED BY LEARNING.

S=T.

（2）

$$Softmax(QK^T) = Z$$

$K^T$ IS TRANSPOSED MATRIX OF K.

Z IS WEIGHTING MATRIX CALCULATED BY SOFTMAX FUNCTION.

（3）

$$ZV$$

ZV IS MATRIX REFLECTING RELEVANCE DEGREE BETWEEN CELLS AND IS CALCULATED ON THE BASIS OF Z AND V.

FIG. 9

```
                    ┌─────────────┐
                    │    START    │
                    └──────┬──────┘
                           │
                           ▼
      ┌────────────────────────────────────────────┐  S1
      │  OBTAIN WAVEFORM DATA CORRESPONDING TO      │
      │   SIGNAL DETECTED BY DETECTION PART         │
      └────────────────────┬───────────────────────┘
                           │
                           ▼
      ┌────────────────────────────────────────────┐  S2
      │  ANALYZE, BY AI ALGORITHM, SET OF PIECES    │
      │     OF WAVEFORM DATA RESPECTIVELY           │
      │  CORRESPONDING TO PLURALITY OF CELLS        │
      └────────────────────┬───────────────────────┘
                           │
                           ▼
      ┌────────────────────────────────────────────┐  S3
      │            PROVIDE ANALYSIS RESULT          │
      └────────────────────┬───────────────────────┘
                           │
                           ▼
                    ┌─────────────┐
                    │     END     │
                    └─────────────┘
```

FIG. 10

SET DATA (MATRIX SO) COMPOSED OF PLURALITY OF PIECES OF ANALYSIS DATA USED IN LEARNING

| WAVEFORM DATA CORRESPONDING TO FORWARD SCATTERED LIGHT SIGNAL | | | WAVEFORM DATA CORRESPONDING TO SIDE SCATTERED LIGHT SIGNAL | | | WAVEFORM DATA CORRESPONDING TO FLUORESCENCE SIGNAL | | |
|---|---|---|---|---|---|---|---|---|
| 10 | ... | 100 | 50 | ... | 100 | 100 | ... | 10 |
| ... | ... | ... | ... | ... | ... | ... | ... | ... |
| 15 | ... | 70 | 30 | ... | 60 | 20 | ... | 150 |

INDEX #1 ⟶

INDEX #N ⟶

TRAINING DATA

CORRECT LABEL VALUE

LABEL VALUE CORRESPONDING TO CLASSIFICATION OF INDEX #1 ⟶

LABEL VALUE CORRESPONDING TO CLASSIFICATION OF INDEX #N ⟶

| 3 |
| ... |
| 2 |

73

CLASSIFICATION INFORMATION

INDEX #1

| LYMPHOCYTE | 0% |
| MONOCYTE | 100% |
| ... | ... |

72

INDEX #2

| LYMPHOCYTE | 100% |
| MONOCYTE | 0% |
| ... | ... |

72

EP 4 439 571 A1

# FIG. 11

FIG. 12

EMBODIMENT 2

CALCULATION PROCESSING ANALYSIS

FSC

SSC

FL

REPRESENTATIVE VALUE

SC

FLP

SSCP

CLASSIFICATION AND ANALYSIS

AI ANALYSIS

FSC

SSC

FL

SET DATA

AI ALGORITHM

60

CLASSIFICATION AND ANALYSIS

FIG. 13

FIG. 14

```
                    ┌──────────────┐
                    │    START     │
                    └──────┬───────┘
                           │
                           ▼
        ┌────────────────────────────────────────┐  S4
        │  OBTAIN OPTICAL SIGNAL AND OBTAIN       │
        │  WAVEFORM DATA FROM OPTICAL SIGNAL      │
        └────────────────────┬───────────────────┘
                             │
                             ▼
        ┌────────────────────────────────────────┐  S5
        │    EXECUTE AI ANALYSIS ON SET OF        │
        │    WAVEFORM DATA (FIRST DATA)           │
        └────────────────────┬───────────────────┘
                             │
                             ▼
        ┌────────────────────────────────────────┐  S6
        │  EXECUTE CALCULATION PROCESSING ANALYSIS│
        │            ON SECOND DATA               │
        └────────────────────┬───────────────────┘
                             │
                             ▼
        ┌────────────────────────────────────────┐  S7
        │         PROVIDE ANALYSIS RESULT         │
        └────────────────────┬───────────────────┘
                             │
                             ▼
                    ┌──────────────┐
                    │     END      │
                    └──────────────┘
```

# FIG. 15

EXAMPLE IN WHICH ANALYSIS IS
EXECUTED ON THE BASIS OF RULE
EMBODIMENT 3

```
                    ┌─────────────┐
                    │    START    │
                    └──────┬──────┘
                           │
        ┌──────────────────▼──────────────────┐   S11
        │  OBTAIN OPTICAL SIGNAL AND OBTAIN    │
        │  WAVEFORM DATA FROM OPTICAL SIGNAL   │
        └──────────────────┬──────────────────┘
                           │
        ┌──────────────────▼──────────────────┐   S12
        │   SPECIFY WAVEFORM DATA ON THE BASIS │
        │                OF RULE               │
        └──────────────────┬──────────────────┘
                           │
                     ◇ S13
          IS THERE WAVEFORM DATA OF
   NO ◄── AI ANALYSIS TARGET? ──┐
                           │ YES
        ┌──────────────────▼──────────────────┐   S14
        │             AI ANALYSIS             │
        └──────────────────┬──────────────────┘
                           │
                     ◇ S15
                   IS THERE
              WAVEFORM DATA OF
          CALCULATION PROCESSING ──► NO
             ANALYSIS TARGET?
                           │ YES
        ┌──────────────────▼──────────────────┐   S16
        │     CALCULATION PROCESSING ANALYSIS │
        └──────────────────┬──────────────────┘
                           │
        ┌──────────────────▼──────────────────┐   S17
        │         PROVIDE ANALYSIS RESULT      │
        └──────────────────┬──────────────────┘
                           │
                    ┌──────▼──────┐
                    │     END     │
                    └─────────────┘
```

# FIG. 16

EXAMPLE IN WHICH ANALYSIS IS EXECUTED
IN ACCORDANCE WITH MEASUREMENT ITEM

EMBODIMENT 4

FIG. 17

| OPERATION MODE SETTING | | |
|---|---|---|

| MEASUREMENT ITEM | AI | CALCULATION PROCESSING |
|---|---|---|
| NRBC# | ☑ | ☐ |
| NRBC% | ☑ | ☐ |
| NEUT# | ☐ | ☑ |
| LYMPH# | ☐ | ☑ |
| MONO# | ☐ | ☑ |
| BASO# | ☑ | ☐ |
| EO# | ☐ | ☑ |
| NEUT% | ☐ | ☑ |
| LYMPH% | ☐ | ☑ |
| MONO% | ☐ | ☑ |
| BASO% | ☑ | ☐ |
| EO% | ☐ | ☑ |

SET

FIG. 18

EXAMPLE IN WHICH ANALYSIS IS EXECUTED
IN ACCORDANCE WITH MEASUREMENT ORDER

START

OBTAIN OPTICAL SIGNAL AND OBTAIN
WAVEFORM DATA FROM OPTICAL SIGNAL    S11

SPECIFY WAVEFORM DATA ON THE BASIS OF
MEASUREMENT ORDER    S31

WAVEFORM DATA OF
MEASUREMENT ORDER OF AI
ANALYSIS TARGET?    S32

NO

YES

AI ANALYSIS    S14

CALCULATION PROCESSING
ANALYSIS    S16

PROVIDE ANALYSIS RESULT    S17

END

FIG. 19

OPERATION MODE SETTING

| SPECIMEN NUMBER | AI ANALYSIS MODE | CALCULATION PROCESSING ANALYSIS MODE |
|---|---|---|
| 0000099266 | ☑ | ☐ |
| 0000099267 | ☐ | ☑ |
| 0000099268 | ☐ | ☑ |
| 0000099269 | ☑ | ☐ |
| 0000099270 | ☐ | ☑ |
| 0000099271 | ☐ | ☑ |
| 0000099272 | ☐ | ☑ |
| 0000099273 | ☑ | ☐ |
| 0000099274 | ☐ | ☑ |
| 0000099275 | ☐ | ☑ |
| 0000099276 | ☑ | ☐ |
| 0000099277 | ☐ | ☑ |

SET

# FIG. 20

EXAMPLE IN WHICH ANALYSIS IS EXECUTED IN
ACCORDANCE WITH ANALYSIS MODE OF APPARATUS

```
                    ( START )
                        │
                        ▼
        ┌───────────────────────────────┐  S11
        │  OBTAIN OPTICAL SIGNAL AND OBTAIN  │
        │  WAVEFORM DATA FROM OPTICAL SIGNAL │
        └───────────────────────────────┘
                        │
                        ▼
        ┌───────────────────────────────┐  S41
        │  SPECIFY WAVEFORM DATA ON THE BASIS OF │
        │    ANALYSIS MODE OF APPARATUS  │
        └───────────────────────────────┘
                        │
                        ▼
                    ◇ S42
            WAVEFORM DATA OF AI
            ANALYSIS TARGET?        ── NO ──┐
                        │                    │
                      YES                    │
                        ▼  S14               ▼  S16
        ┌───────────────────┐   ┌───────────────────────────┐
        │   AI ANALYSIS     │   │ CALCULATION PROCESSING ANALYSIS │
        └───────────────────┘   └───────────────────────────┘
                        │               │
                        ◄───────────────┘
                        ▼
        ┌───────────────────────────────┐  S17
        │      PROVIDE ANALYSIS RESULT   │
        └───────────────────────────────┘
                        │
                        ▼
                    ( END )
```

69

FIG. 21

```
┌─────────────────────────────────────────────────────────────┐
│ OPERATION MODE SETTING                                        │
├─────────────────────────────────────────────────────────────┤
│                                                               │
│                                                               │
│        ☑  AI ANALYSIS MODE                                    │
│                                                               │
│        ☐  CALCULATION PROCESSING ANALYSIS MODE                │
│                                                               │
│                                          ┌──────────┐         │
│                                          │   SET    │         │
│                                          └──────────┘         │
│                                                               │
└─────────────────────────────────────────────────────────────┘
```

FIG. 22

EXAMPLE IN WHICH ANALYSIS IS EXECUTED IN
ACCORDANCE WITH TYPE OF MEASUREMENT ORDER

START

OBTAIN OPTICAL SIGNAL AND OBTAIN
WAVEFORM DATA FROM OPTICAL SIGNAL — S11

SPECIFY WAVEFORM DATA ON THE BASIS OF
TYPE OF MEASUREMENT ORDER — S51

WAVEFORM DATA OF AI
ANALYSIS TARGET? — S52

NO

YES

AI ANALYSIS — S14

CALCULATION PROCESSING ANALYSIS — S16

PROVIDE ANALYSIS RESULT — S17

END

FIG. 23

| OPERATION MODE SETTING | | |
|---|---|---|

| MEASUREMENT ORDER TYPE | AI ANALYSIS MODE | CALCULATION PROCESSING ANALYSIS MODE |
|---|---|---|
| Normal | ☐ | ☑ |
| Rerun | ☑ | ☐ |
| Reflex | ☑ | ☐ |

SET

# FIG. 24

EXAMPLE IN WHICH ANALYSIS IS EXECUTED IN ACCORDANCE
WITH MEASUREMENT ITEM AND TYPE OF MEASUREMENT ORDER

FIG. 25

OPERATION MODE SETTING

| MEASUREMENT ITEM | AI ANALYSIS | CALCULATION PROCESSING ANALYSIS |
|---|---|---|
| NRBC# | ☑ | ☐ |
| NRBC% | ☑ | ☐ |
| NEUT# | ☐ | ☑ |
| LYMPH# | ☐ | ☑ |
| MONO# | ☐ | ☑ |
| BASO# | ☑ | ☐ |
| EO# | ☐ | ☑ |
| NEUT% | ☐ | ☑ |
| LYMPH% | ☐ | ☑ |
| MONO% | ☐ | ☑ |
| BASO% | ☑ | ☐ |
| EO% | ☐ | ☑ |

| MEASUREMENT ORDER TYPE | AI ANALYSIS MODE | CALCULATION PROCESSING ANALYSIS MODE |
|---|---|---|
| Normal | ☐ | ☑ |
| Rerun | ☑ | ☐ |
| Reflex | ☑ | ☐ |

SET

# FIG. 26

EXAMPLE IN WHICH WHETHER OR NOT AI ANALYSIS IS
NECESSARY IS DETERMINED ON THE BASIS OF FLAG
ACCORDING TO CALCULATION PROCESSING ANALYSIS

```
                    ( START )
                        |
                        v
        +-------------------------------+
        | OBTAIN OPTICAL SIGNAL AND OBTAIN |  S11
        | WAVEFORM DATA FROM OPTICAL SIGNAL |
        +-------------------------------+
                        |
                        v
        +-------------------------------+
        | CALCULATION PROCESSING ANALYSIS |  S71
        +-------------------------------+
                        |
                        v
        +-------------------------------+
        | REFER TO RULE FOR SELECTING ANALYSIS |  S72
        |           OPERATION           |
        +-------------------------------+
                        |
                        v
                                          S73
                  /                 \
                 /  IS SPECIMEN TARGET OF AI  \
                 \      ANALYSIS?      /       NO
                  \                 /
                        | YES
                        v
        +-------------------------------+
        |          AI ANALYSIS          |  S74
        +-------------------------------+
                        |
                        v
        +-------------------------------+
        |      PROVIDE ANALYSIS RESULT      |  S17
        +-------------------------------+
                        |
                        v
                    ( END )
```

FIG. 27

OPERATION MODE SETTING

| FLAG | AI ANALYSIS |
|---|---|
| ABNORMALITY IN DIFFERENTIATION OF WHITE BLOOD CELL | ☐ |
| DECREASE OF NEUTROPHIL | ☐ |
| INCREASE OF NEUTROPHIL | ☐ |
| DECREASE OF LYMPHOCYTE | ☐ |
| INCREASE OF LYMPHOCYTE | ☐ |
| INCREASE OF MONOCYTE | ☐ |
| INCREASE OF EOSINOPHIL | ☐ |
| INCREASE OF BASOPHIL | ☐ |
| DECREASE OF WHITE BLOOD CELL | ☐ |
| INCREASE OF WHITE BLOOD CELL | ☐ |
| EMERGENCE OF NUCLEATED RED BLOOD CELL | ☐ |
| EMERGENCE OF IMMATURE GRANULOCYTE | ☐ |
| BLAST/ABNORMAL LYMPHOCYTE | ☑ |
| BLAST | ☑ |
| ABNORMAL LYMPHOCYTE | ☑ |
| ATYPICAL LYMPHOCYTE | ☑ |

SET

# FIG. 28

EXAMPLE IN WHICH AI ANALYSIS IS EXECUTED WITH
RESPECT TO SPECIFIC ANALYTE CLASSIFIED IN
CALCULATION PROCESSING ANALYSIS

START

OBTAIN OPTICAL SIGNAL AND OBTAIN
WAVEFORM DATA FROM OPTICAL SIGNAL — S11

CALCULATION PROCESSING ANALYSIS — S81

REFER TO RULE FOR SELECTING ANALYSIS
OPERATION — S82

SPECIFY WAVEFORM DATA CORRESPONDING TO
ANALYTE CLASSIFIED AS SPECIFIC
CLASSIFICATION — S83

EXECUTE AI ANALYSIS ON SPECIFIED
WAVEFORM DATA — S84

PROVIDE ANALYSIS RESULT — S17

END

FIG. 29

OPERATION MODE SETTING

| TYPE | AI ANALYSIS |
|------|-------------|
| MONOCYTE | ☑ |
| LYMPHOCYTE | ☑ |
| NEUTROPHIL | ☐ |
| EOSINOPHIL | ☐ |

SET

FIG. 30

CLASSIFY INTO MONOCYTE AND LYMPHOCYTE
THROUGH CALCULATION PROCESSING ANALYSIS

FLP

SSCP

WAVEFORM DATA

AI ANALYSIS

# FIG. 31

EXAMPLE IN WHICH AI ANALYSIS IS EXECUTED WHEN
SPECIFIC CLASSIFICATION HAS BEEN PERFORMED IN
CALCULATION PROCESSING ANALYSIS

START

OBTAIN OPTICAL SIGNAL AND OBTAIN WAVEFORM DATA FROM OPTICAL SIGNAL — S11

CALCULATION PROCESSING ANALYSIS — S91

REFER TO RULE FOR SELECTING ANALYSIS OPERATION — S92

HAS SPECIFIC CLASSIFICATION BEEN DETECTED? — S93

NO

YES

SPECIFY WAVEFORM DATA CORRESPONDING TO ANALYTE CLASSIFIED AS SPECIFIC CLASSIFICATION — S94

EXECUTE AI ANALYSIS ON SPECIFIED WAVEFORM DATA — S95

PROVIDE ANALYSIS RESULT — S17

END

FIG. 32

| OPERATION MODE SETTING |
|---|

| TYPE | AI ANALYSIS |
|---|---|
| BLAST | ☑ |
| ABNORMAL LYMPHOCYTE | ☑ |
| ATYPICAL LYMPHOCYTE | ☑ |
| IMMATURE GRANULOCYTE | ☐ |

SET

FIG. 33

EMBODIMENT 5

- 300
- PROCESSOR — 3001
- RAM — 3017
- BUS — 3003
- STORAGE — 3004
- IF PART — 3006
- DISPLAY PART — 3011
- OPERATION PART — 3012
- MEASUREMENT UNIT — 400

FIG. 34

FIG. 35

FIG. 36

# FIG. 37

EXAMPLE IN WHICH ANALYSIS IS EXECUTED IN
ACCORDANCE WITH MEASUREMENT CHANNEL

START

OBTAIN OPTICAL SIGNAL AND OBTAIN
WAVEFORM DATA FROM OPTICAL SIGNAL — S11

SPECIFY WAVEFORM DATA ON THE BASIS OF
MEASUREMENT CHANNEL — S101

IS THERE WAVEFORM DATA OF
AI ANALYSIS TARGET? — S13

NO

YES

AI ANALYSIS — S14

IS THERE
WAVEFORM DATA OF
CALCULATION PROCESSING
ANALYSIS TARGET? — S15

NO

YES

CALCULATION PROCESSING ANALYSIS — S16

PROVIDE ANALYSIS RESULT — S17

END

FIG. 38

OPERATION MODE SETTING

| MEASUREMENT CHANNEL TYPE | AI ANALYSIS | CALCULATION PROCESSING ANALYSIS |
|---|---|---|
| WDF | ☑ | ☐ |
| RET | ☐ | ☑ |
| WNR | ☐ | ☑ |
| PLT-F | ☐ | ☑ |
| WPC | ☐ | ☑ |

SET

FIG. 39

EXAMPLE IN WHICH AI ANALYSIS IS EXECUTED ON
WAVEFORM DATA OBTAINED IN WDF CHANNEL

START

OBTAIN OPTICAL SIGNAL OF WDF CHANNEL AND
OBTAIN WAVEFORM DATA FROM OPTICAL SIGNAL — S111

AI ANALYSIS — S112

PROVIDE ANALYSIS RESULT — S113

END

FIG. 40

EXAMPLE IN WHICH, ON THE BASIS OF WAVEFORM DATA OBTAINED
IN WDF CHANNEL, NUCLEATED RED BLOOD CELL AND BASOPHIL ARE
CLASSIFIED THROUGH AI ANALYSIS AND OTHERS ARE CLASSIFIED
THROUGH CALCULATION PROCESSING ANALYSIS

START

OBTAIN OPTICAL SIGNAL OF WDF CHANNEL AND
OBTAIN WAVEFORM DATA FROM OPTICAL SIGNAL — S121

AI ANALYSIS — S122

SPECIFY WAVEFORM DATA CORRESPONDING TO CELL
CLASSIFIED AS NEITHER NUCLEATED RED BLOOD CELL
NOR BASOPHIL — S123

EXECUTE CALCULATION PROCESSING ANALYSIS
ON SPECIFIED WAVEFORM DATA — S124

PROVIDE ANALYSIS RESULT — S125

END

# FIG. 41

EXAMPLE IN WHICH AI ANALYSIS IS EXECUTED WITH RESPECT
TO NEUTROPHIL/BASOPHIL SPECIFIED THROUGH CALCULATION
PROCESSING ANALYSIS IN WDF CHANNEL

START

OBTAIN OPTICAL SIGNAL OF WDF CHANNEL AND
OBTAIN WAVEFORM DATA FROM OPTICAL SIGNAL — S131

CALCULATION PROCESSING ANALYSIS — S132

SPECIFY WAVEFORM DATA CORRESPONDING TO CELLS BELOW: — S133

(1) CELL CLASSIFIED AS NONE OF LYMPHOCYTE, MONOCYTE,
EOSINOPHIL, AND NEUTROPHIL OR BASOPHIL

(2) CELL CLASSIFIED AS NEUTROPHIL OR BASOPHIL

EXECUTE AI ANALYSIS ON SPECIFIED
WAVEFORM DATA — S134

PROVIDE ANALYSIS RESULT — S135

END

FIG. 42

EP 4 439 571 A1

FIG. 43

EP 4 439 571 A1

EP 4 439 571 A1

FIG. 44

FIG. 45

3200:PLURALITY OF ARITHMETIC UNITS

3002

3200

3201

RAM

FIG. 46

FIG. 47

FIG. 48

FIG. 49

FIG. 50

EP 4 439 571 A1

FIG. 51

CALCULATION OF PRODUCT OF MATRIX

$$C_{ij} = \sum_{k=1}^{n} a_{ik} \times b_{kj}$$

```
for(int i=0; i<n; i++){
    for(int j=0; j<n; j++){
        for(int k=0; k<n; k++){
            c[ i ][ j ] += a[ i ][ k ]*b[ k ][ j ]
} } }
```

CALCULATION PROCESSING BY PARALLEL-PROCESSING PROCESSOR

```
for(int k=0; k<n; k++){
c[0][0] += a[0][k]*b[k][0] }
```

```
for(int k=0; k<n; k++){
c[0][1] += a[0][k]*b[k][1] }
```

...

```
for(int k=0; k<n; k++){
c[1][0] += a[1][k]*b[k][0] }
```

```
for(int k=0; k<n; k++){
c[1][1] += a[1][k]*b[k][1] }
```

...

:                                    :

EP 4 439 571 A1

FIG. 52

```
for(int k=0; k<n; k++){
c[0][0] += a[0][k]*b[k][0] }
```

```
for(int k=0; k<n; k++){
c[1][0] += a[1][k]*b[k][0] }
```

```
for(int k=0; k<n; k++){
c[0][1] += a[0][k]*b[k][1] }
```
...

```
for(int k=0; k<n; k++){
c[1][1] += a[1][k]*b[k][1] }
```
...

3002

3200: PLURALITY OF
ARITHMETIC UNITS

3200

3201

RAM

# FIG. 53

<u>WAVEFORM DATA AND FILTER</u>

FSC

WAVEFORM DATA

| 1 | 1 | 1 | 10 | 30 | 60 | 70 | 60 | 55 | 40 | ··· |

FILTER

① | 1 | 0 | 1 |

② | 1 | 1 | 0 |

③ | 1 | 0 | 0 |

···

<u>MATRIX OPERATION BETWEEN WAVEFORM DATA AND FILTER</u>

① | 1 | 0 | 1 |

⊗

| 1 | 1 | 1 | 10 | 30 | 60 | 70 | 60 | 55 | 40 | ··· |

⬇

① | 1 | 0 | 1 |

⊗

| 1 | 1 | 1 | 10 | 30 | 60 | 70 | 60 | 55 | 40 | ··· |

⋮

EP 4 439 571 A1

# FIG. 54

ANALYSIS UNIT 300

MEASUREMENT UNIT 400

START

START

S200

MEASUREMENT INSTRUCTION

S300

SUCTION AND DISCHARGE SPECIMEN

S301

PREPARE MEASUREMENT SAMPLE

S302

MEASURE BY DETECTION PART

S303

GENERATE WAVEFORM DATA

S201

CLASSIFY ANALYTE IN SPECIMEN

END

S202

ANALYZE CLASSIFICATION RESULT

S203

OUTPUT TEST RESULT OF SPECIMEN

END

FIG. 55

CLASSIFY ANALYTE IN SPECIMEN — S201

TRANSFER WAVEFORM DATA TO PARALLEL-PROCESSING PROCESSOR — S2010

EXECUTE PARALLEL PROCESSING — S2011

RECEIVE ARITHMETIC RESULT FROM PARALLEL-PROCESSING PROCESSOR — S2012

GENERATE CLASSIFICATION RESULT — S2013

RETURN

FIG. 56

```
┌─┬──────────────────────────┬─┐
│ │      EXECUTE PARALLEL      │ │  S2011
│ │        PROCESSING          │ │
└─┴──────────────────────────┴─┘
              │
              ▼
┌────────────────────────────────┐
│   ASSIGN ARITHMETIC PROCESSES TO │  S2100
│ ARITHMETIC UNITS OF PARALLEL-PROCESSING │
│            PROCESSOR             │
└────────────────────────────────┘
              │
              ▼
┌────────────────────────────────┐
│    ARITHMETIC UNITS ASSIGNED WITH │  S2101
│ ARITHMETIC PROCESSES EXECUTE ARITHMETIC │
│         OPERATIONS IN PARALLEL   │
└────────────────────────────────┘
              │
              ▼
┌────────────────────────────────┐
│   TRANSFER ARITHMETIC RESULT TO RAM │  S2102
└────────────────────────────────┘
              │
              ▼
         ( RETURN )
```

FIG. 57

FIG. 58

EP 4 439 571 A1

FIG. 59

EP 4 439 571 A1

FIG. 60

EP 4 439 571 A1

FIG. 61

4000

400

600

301

| MEASUREMENT UNIT | | ANALYSIS UNIT | | COMPUTER |

FIG. 62

EP 4 439 571 A1

FIG. 63

EP 4 439 571 A1

FIG. 64

ORDER

6001

6100

ANALYSIS SOFTWARE

6002

6200:PLURALITY OF ARITHMETIC UNITS

6200

6005

RAM

6201

RAM

DMA

FIG. 65

FIG. 66

400

4210

410 FCM DETECTION PART

420 ANALOG PROCESSING PART

421 CONNECTION PORT

463 BUS

450 SPECIMEN SUCTION PART

430 APPARATUS MECHANISM PART

440 SAMPLE PREPARATION PART

464 IF PART

465 IF PART

301 COMPUTER

600 ANALYSIS UNIT

EP 4 439 571 A1

FIG. 67

EP 4 439 571 A1

FIG. 68

FCM DETECTION PART — 410
ANALOG PROCESSING PART — 420
CONNECTION PORT — 421

RBC/PLT DETECTION PART — 4101
ANALOG PROCESSING PART — 4201
CONNECTION PORT — 4211

HGB DETECTION PART — 4102
ANALOG PROCESSING PART — 4202
CONNECTION PORT — 4212

4210

400

BUS — 463

SPECIMEN SUCTION PART — 450
APPARATUS MECHANISM PART — 430
SAMPLE PREPARATION PART — 440

IF PART — 464

IF PART — 465

ANALYSIS UNIT — 300

EP 4 439 571 A1

# FIG. 69

EP 4 439 571 A1

FIG. 70

FCM DETECTION PART — 410

ANALOG PROCESSING PART — 420

A/D CONVERTER — 461

IF PART — 462

IF PART — 4631

4632

400

RBC/PLT DETECTION PART — 4101

ANALOG PROCESSING PART — 4201

A/D CONVERTER — 4611

IF PART

HGB DETECTION PART — 4102

ANALOG PROCESSING PART — 4202

A/D CONVERTER — 4612

BUS — 463

SPECIMEN SUCTION PART — 450

APPARATUS MECHANISM PART — 430

SAMPLE PREPARATION PART — 440

IF PART — 464

IF PART — 465

ANALYSIS UNIT — 300

EP 4 439 571 A1

119

FIG. 71

EP 4 439 571 A1

FIG. 72

EP 4 439 571 A1

FIG. 73

ANALYSIS UNIT — 600

PROCESSOR — 6001

PARALLEL-PROCESSING PROCESSOR — 6002

BUS — 6003

STORAGE — 6004

RAM — 6005

A/D CONVERTER — 6009

A/D CONVERTER — 6009

A/D CONVERTER — 6009

IF PART — 6007

CONNECTION PORT — 6008

CONNECTION PORT — 6008

CONNECTION PORT — 6008

ANALOG SIGNAL

COMPUTER — 301

# FIG. 74

EMBODIMENT 7

FIG. 75

FIG. 76

FIG. 77

START

S401
GENERATE TRAINING DATA FROM TRAINING
WAVEFORM DATA AND CORRECT LABEL VALUE

S402
TRAIN ALGORITHM BY USING TRAINING DATA

S403
HAVE TRAINING
RESULTS OF PREDETERMINED
NUMBER OF TIMES OF TRIALS
BEEN ACCUMULATED?
NO

YES

S404
UPDATE CONNECTION WEIGHT w OF
NEURAL NETWORK BY USING ACCUMULATED
TRAINING RESULTS

S405
TRAINED
BY PRESCRIBED NUMBER
OF PIECES OF TRAINING
DATA?
NO

YES

S406
TAKE IN DIFFERENT TRAINING
WAVEFORM DATA AND CORRECT
LABEL VALUE

END

# FIG. 78

STRUCTURE OF NEURAL NETWORK

ARITHMETIC OPERATION AT EACH NODE

ARITHMETIC OPERATION BETWEEN NODES

Europäisches
Patentamt

European
Patent Office

Office européen
des brevets

# EUROPEAN SEARCH REPORT

**Application Number**

EP 24 16 5815

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/013155 A1 (PUTMAN MATTHEW C [US] ET AL) 9 January 2020 (2020-01-09) | 1,2, 7-28,33, 34 | INV. G16H10/40 |
| Y | * paragraphs [0031], [0068], [0097] * | 3-6, 29-32 | |
| Y | CN 115 825 311 A (SUZHOU BIONOVOGENE BIOLOGICAL MEDICINE TECH CO LTD) 21 March 2023 (2023-03-21) * page 1 * | 3,4,29, 30 | |
| Y | WO 2022/058980 A1 (INSILICO MEDICINE IP LTD [CN]) 24 March 2022 (2022-03-24) * paragraph [0377] * | 5,6,31, 32 | |
| A | WANG HUAMAO ET AL: "Tension in big data using machine learning: Analysis and applications", TECHNOLOGICAL FORECASTING AND SOCIAL CHANGE, ELSEVIER, NEW YORK, NY, US, vol. 158, 30 June 2020 (2020-06-30), XP086226419, ISSN: 0040-1625, DOI: 10.1016/J.TECHFORE.2020.120175 [retrieved on 2020-06-30] * the whole document * | 1-34 | |

**TECHNICAL FIELDS SEARCHED (IPC)**

G16H

The present search report has been drawn up for all claims

| Place of search | Date of completion of the search | Examiner |
|---|---|---|
| Munich | 14 August 2024 | Laub, Christoph |

EPO FORM 1503 03.82 (P04C01)

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

## ANNEX TO THE EUROPEAN SEARCH REPORT
## ON EUROPEAN PATENT APPLICATION NO.

EP 24 16 5815

This annex lists the patent family members relating to the patent documents cited in the above-mentioned European search report.
The members are as contained in the European Patent Office EDP file on
The European Patent Office is in no way liable for these particulars which are merely given for the purpose of information.

14-08-2024

| Patent document cited in search report | Publication date | Patent family member(s) | Publication date |
|---|---|---|---|
| US 2020013155 A1 | 09-01-2020 | CN 112313554 A | 02-02-2021 |
| | | CN 117350918 A | 05-01-2024 |
| | | EP 3818407 A1 | 12-05-2021 |
| | | JP 7011866 B2 | 27-01-2022 |
| | | JP 7431458 B2 | 15-02-2024 |
| | | JP 2021528775 A | 21-10-2021 |
| | | JP 2022050565 A | 30-03-2022 |
| | | KR 20210025104 A | 08-03-2021 |
| | | TW 202018366 A | 16-05-2020 |
| | | US 10169852 B1 | 01-01-2019 |
| | | US 10467740 B1 | 05-11-2019 |
| | | US 2020013155 A1 | 09-01-2020 |
| | | US 2021012473 A1 | 14-01-2021 |
| | | US 2021224966 A1 | 22-07-2021 |
| | | US 2023419444 A1 | 28-12-2023 |
| | | WO 2020009749 A1 | 09-01-2020 |
| CN 115825311 A | 21-03-2023 | NONE | |
| WO 2022058980 A1 | 24-03-2022 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023052355 A

- WO 2018203568 A